(19) Europäisches Patentamt
European Patent Office
Office européen des brevets

(11) **EP 2 997 886 B1**

## (12) EUROPEAN PATENT SPECIFICATION

(45) Date of publication and mention
of the grant of the patent:
**08.08.2018 Bulletin 2018/32**

(51) Int Cl.:
*A61B 5/00* (2006.01)          *A61B 5/01* (2006.01)
*G01K 13/00* (2006.01)

(21) Application number: **15184759.7**

(22) Date of filing: **10.09.2015**

(54) **NON-TOUCH DETECTION OF BODY CORE TEMPERATURE**

BERÜHRUNGSLOSE DETEKTION DER KÖRPERKERNTEMPERATUR

DÉTECTION SANS CONTACT DE LA TEMPÉRATURE CENTRALE D'UN CORPS

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR** | • **Crawley, Martin**<br>**Belfast**<br>**County Antrim, BT1 2DX (GB)**<br>• **Gross, Irwin**<br>**Boca Raton, FL 33432 (US)** |

(30) Priority: 13.09.2014  US 201414485724
14.09.2014  US 201414485767
14.09.2014  US 201414485772
14.09.2014  US 201414485777
14.09.2014  US 201414485784
14.09.2014  US 201414485788
14.09.2014  US 201414485794
14.09.2014  US 201414485792
15.09.2014  US 201414485796
15.09.2014  US 201414485798
15.09.2014  US 201414485802
15.09.2014  US 201414485803
15.09.2014  US 201414485804
15.09.2014  US 201414485806

• **Smith, Michael G.**
**Austin, TX 78734 (US)**
• **Gest, Steven**
**Boca Raton, FL 33432 (US)**
• **Barrett, John**
**New Mallow Road, Cork (IE)**
• **Cronin, Michael**
**New Mallow Road, Cork (IE)**
• **Turnbull, Derek**
**New Mallow Road, Cork (IE)**

(43) Date of publication of application:
**23.03.2016 Bulletin 2016/12**

(74) Representative: **Petraz, Gilberto Luigi et al**
**GLP S.r.l.**
**Viale Europa Unita, 171**
**33100 Udine (IT)**

(73) Proprietor: **Arc Devices Limited**
**Galway (IE)**

(56) References cited:
WO-A1-2015/025311     CN-A- 101 843 476
JP-A- H11 113 858       JP-A- 2005 148 038
KR-A- 20110 082 282    US-A1- 2010 280 331
US-A1- 2013 085 708    US-A1- 2014 072 190
US-B1- 8 452 382

(72) Inventors:
• **Khachaturian, Mark**
**Boca Raton, FL 33432 (US)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD

This disclosure relates generally to generating a representation of animal body core temperature from animal skin temperature readings.

BACKGROUND

Prior techniques of generating the representation of animal body core temperature from animal skin temperature readings typically overestimate body core temperatures for a skin temperature reading that is below 86 degrees Fahrenheit. Prior techniques of generating the representation of animal body core temperature from animal skin temperature readings typically overestimate body core temperatures for a skin temperature reading that is greater than 101 degrees Fahrenheit. Skin is one example of an external source point.

One prior technique of generating of animal body core temperature from animal skin temperature readings includes a calculation based on Formula 1:

$$T_{body} = |f_{stb}(T_{surface\ temp} + f_{ntc}(T_{ntc})) + F4_{body}|$$

Formula 1

where $T_{body}$ is the temperature of a body or subject
where $f_{stb}$ is a mathematical formula of a surface of a body
where $f_{ntc}$ is mathematical formula for ambient temperature reading
where $T_{surface\ temp}$ is a surface temperature determined from the sensing.
where $T_{ntc}$ is an ambient air temperature reading
where $F4_{body}$ is a calibration difference in axillary mode, which is stored or set in a memory of the apparatus either during manufacturing or in the field. The apparatus also sets, stores and retrieves $F4_{oral}$, $F4_{core}$, and $F4_{rectal}$ in the memory.
$f_{ntc}(T_{ntc})$ is a bias in consideration of the temperature sensing mode. For example $f_{axillary}(T_{axillary}) = 0.2$ °C, $f_{oral}(T_{oral})$ = 0.4 °C, $f_{rectal}(T_{rectal}) = 0.5$ °C and $f_{core}(T_{core}) = 0.3$ °C.

In one implementation of generating a body core temperature of from a reading of skin temperature of a carotid artery biases a sensed temperature of the carotid artery. The sensed temperature is biased by +0.5 °C to yield the correlated body temperature. In another example, the sensed (skin reading) temperature is biased by -0.5 °C to yield the correlated body temperature. An example of correlating body temperature of a carotid artery follows:

$f_{ntc}(T_{ntc}) = 0.2$ °C when $T_{ntc}$ =26.2 °C as retrieved from a data table for body sensing mode.
assumption: $T_{surface\ temp} = 37.8$ °C

$$T_{surface\ temp} + f_{ntc}(T_{ntc}) = 37.8\ °C + 0.2\ °C = 38.0\ °C$$

$$f_{stb}(T_{surface\ temp} + f_{ntc}(T_{ntc})) = 38\ °C + 1.4\ °C = 39.4\ °C$$

assumption: $F4_{body} = 0.5$ °C

$$T_{body} = |f_{stb}(T_{surface\ temp} + f_{ntc}(T_{ntc})) + F4_{body}| = |39.4\ °C + 0.5\ C\ | = 39.9$$
$$°C$$

The generated body core temperature from the carotid artery skin reading is 39.9 °C.
In an example of generating body core temperature from a plurality of external locations, such as a forehead and a carotid artery to an axillary temperature, first a forehead temperature is generated using formula as follows:

$f_{ntc}(T_{ntc}) = 0.2$ °C when $T_{ntc}$ =26.2 °C as retrieved from a data table for axillary sensing mode.
assumption: $T_{surface\ temp} = 37.8$ °C

$$T_{surface\ temp} + f_{ntc}(T_{ntc}) = 37.8\ °C + 0.2\ °C = 38.0\ °C$$

$$f_{stb}(T_{surface\ temp} + f_{ntc}(T_{ntc})) = 38\ °C + 1.4\ °C = 39.4\ °C$$

assumption: $F4_{body} = 0\ °C$

$$T_{body} = |f_{stb}(T_{surface\ temp} + f_{ntc}(T_{ntc})) + F4_{body}| = |39.4\ °C + 0\ C| = 39.4\ °C$$

And second, a carotid temperature is generated using formula as follows:

$f_{ntc}(T_{ntc}) = 0.6\ °C$ when $T_{ntc} = 26.4\ °C$ as retrieved from a data table.
assumption: $T_{surface\ temp} = 38.0\ °C$

$$T_{surface\ temp} + f_{ntc}(T_{ntc}) = 38.0\ °C + 0.6\ °C = 38.6\ °C$$

$$f_{stb}(T_{surface\ temp} + f_{ntc}(T_{ntc})) = 38.6\ °C + 1.4\ C = 40.0\ °C$$

assumption: $F4_{body} = 0\ °C$

$$T_{body} = |f_{stb}(T_{surface\ temp} + f_{ntc}(T_{ntc})) + F4_{body}| = |40.0\ °C + 0\ C| = 40.0\ °C$$

Thereafter the generated temperature for the forehead (39.4 °C) and the correlated temperature for the carotid artery (40.0 °C) are averaged, yielding the final result of the scan of the forehead and the carotid artery as 39.7 °C.
JP 11113858 discloses an apparatus to estimate a body core temperature from an external source point according to the preamble of claim 1.

BRIEF DESCRIPTION

The object of the present invention is achieved with an apparatus according to the independent claim. In one aspect, an apparatus estimates body core temperature from an infrared measurement of an external source point using a cubic relationship between the body core temperature and the measurement of an external source point.
In a further aspect, a non-touch biologic detector estimates body core temperature from an infrared measurement of an external source point and determines vital signs from a solid-state image transducer.
In another aspect, a non-touch biologic detector determines vital signs from a solid-state image transducer and estimates body core temperature from an infrared measurement of an external source point using a cubic relationship between the body core temperature and the measurement of an external source point.
Apparatus, systems, and methods of varying scope are described herein. In addition to the aspects and advantages described in this summary, further aspects and advantages will become apparent by reference to the drawings and by reading the detailed description that follows.

BRIEF DESCRIPTION OF THE DRAWINGS

FIG. 1 is a block diagram of a non-touch biologic detector that includes a digital infrared sensor, according to an implementation;
FIG. 2 is a block diagram of a non-touch biologic detector that includes a digital infrared sensor and that does not include an analog-to-digital converter, according to an implementation;
FIG. 3 is a block diagram of a non-touch biologic detector that includes a digital infrared sensor and a color display device, according to an implementation;
FIG. 4 is a block diagram of apparatus that estimates a body core temperature of an external source point from a no-touch electromagnetic sensor, according to an implementation;
FIG. 5 is a block diagram of apparatus to estimate a body core temperature from an external source point from an analog infrared sensor, according to an implementation;
FIG. 6 is a block diagram of apparatus to estimate a body core temperature from an external source point from a digital infrared sensor, according to an implementation;
FIG. 7 is a block diagram of apparatus that estimates a body core temperature of an external source point from a non-touch electromagnetic sensor and that detects vital-signs from images captured by a solid-state image transducer, according to an implementation;

FIG. 8 is a block diagram of apparatus that estimates a body core temperature of an external source point from an analog infrared sensor and that detects vital-signs from images captured by a solid-state image transducer, according to an implementation.

FIG. 9 is a block diagram of apparatus that estimates a body core temperature of an external source point from a digital infrared sensor and that detects vital-signs from images captured by a solid-state image transducer, according to an implementation;

FIG. 10 is a block diagram of apparatus that estimates a body core temperature of an external source point from a digital infrared sensor, that does not include an analog-to-digital converter and that detects vital-signs from images captured by a solid-state image transducer, according to an implementation;

FIG. 11 is a flowchart of a method to determine a temperature from a digital infrared sensor, according to an implementation;

FIG. 12 is a flowchart of a method to display temperature color indicators, according to an implementation of three colors;

FIG. 13 is a flowchart of a method to manage power in a non-touch biologic detector or thermometer having a digital infrared sensor, according to an implementation;

FIG. 14 is a block diagram of an apparatus of motion amplification, according to an implementation.

FIG. 15 is a block diagram of an apparatus of motion amplification, according to an implementation.

FIG. 16 is a block diagram of an apparatus of motion amplification, according to an implementation.

FIG. 17 is a block diagram of an apparatus of motion amplification, according to an implementation.

FIG. 18 is a block diagram of an apparatus of motion amplification, according to an implementation;

FIG. 19 is a block diagram of an apparatus to generate and present any one of a number of biological vital signs from amplified motion, according to an implementation;

FIG. 20 is a block diagram of an apparatus of motion amplification, according to an implementation;

FIG. 21 is a block diagram of an apparatus of motion amplification, according to an implementation;

FIG. 22 is an apparatus that performs motion amplification to generate biological vital signs, according to an implementation;

FIG. 23 is a flowchart of a method of motion amplification, according to an implementation;

FIG. 24 is a flowchart of a method of motion amplification, according to an implementation that does not include a separate action of determining a temporal variation;

FIG. 25 is a flowchart of a method of motion amplification, according to an implementation;

FIG. 26 is a flowchart of a method of motion amplification, according to an implementation;

FIG. 27 is a flowchart of a method of motion amplification from which to generate and communicate biological vital signs, according to an implementation;

FIG. 28 is a flowchart of a method to estimate a body core temperature from an external source point in reference to a cubic relationship, according to an implementation;

FIG. 29 is a flowchart of a method to estimate a body core temperature from an external source point and other measurements in reference to a cubic relationship, according to an implementation;

FIG. 30 is a block diagram of a hand-held device, according to an implementation;

FIG. 31 illustrates an example of a computer environment, according to an implementation;

FIG. 32 is a representation of display that is presented on the display device of apparatus in FIG. 1-10 and 33-38, according to an implementation;

FIG. 33 is a portion of a schematic of a circuit board of a non-touch thermometer, according to an implementation;

FIG. 34 is a portion of the schematic of the non-touch thermometer having the digital IR sensor, according to an implementation;

FIG. 35 is a portion of the schematic of the non-touch thermometer having the digital IR sensor, according to an implementation;

FIG. 36 is a circuit that is a portion of the schematic of the non-touch thermometer having the digital IR sensor, according to an implementation;

FIG. 37 is a circuit that is a portion of the schematic of the non-touch thermometer having the digital IR sensor, according to an implementation;

FIG. 38 is a block diagram of a solid-state image transducer, according to an implementation.

DETAILED DESCRIPTION

In the following detailed description, reference is made to the accompanying drawings that form a part hereof, and in which is shown by way of illustration specific implementations which may be practiced. These implementations are described in sufficient detail to enable those skilled in the art to practice the implementations, and it is to be understood that other implementations may be utilized and that logical, mechanical, electrical and other changes may be made without departing from the scope of the implementations. The following detailed description is, therefore, not to be taken in a limiting sense.

The detailed description is divided into nine sections. In the first section, implementations of apparatus of digital non-touch thermometers and vital sign motion amplification detectors are described. In the second section, implementations of apparatus of non-touch cubic-estimation thermometers are described. In the third section, implementations of apparatus of non-touch cubic estimation thermometers and vital sign detectors are described. In the fourth section, methods of digital infrared thermometers are described. In the fifth section, implementations of apparatus of vital sign motion amplification detectors are described. In the sixth section, implementations of methods of vital sign amplification are described. In the seventh section, implementations of methods of non-touch cubic-estimation are described. In the eight section, hardware and operating environments in which implementations may be practiced are described. Finally, in the ninth section, a conclusion of the detailed description is provided.

Digital Non-touch Thermometers and Vital Sign Motion Amplification Detectors Apparatus Implementations

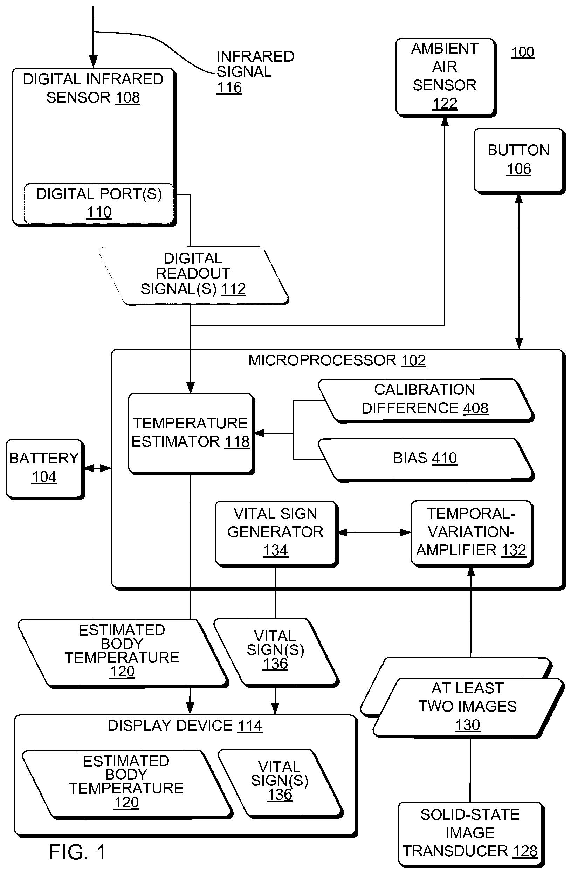

FIG. 1 is a block diagram of a non-touch biologic detector 100 that includes a digital infrared sensor, according to an implementation. Non-touch biologic detector 100 is an apparatus to measure temperature and other vital signs.
The non-touch biologic detector 100 includes a microprocessor 102. The non-touch biologic detector 100 includes a battery 104, a single button 106 and a digital infrared sensor 108 that is operably coupled to the microprocessor 102. The digital infrared sensor 108 includes digital ports 110 that provide only digital readout signal 112. The non-touch biologic detector 100 includes a display device 114 that is operably coupled to the microprocessor 102. The microprocessor 102 is operable to receive from the digital ports 110 that provide only digital readout signal 112. The digital readout signal 112 is representative of an infrared signal 116 detected by the digital infrared sensor 108. A temperature estimator 118 in the microprocessor 102 is operable to estimate the temperature 120 from the digital readout signal 112 that is representative of the infrared signal 116, a representation of an ambient air temperature reading from an ambient air sensor 122, a representation of a calibration difference from a memory location that stores a calibration difference 124 and a memory location that stores a representation of a bias 126 in consideration of a temperature sensing mode.
Some implementations of the non-touch biologic detector 100 include a solid-state image transducer 128 that is operably coupled to the microprocessor 102 and is operable to provide two or more images 130 to a temporal-variation-amplifier 132 and a vital sign generator 134 in the microprocessor 102 to estimate one or more vital signs 136 that are displayed on the display device 114.
In some implementations, the digital IR sensor 108 is a low noise amplifier, 17-bit ADC and powerful DSP unit through which high accuracy and resolution of the estimated body temperature 120 by the apparatus in FIG. 1-3, 6, 9-10 and 33-37 is achieved.
In some implementations, the digital IR sensor 108, 10-bit pulse width modulation (PWM) is configured to continuously transmit the measured temperature in range of -20...120°C, with an output resolution of 0.14°C. The factory default power on reset (POR) setting is SMBus.
In some implementations, the digital IR sensor 108 is packaged in an industry standard TO-39 package.
In some implementations, the generated object and ambient temperatures are available in RAM of the digital IR sensor 108 with resolution of 0.01 °C. The temperatures are accessible by 2 wire serial SMBus compatible protocol (0.02°C resolution) or via 10-bit PWM (Pulse Width Modulated) output of the digital IR sensor 108.
In some implementations, the digital IR sensor 108 is factory calibrated in wide temperature ranges: -40...85°C for the ambient temperature and -70...380°C for the object temperature.
In some implementations of the digital IR sensor 108, the measured value is the average temperature of all objects in the Field Of View (FOV) of the sensor. In some implementations, the digital IR sensor 108 has a standard accuracy of $\pm 0.5$°C around room temperatures, and in some implementations, the digital IR sensor 108 has an accuracy of $\pm 0.2$°C in a limited temperature range around the human body temperature.
These accuracies are only guaranteed and achievable when the sensor is in thermal equilibrium and under isothermal conditions (there are no temperature differences across the sensor package). The accuracy of the detector can be influenced by temperature differences in the package induced by causes like (among others): Hot electronics behind the sensor, heaters/coolers behind or beside the sensor or by a hot/cold object very close to the sensor that not only heats the sensing element in the detector but also the detector package. In some implementations of the digital IR sensor 108, the thermal gradients are measured internally and the measured temperature is compensated in consideration of the thermal gradients, but the effect is not totally eliminated. It is therefore important to avoid the causes of thermal gradients as much as possible or to shield the sensor from the thermal gradients.
In some implementations, the digital IR sensor 108 is calibrated for an object emissivity of 1, but in some implementations, the digital IR sensor 108 is calibrated for any emissivity in the range 0.1 ...1.0 without the need of recalibration with a black body.

In some implementations of the digital IR sensor 108, the PWM can be easily customized for virtually any range desired by the customer by changing the content of 2 EEPROM cells. Changing the content of 2 EEPROM cells has no effect on the factory calibration of the device. The PWM pin can also be configured to act as a thermal relay (input is To), thus allowing for an easy and cost effective implementation in thermostats or temperature (freezing / boiling) alert applications. The temperature threshold is programmable by the microprocessor 102 of the non-touch biologic detector. In a non-touch biologic detector having a SMBus system the programming can act as a processor interrupt that can trigger reading all slaves on the bus and to determine the precise condition.

In some implementations, the digital IR sensor 108 has an optical filter (long-wave pass) that cuts off the visible and near infra-red radiant flux is integrated in the package to provide ambient and sunlight immunity. The wavelength pass band of the optical filter is from 5.5 till $14\mu m$.

In some implementations, the digital IR sensor 108 is controlled by an internal state machine, which controls the measurements and generations of the object and ambient temperatures and does the post-processing of the temperatures to output the temperatures through the PWM output or the SMBus compatible interface.

Some implementations of the non-touch biologic detector includes 2 IR sensors, the output of the IR sensors being amplified by a low noise low offset chopper amplifier with programmable gain, converted by a Sigma Delta modulator to a single bit stream and fed to a DSP for further processing. The signal is treated by programmable (by means of EEPROM contend) FIR and IIR low pass filters for further reduction of the bandwidth of the input signal to achieve the desired noise performance and refresh rate. The output of the IIR filter is the measurement result and is available in the internal RAM. 3 different cells are available: One for the on-board temperature sensor and 2 for the IR sensors. Based on results of the above measurements, the corresponding ambient temperature Ta and object temperatures To are generated. Both generated temperatures have a resolution of 0.01°C. The data for Ta and To is read in two ways: Reading RAM cells dedicated for this purpose via the 2-wire interface (0.02°C resolution, fixed ranges), or through the PWM digital output (10 bit resolution, configurable range). In the last step of the measurement cycle, the measured Ta and To are rescaled to the desired output resolution of the PWM) and the regenerated data is loaded in the registers of the PWM state machine, which creates a constant frequency with a duty cycle representing the measured data.

In some implementations, the digital IR sensor 108 includes a SCL pin for Serial clock input for 2 wire communications protocol, which supports digital input only, used as the clock for SMBus compatible communication. The SCL pin has the auxiliary function for building an external voltage regulator. When the external voltage regulator is used, the 2-wire protocol for a power supply regulator is overdriven.

In some implementations, the digital IR sensor 108 includes a slave deviceA/PWM pin for Digital input/output. In normal mode the measured object temperature is accessed at this pin Pulse Width Modulated. In SMBus compatible mode the pin is automatically configured as open drain NMOS. Digital input / output, used for both the PWM output of the measured object temperature(s) or the digital input / output for the SMBus. In PWM mode the pin can be programmed in EEPROM to operate as Push / Pull or open drain NMOS (open drain NMOS is factory default). In SMBus mode slave deviceA is forced to open drain NMOS I/O, push-pull selection bit defines PWM / Thermal relay operation. The PWM / slave deviceA pin the digital IR sensor 108 operates as PWM output, depending on the EEPROM settings. When WPWM is enabled, after POR the PWM / slave deviceA pin is directly configured as PWM output. When the digital IR sensor 108 is in PWM mode, SMBus communication is restored by a special command. In some implementations, the digital IR sensor 108 is read via PWM or SMBus compatible interface. Selection of PWM output is done in EEPROM configuration (factory default is SMBus). PWM output has two programmable formats, single and dual data transmission, providing single wire reading of two temperatures (dual zone object or object and ambient). The PWM period is derived from the on-chip oscillator and is programmable.

In some implementations, the digital IR sensor 108 includes a VDD pin for External supply voltage and a VSS pin for ground.

The microprocessor 102 has read access to the RAM and EEPROM and write access to 9 EEPROM cells (at addresses 0x00, 0x01, 0x02, 0x03, 0x04, 0x05*, 0x0E, 0x0F, 0x09). When the access to the digital IR sensor 108 is a read operation, the digital IR sensor 108 responds with 16 data bits and 8 bit PEC only if its own slave address, programmed in internal EEPROM, is equal to the SA, sent by the master. A slave feature allows connecting up to 127 devices (SA=0x00...0x07F) with only 2 wires. In order to provide access to any device or to assign an address to a slave device before slave device is connected to the bus system, the communication starts with zero slave address followed by low R/W bit. When the zero slave address followed by low R/W bit sent from the microprocessor 102, the digital IR sensor 108 responds and ignores the internal chip code information.

In some implementations, two digital IR sensors 108 are not configured with the same slave address on the same bus.

In regards to bus protocol, after every received 8 bits the slave device should issue ACK or NACK. When a microprocessor 102 initiates communication, the microprocessor 102 first sends the address of the slave and only the slave device which recognizes the address will ACK, the rest will remain silent. In case the slave device NACKs one of the bytes, the microprocessor 102 stops the communication and repeat the message. A NACK could be received after the packet error code (PEC). A NACK after the PEC means that there is an error in the received message and the microprocessor 102 will try resending the message. PEC generation includes all bits except the START, REPEATED START, STOP, ACK, and NACK bits. The PEC is a CRC-8 with polynomial X8+X2+X1+1. The Most Significant Bit of every byte is transferred first.

In single PWM output mode the settings for PWM1 data only are used. The temperature reading can be generated from the signal timing as:

$$T_{OUT} = \left( \frac{2t_2}{T} \times \left( T_{O\_MAX} - T_{O\_MIN} \right) \right) + T_{O\_MIN}$$

where Tmin and Tmax are the corresponding rescale coefficients in EEPROM for the selected temperature output (Ta, object temperature range is valid for both Tobj1 and Tobj2 as specified in the previous table) and T is the PWM period. Tout is TO1, TO2 or Ta according to Config Register [5:4] settings.

The different time intervals t1...t4 have following meaning:

t1: Start buffer. During t1 the signal is always high. t1 = 0.125s x T (where T is the PWM period)
t2: Valid Data Output Band, 0...1/2T. PWM output data resolution is 10 bit.
t3: Error band - information for fatal error in EEPROM (double error detected, not correctable).
t3 = 0.25s x T. Therefore a PWM pulse train with a duty cycle of 0.875 will indicate a fatal error in EEPROM (for single PWM format). FE means Fatal Error.

In regards to a format for extended PWM, the temperature transmitted in Data 1 field can be generated using the following equation:

$$T_{OUT1} = \left( \frac{4t_2}{T} \times \left( T_{MAX1} - T_{MIN1} \right) \right) + T_{MIN1}$$

For Data 2 field the equation is:

$$T_{OUT2} = \left( \frac{4t_5}{T} \times \left( T_{MAX2} - T_{MIN2} \right) \right) + T_{MIN2}$$

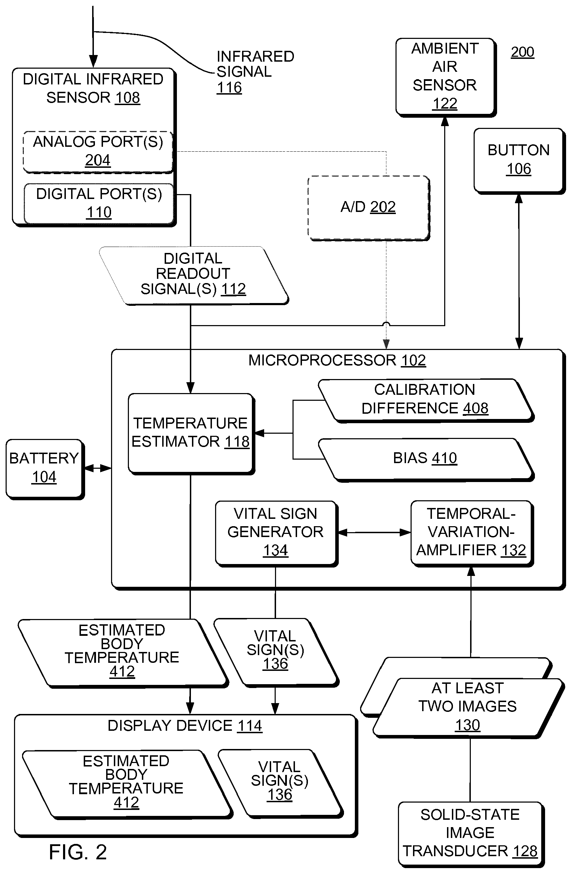

FIG. 2 is a block diagram of a non-touch biologic detector that includes a digital infrared sensor and that does not include an analog-to-digital converter, according to an implementation. The non-touch biologic detector 200 does not include an analog-to-digital (A/D) converter 202 operably coupled between the digital infrared sensor 108 and the microprocessor 102. The digital infrared sensor 108 also does not include analog readout ports 204. The dashed lines of the A/D converter 202 and the analog readout ports 204 indicates absence of the A/D converter 202 and the analog readout ports 204 in the non-touch biologic detector 200. The non-touch biologic detector 200 includes a microprocessor 102. The non-touch biologic detector 200 includes a battery 104, a single button 106, a display device 114 and a digital infrared sensor 108 that is operably coupled to the microprocessor 102. No analog-to-digital converter is operably coupled between the digital infrared sensor 108 and the microprocessor 102. The digital infrared sensor 108 has only digital ports 110 and the digital infrared sensor 108 has no analog sensor readout ports. The microprocessor 102 is operable to receive from the digital ports 110 a digital readout signal 112 that is representative of an infrared signal 116 detected by the digital infrared sensor 108 and to determine the temperature 120 from the digital readout signal 112 that is representative of the infrared signal 116.

Some implementations of the non-touch biologic detector 200 include a solid-state image transducer 128 that is operably coupled to the microprocessor 102 and is operable to provide two or more images 130 to a temporal-variation-amplifier 132 and a vital sign generator 134 in the microprocessor 102 to estimate one or more vital signs 136 that are displayed on the display device 114.

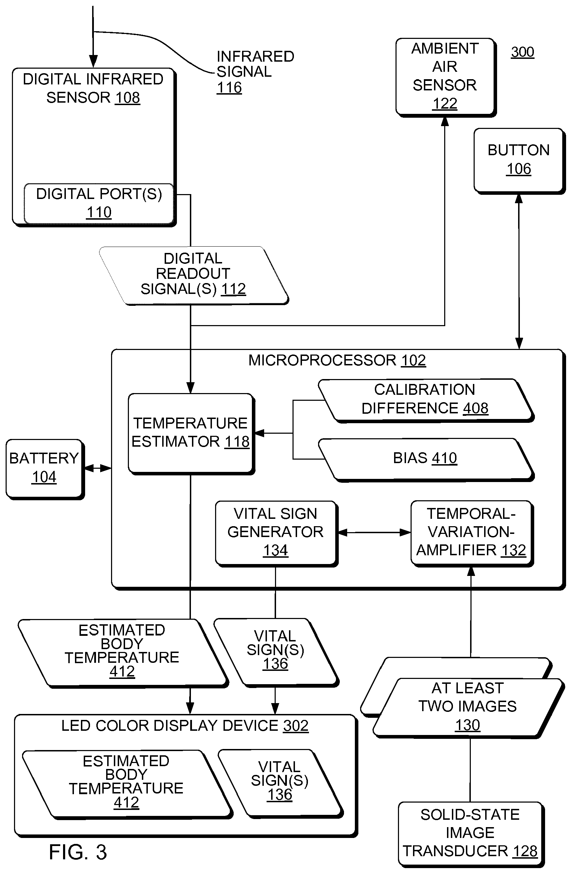

FIG. 3 is a block diagram of a non-touch biologic detector 300 that includes a digital infrared sensor and a color display device, according to an implementation. In FIG. 3, the display device 114 of FIG. 1 is a LED color display device 302.

Non-Touch Cubic-Estimation Thermometers Apparatus Implementations

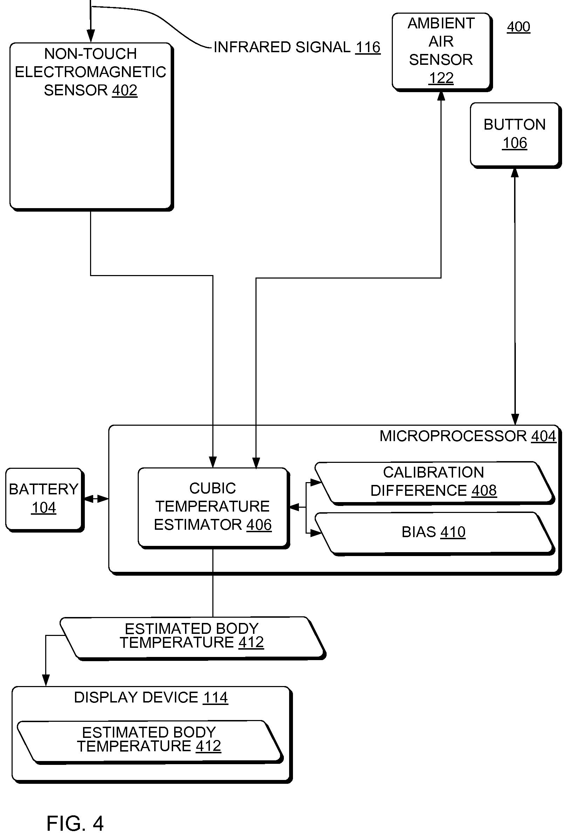

FIG. 4 is a block diagram of apparatus 400 that estimates a body core temperature of an external source point from a non-touch electromagnetic sensor, according to an implementation. The apparatus 400 includes a battery 104, a single button 106, a display device 114, a non-touch electromagnetic sensor 402 and an ambient air sensor 122 that are operably coupled to the microprocessor 404. The microprocessor 404 is operable to receive a representation of an infrared signal 116 of the external source point from the non-touch electromagnetic sensor 402. The microprocessor 404 includes a cubic temperature estimator 406 that is operable to estimate the body core temperature 412 of the subject from the representation of the electromagnetic energy of the external source point.

The cubic temperature estimator 406 that estimates body temperature in reference to a cubic relationship that represents three thermal ranges between the body core temperature and the numerical representation of the electromagnetic energy of the external source point. The cubic relationship includes a coefficient representative of different relationships between the external source point and the body core temperature in the three thermal ranges in reference to the numerical representation of the electromagnetic energy of the external source point, numerical constants for each cubic factor, ambient air temperature and the three thermal ranges. The cubic relationship for all ranges of ambient temperatures provides best results because a linear or a quadratic relationship provide inaccurate estimates of body temperature, yet a quartic relationship, a quintic relationship, sextic relationship, a septic relationship or an octic relationship provide estimates along a highly irregular curve that is far too wavy or twisting with relatively sharp deviations from one ambient temperature to another ambient temperature.

The non-touch electromagnetic sensor 402 detects temperature in response to remote sensing of a surface a human or animal. In some implementations, the non-touch thermometer is an infrared temperature sensor. All humans or animals radiate infrared energy. The intensity of this infrared energy depends on the temperature of the human or animal, thus the amount of infrared energy emitted by a human or animal can be interpreted as a proxy or indication of the temperature of the human or animal. The non-touch electromagnetic sensor 402 measures the temperature of a human or animal based on the electromagnetic energy radiated by the human or animal. The measurement of electromagnetic energy is taken by the non-touch electromagnetic sensor 402 which constantly analyzes and registers the ambient temperature. When the operator of apparatus in FIG. 1 holds the non-touch electromagnetic sensor 402 about 5-8 cm (2-3 inches) from the forehead and activates the radiation sensor, the measurement is instantaneously measured. To measure a temperature using the non-touch electromagnetic sensor 402, pushing the button 106 causes a reading of temperature measurement from the non-touch electromagnetic sensor 402 and the measured temperature is thereafter displayed on the display device 114.

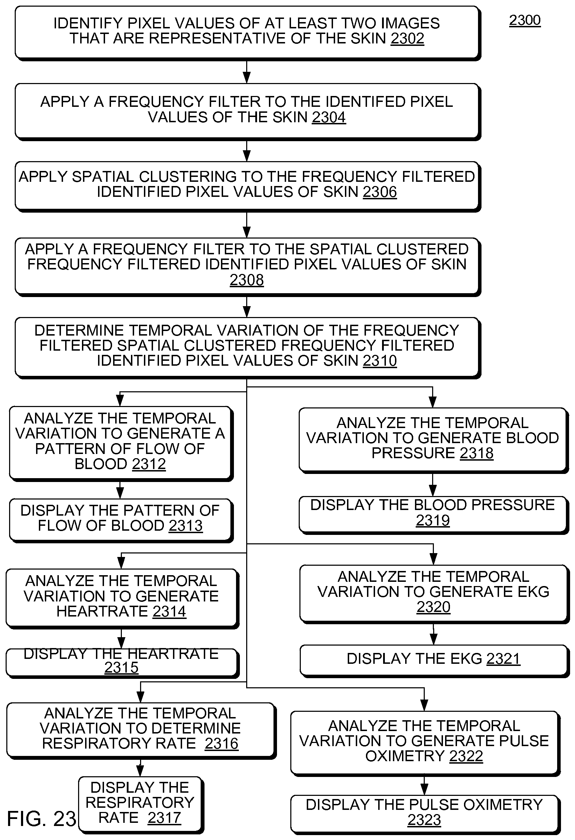

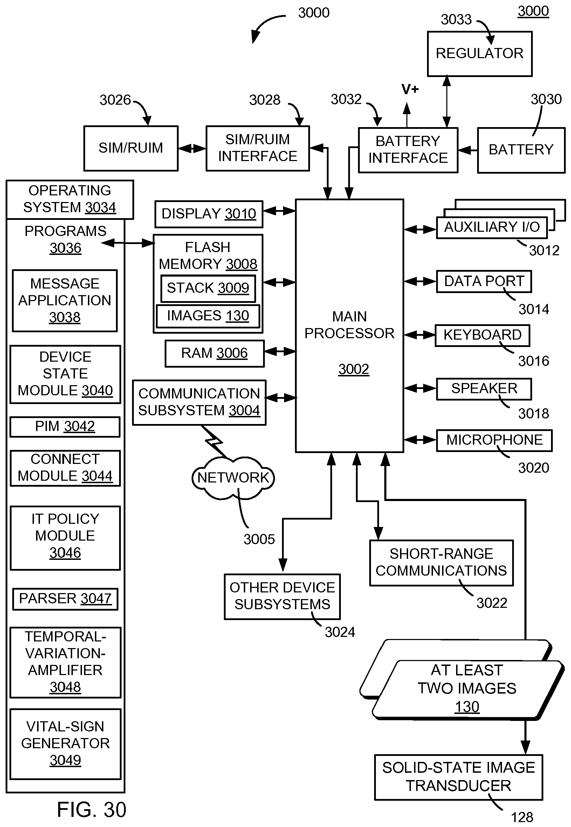

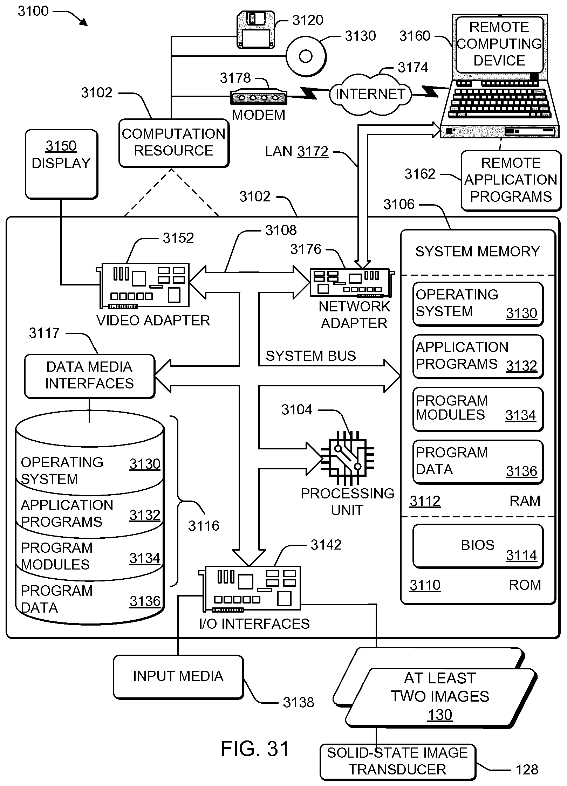

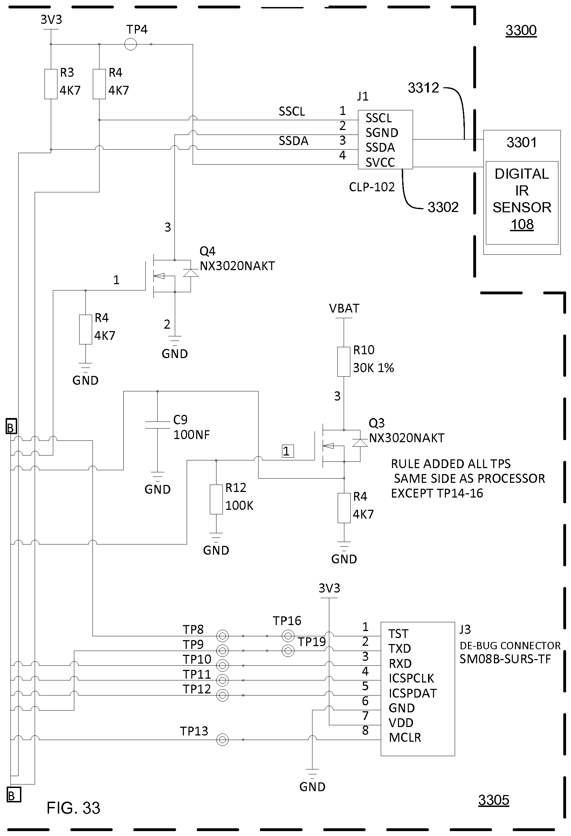

Body temperature of a human or animal can be measured in many surface locations of the body. Most commonly, temperature measurements are taken of the forehead, mouth (oral), inner ear (tympanic), armpit (axillary) or rectum. In addition, temperature measurements are taken of a carotid artery (the external carotid artery on the right side of a human neck). An ideal place to measure temperature is the forehead in addition to the carotid artery. When electromagnetic energy is sensed from two or more source points, for example, the forehead and the external carotid artery on the right side of a human neck, a cubic temperature estimator 406 performs one or more of the actions in the methods that are described in FIG. 23-29. The cubic temperature estimator 406 correlates the temperatures sensed by the non-touch electromagnetic sensor 402 from the multiple source points (e.g. the forehead and the carotid artery) to another temperature, such as a core temperature of the subject, an axillary temperature of the subject, a rectal temperature of the subject and/or an oral temperature of the subject. The cubic temperature estimator 406 can be implemented as a component on a microprocessor, such as main processor 3002 in FIG. 30, processing unit 3104 in FIG. 31 or microprocessor 3304 in FIG. 33 or on a memory such as flash memory 3008 in FIG. 30 or system memory 3106.

The apparatus 400 also detects the body temperature of a human or animal regardless of the room temperature because the measured temperature of the non-touch electromagnetic sensor 402 is adjusted in reference to the ambient temperature in the air in the vicinity of the apparatus. The human or animal must not have undertaken vigorous physical activity prior to temperature measurement in order to avoid a misleading high temperature. Also, the room temperature should be moderate, 50°F to 120°F.

The apparatus 400 provides a non-invasive and non-irritating means of measuring human or animal temperature to help ensure good health.

When evaluating results, the potential for daily variations in temperature can be considered. In children less than 6 months of age daily variation is small. In children 6 months to 2 years old the variation is about 1 degree. By age 6 variations gradually increase to 2 degrees per day. In adults there is less body temperature variation.

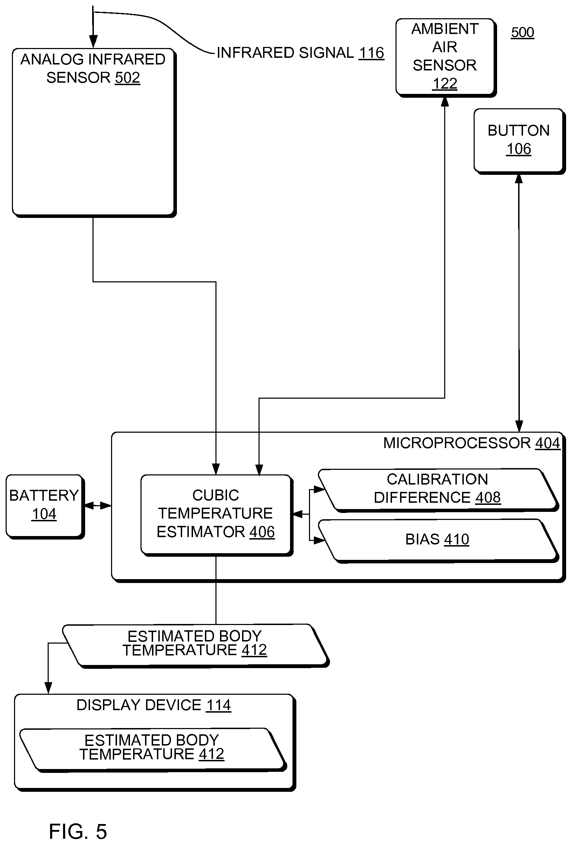

FIG. 5 is a block diagram of apparatus 500 to estimate a body core temperature from an external source point from an analog infrared sensor, according to an implementation. The apparatus 500 includes a battery 104, a single button 106, a display device 114, an analog infrared sensor 502 and an ambient air sensor 122 that are operably coupled to the microprocessor 404. The microprocessor 404 is operable to receive a representation of an infrared signal 116 of the external source point from the analog infrared sensor 502. The microprocessor 404 includes a cubic temperature estimator 406 that is operable to estimate the body core temperature 412 of the subject from the representation of the electromagnetic energy of the external source point.

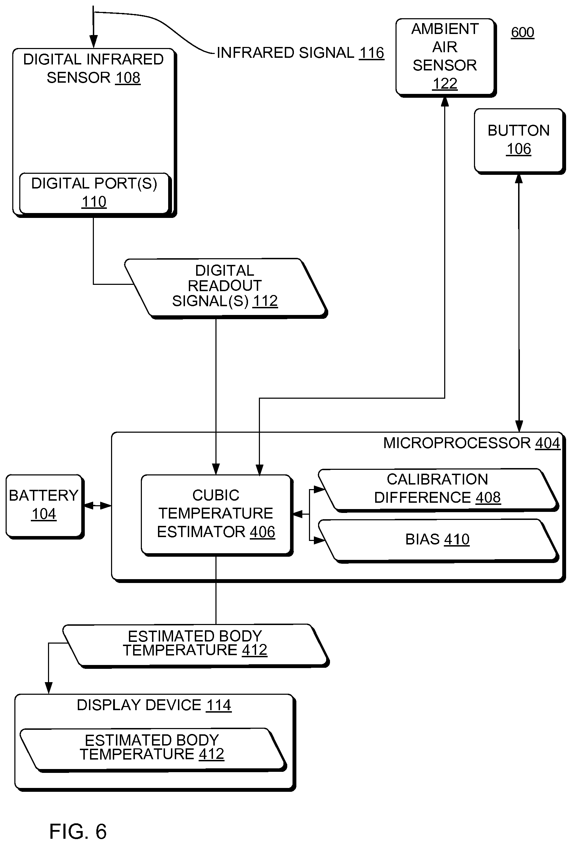

FIG. 6 is a block diagram of apparatus 600 to estimate a body core temperature from an external source point from a digital infrared sensor, according to an implementation. The apparatus 600 includes a battery 104, a single button 106, a display device 114, a digital infrared sensor 108 and an ambient air sensor 122 that are operably coupled to the microprocessor 404. The microprocessor 404 is operable to receive a representation of an infrared signal 116 of the external source point from the digital infrared sensor 108. The microprocessor 404 includes a cubic temperature estimator 406 that is operable to estimate the body core temperature 412 of the subject from the representation of the electromagnetic energy of the external source point.

Non-Touch Cubic-Estimation Thermometer and Vital Sign Detection Apparatus Implementations

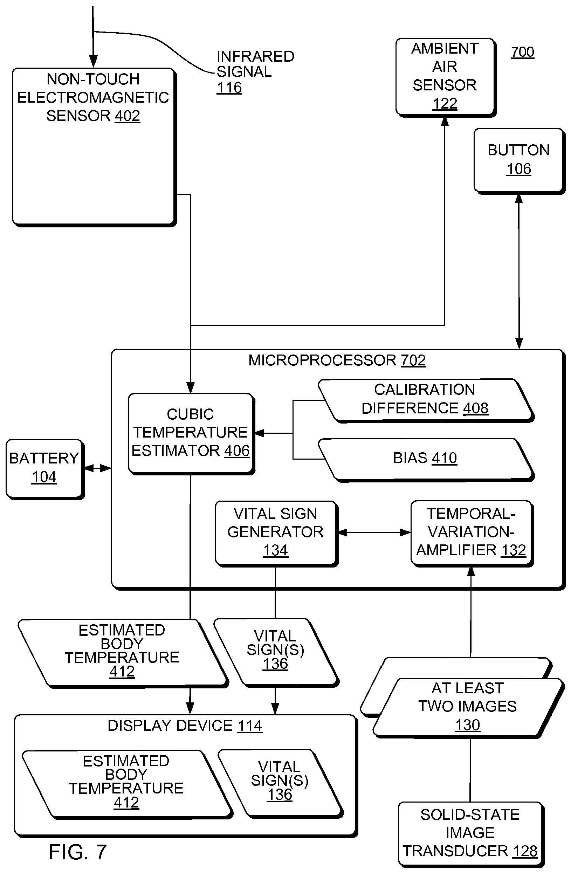

FIG. 7 is a block diagram of apparatus 700 that estimates a body core temperature of an external source point from a non-touch electromagnetic sensor and that detects vital-signs from images captured by a solid-state image transducer, according to an implementation. The apparatus 700 includes a battery 104, a single button 106, a display device 114, a non-touch electromagnetic sensor 702 and an ambient air sensor 122 that are operably coupled to the microprocessor 702. The microprocessor 702 is operable to receive a representation of an infrared signal 116 of the external source point from the non-touch electromagnetic sensor 702. The microprocessor 702 includes a cubic temperature estimator 406 that is operable to estimate the body core temperature 412 of the subject from the representation of the electromagnetic energy of the external source point. The apparatus 700 includes a solid-state image transducer 128 that is operably coupled to the microprocessor 702 and is operable to provide two or more images 130 to the microprocessor 702.

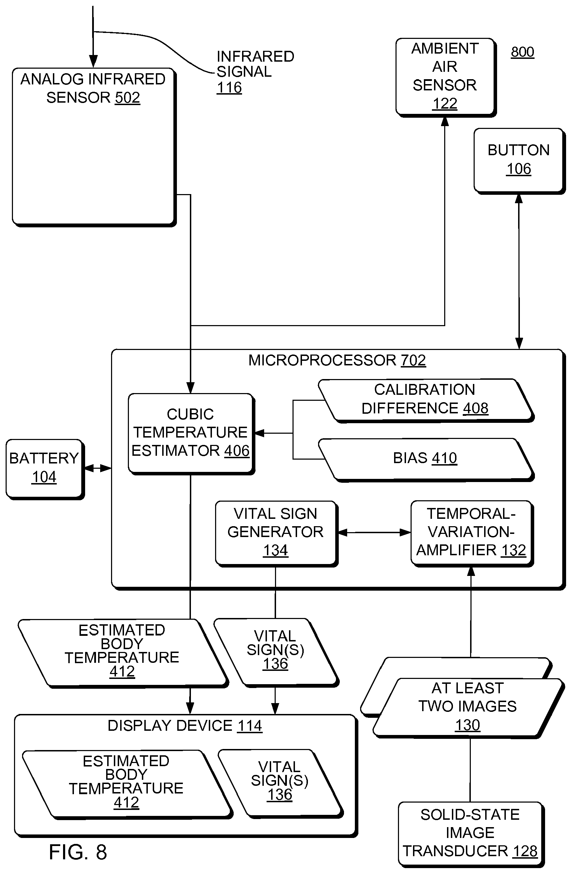

FIG. 8 is a block diagram of apparatus 800 that estimates a body core temperature of an external source point from an analog infrared sensor and that detects vital-signs from images captured by a solid-state image transducer, according to an implementation. The apparatus 800 includes a battery 104, a single button 106, a display device 114, an analog infrared sensor 502 and an ambient air sensor 122 that are operably coupled to the microprocessor 702. The microprocessor 702 is operable to receive a representation of an infrared signal 116 of the external source point from the analog infrared sensor 502. The microprocessor 702 includes a cubic temperature estimator 406 that is operable to estimate the body core temperature 412 of the subject from the representation of the electromagnetic energy of the external source point. The apparatus 800 includes a solid-state image transducer 128 that is operably coupled to the microprocessor 702 and is operable to provide two or more images 130 to the microprocessor 702.

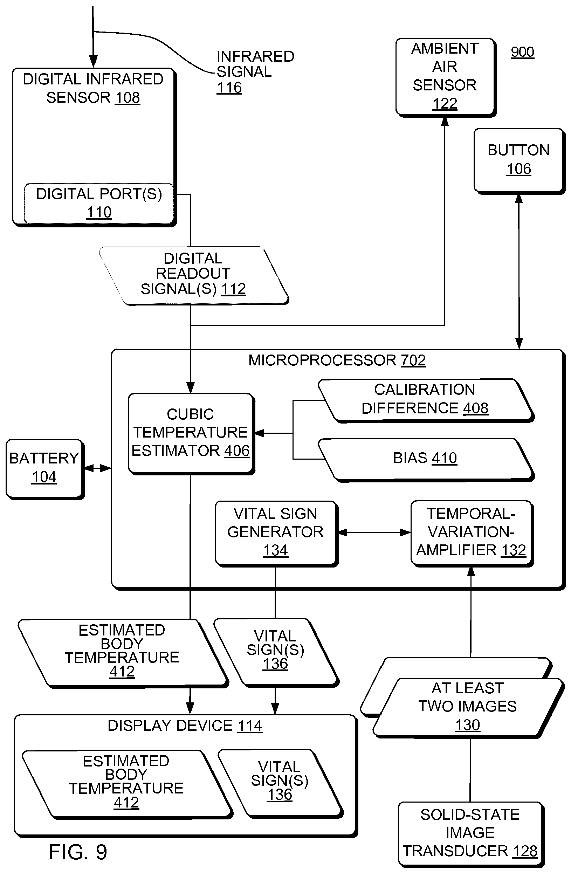

FIG. 9 is a block diagram of apparatus 900 that estimates a body core temperature of an external source point from a digital infrared sensor and that detects vital-signs from images captured by a solid-state image transducer, according to an implementation. The apparatus 900 includes a battery 104, a single button 106, a display device 114, a digital infrared sensor 108 and an ambient air sensor 122 that are operably coupled to the microprocessor 702. The microprocessor 702 is operable to receive a representation of an infrared signal 116 of the external source point from the digital infrared sensor 108. The microprocessor 702 includes a cubic temperature estimator 406 that is operable to estimate the body core temperature 412 of the subject from the representation of the electromagnetic energy of the external source point. The apparatus 900 includes a solid-state image transducer 128 that is operably coupled to the microprocessor 702 and is operable to provide two or more images 130 to the microprocessor 702.

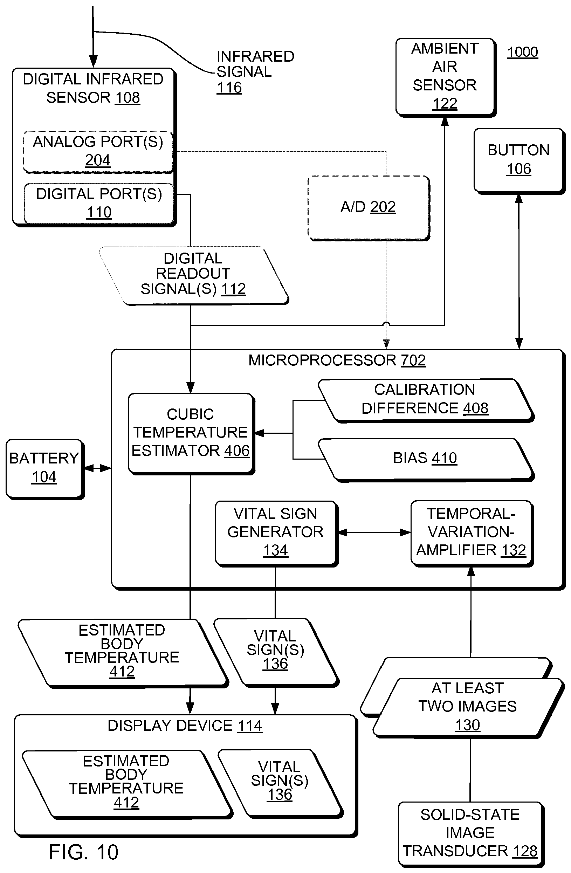

FIG. 10 is a block diagram of apparatus 1000 that estimates a body core temperature of an external source point from a digital infrared sensor, that does not include an analog-to-digital converter and that detects vital-signs from images captured by a solid-state image transducer, according to an implementation. The apparatus 1000 includes a battery 104, a single button 106, a display device 114, a digital infrared sensor 108 and an ambient air sensor 122 that are operably coupled to the microprocessor 702. The microprocessor 702 is operable to receive a representation of an infrared signal 116 of the external source point from the digital infrared sensor 108. The microprocessor 702 includes a cubic temperature estimator 406 that is operable to estimate the body core temperature 412 of the subject from the representation of the electromagnetic energy of the external source point. The apparatus 1000 includes a solid-state image transducer 128 that is operably coupled to the microprocessor 702 and is operable to provide two or more images 130 to the microprocessor 702. The apparatus 700 does not include an analog-to-digital (A/D) converter 202 operably coupled between the digital infrared sensor 108 and the microprocessor 702. The digital infrared sensor 108 also does not include analog readout ports 204. The dashed lines of the analog-to-digital (A/D) converter 202 and the analog readout ports 204 indicates absence of the A/D converter 202 and the analog readout ports 204 in the apparatus 700.

In regards to the structural relationship of the digital infrared sensor 108 and the microprocessor 102 in FIG. 1-3, 6 and 9-10, heat radiation on the digital infrared sensor 108 from any source such as the microprocessor 102 or heat sink, will distort detection of infrared energy by the digital infrared sensor 108. In order to prevent or at least reduce heat transfer between the digital infrared sensor 108 and the microprocessor 102, the apparatus in FIG. 1-3, 6 and 9-10 are low-powered devices and thus low heat-generating devices that are also powered by a battery 104; and that are only used for approximately a 5 second period of time for each measurement (1 second to acquire the temperature samples and generate the body core temperature result, and 4 seconds to display that result to the operator) so there is little heat generated by the apparatus in FIG. 1-3, 6 and 9-10 in active use.

The internal layout of the apparatus in FIG. 1-3, 6 and 9-10 minimizes as practically as possible the digital infrared sensor as far away in distance from all other components such the microprocessor (102, 404 or 702) within the practical limitations of the industrial design of the apparatus in FIG. 1-3, 6 and 9-10.

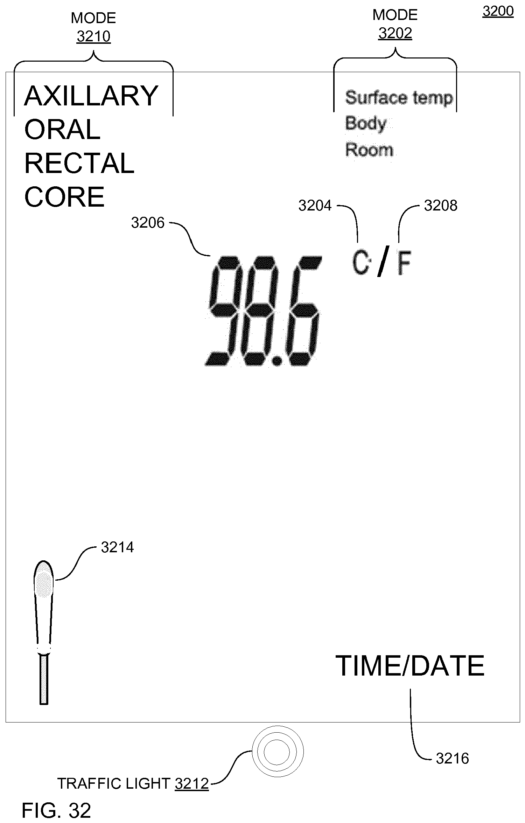

More specifically, to prevent or at least reduce heat transfer between the digital infrared sensor 108 and the microprocessor (102, 404 or 702) in some implementations the digital infrared sensor 108 is isolated on a separate PCB from the PCB that has the microprocessor (102, 404 or 702), as shown in FIG. 32, and the two PCBs are connected by only a connector that has 4 pins. The minimal connection of the single connector having 4 pins reduces heat transfer from the microprocessor (102, 404 or 702) to the digital infrared sensor 108 through the electrical connector and through transfer that would occur through the PCB material if the digital infrared sensor 108 and the microprocessor 102 were mounted on the same PCB.

In some implementations, the apparatus in FIG. 1-10 includes only one printed circuit board, in which case the printed circuit board includes the microprocessor 102 and the digital infrared sensor 108, non-touch electromagnetic sensor 402 or the analog infrared sensor 502 are mounted on the singular printed circuit board. In some implementations, the apparatus in FIG. 1-10 includes two printed circuit boards, such as a first printed circuit board and a second printed circuit board in which the microprocessor 102 is on the first printed circuit board and the digital infrared sensor 108, non-touch electromagnetic sensor 402 or the analog infrared sensor 502 are on the second printed circuit board. In some implementations, the apparatus in FIG. 1-10 includes only one display device 114, in which case the display device 114 includes not more than one display device 114. In some implementations, the display device 114 is a liquid-crystal diode (LCD) display device. In some implementations, the display device 114 is a light-emitting diode (LED) display device. In some implementations, the apparatus in FIG. 1-10 includes only one battery 104.

Digital Infrared Thermometer Method Implementations

In the previous section, apparatus of the operation of an implementation was described. In this section, the particular methods performed by FIG. 1-3, 6 and 9-10 are described by reference to a series of flowcharts.

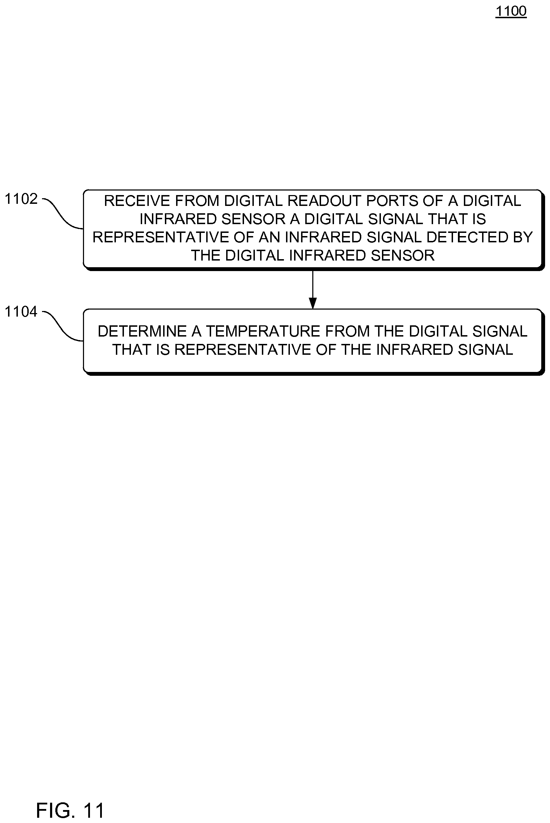

FIG. 11 is a flowchart of a method 1100 to determine a temperature from a digital infrared sensor, according to an implementation. Method 1100 includes receiving from the digital readout ports of a digital infrared sensor a digital signal that is representative of an infrared signal detected by the digital infrared sensor, at block 1102. No signal that is representative of the infrared signal is received from an analog infrared sensor.

Method 1100 also includes determining a temperature from the digital signal that is representative of the infrared signal, at block 1104.

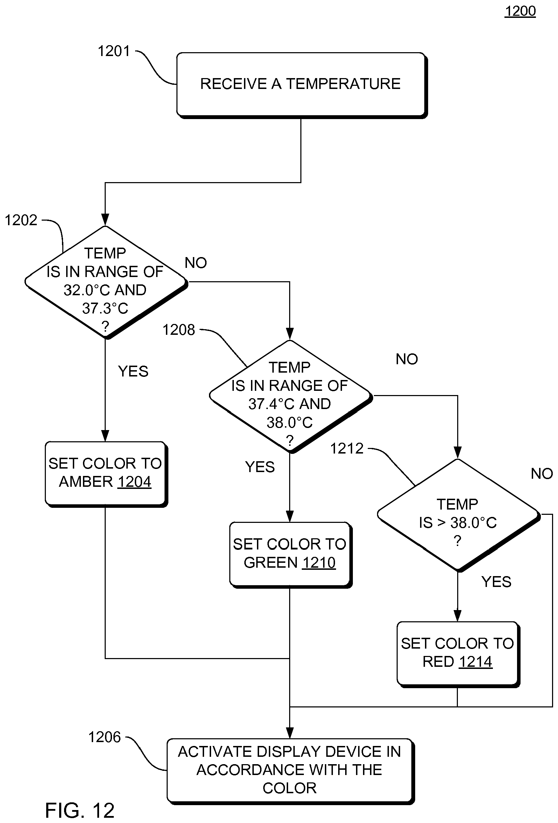

FIG. 12 is a flowchart of a method 1200 to display temperature color indicators, according to an implementation of three colors. Method 1200 provides color rendering in the color LED 3212 to indicate a general range of a temperature.

Method 1200 includes receiving a temperature (such as temperature 120 in FIG. 1), at block 1201.

Method 1200 also includes determining whether or not the temperature is in the range of 32.0°C and 37.3°C, at block 1202. If the temperature is in the range of 32.0°C and 37.3°C, then the color is set to 'amber' to indicate a temperature that is low, at block 1204 and the background of the color LED 3212 is activated in accordance with the color, at block 1206.

If the temperature is not the range of 32.0°C and 37.3°C, then method 1200 also includes determining whether or not the temperature is in the range of 37.4°C and 38.0°C, at block 1208. If the sensed temperature is in the range of 37.4°C and 38.0°C, then the color is set to green to indicate no medical concern, at block 1210 and the background of the color LED 3212 is activated in accordance with the color, at block 1206.

If the temperature is not the range of 37.4°C and 38.0°C, then method 1200 also includes determining whether or not the temperature is over 38.0°C, at block 1212. If the temperature is over 38.0°C, then the color is set to 'red' to indicate alert, at block 1212 and the background of the color LED 3212 is activated in accordance with the color, at block 1206.

Method 1200 assumes that temperature is in gradients of 10ths of a degree. Other temperature range boundaries are used in accordance with other gradients of temperature sensing.

In some implementations, some pixels in the color LED 3212 are activated as an amber color when the temperature is between 36.3°C and 37.3°C (97.3°F to 99.1°F), some pixels in the color LED 3212 are activated as a green when the temperature is between 37.4°C and 37.9°C (99.3°F to 100.2°F), some pixels in the color LED 3212 are activated as a red color when the temperature is greater than 38°C (100.4°F). In some implementations, the color LED 3212 is a backlit LCD screen 302 in FIG. 3 (which is easy to read in a dark room) and some pixels in the color LED 3212 are activated (remain lit) for about 5 seconds after the single button 106 is released. After the color LED 3212 has shut off, another temperature reading can be taken by the apparatus. The color change of the color LED 3212 is to alert the operator of the apparatus of a potential change of body temperature of the human or animal subject. The temperature reported on the display can be used for treatment decisions.

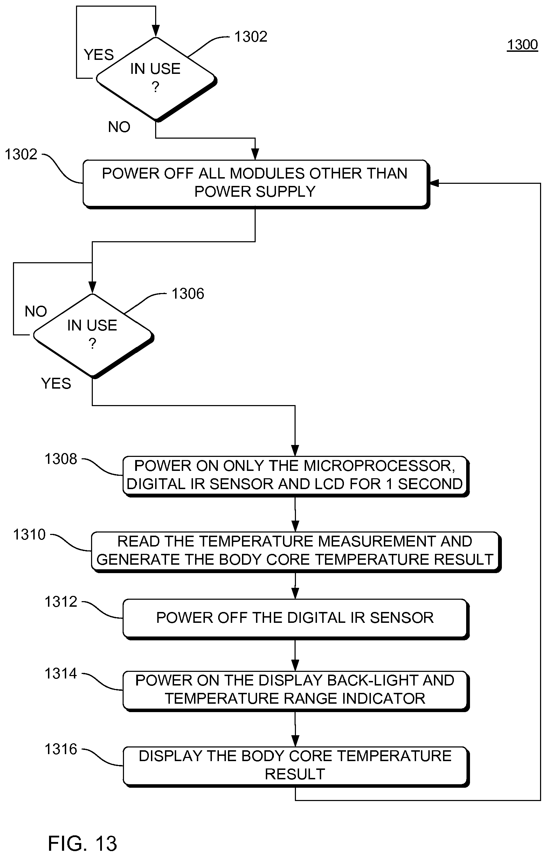

FIG. 13 is a flowchart of a method 1300 to manage power in a non-touch device having a digital infrared sensor, according to an implementation. The method 1300 manages power in the device, such as non-touch biologic detectors and thermometers in FIG. 1-10, the non-touch thermometer 3300 in FIG. 33, the hand-held device 3000 in FIG. 30 and/or the computer 3100 in FIG. 31 in order to reduce heat pollution in the digital infrared sensor.

To prevent or at least reduce heat transfer between the digital infrared sensor 108 and the microprocessor 102, microprocessor 404, microprocessor 3304 In FIG. 33, main processor 3002 in FIG. 30 or processing unit 3104 in FIG. 31, the components of the non-touch biologic detectors 100, 200 and 300 in FIG. 1-10, the non-touch thermometer 3300 in FIG. 33, the hand-held device 3000 in FIG. 30 and/or the computer 3100 in FIG. 31 are power controlled, i.e. the non-touch biologic detectors 100, 200 and 300 in FIG. 1-10, the non-touch thermometer 3300 in FIG. 33, the hand-held device 3000 in FIG. 30 and/or the computer 3100 in FIG. 31 turn sub-systems on and off, and the components are only activated when needed in the measurement and display process, which reduces power consumption and thus heat generation by the microprocessor 102 , microprocessor 3304 In FIG. 33, main processor 3002 in FIG. 30 or processing unit 3104 in FIG. 31, of the non-touch biologic detectors 100, 200 and 300 in FIG. 1-10, the non-touch thermometer 3300 in FIG. 33, the hand-held device 3000 in FIG. 30 and/or the computer 3100 in FIG. 31, respectively. When not in use, at block 1302, the non-touch biologic detectors 100, 200 and 300 in FIG. 1-10, the non-touch thermometer 3300 in FIG. 33, the hand-held device 3000 in FIG. 30 and/or the computer 3100 in FIG. 31 are completely powered-off, at block 1304 (including the main PCB having the microprocessor 102, microprocessor 404, microprocessor 3304 In FIG. 33, main processor 3002 in FIG. 30 or processing unit 3104 in FIG. 31, and the sensor PCB having the digital infrared sensor 108) and not drawing any power, other than a power supply, i.e. a boost regulator, which has the effect that the non-touch biologic detectors 100, 200 and 300 in FIG. 1-10, the non-touch thermometer 3300 in FIG. 33, the hand-held device 3000 in FIG. 30 and/or the computer 3100 in FIG. 31 draw only drawing micro-amps from the battery 104 while in the off state, which is required for the life time requirement of 3 years of operation, but which also means that in the non-use state there is very little powered circuitry in the non-touch biologic detectors 100, 200 and 300 in FIG. 1-10, the non-touch thermometer 3300 in FIG. 33, the hand-held device 3000 in FIG. 30 and/or the computer 3100 in FIG. 31 and therefore very little heat generated in the non-touch biologic detectors 100, 200 and 300 in FIG. 1-10, the non-touch thermometer 3300 in FIG. 33, the hand-held device 3000 in FIG. 30 and/or the computer 3100 in FIG. 31.

When the non-touch biologic detectors 100, 200 and 300 in FIG. 1-10, the non-touch thermometer 3300 in FIG. 33, the hand-held device 3000 in FIG. 30 and/or the computer 3100 in FIG. 31 are started by the operator, at block 1306, only the microprocessor 102, microprocessor 404, microprocessor 3304 In FIG. 33, main processor 3002 in FIG. 30 or processing unit 3104 in FIG. 31, digital infrared sensor 108, and low power LCD (e.g. display device 114) are turned on for the first 1 second, at block 1308, to take the temperature measurement via the digital infrared sensor 108 and generate the body core temperature result via the microprocessor 102 in FIG. 1-10, microprocessor 3304 in FIG. 33, main processor 3002 in FIG. 30 or processing unit 3104 in FIG. 31, at block 1310. In this way, the main heat generating components (the LCD 114, the main PCB having the microprocessor 102 and the sensor PCB having the digital infrared sensor 108), the display back-light and the temperature range indicator (i.e. the traffic light indicator 3212) are not on and therefore not generating heat during the critical start-up and measurement process, no more than 1 second. After the measurement process of block 1310 has been completed, the digital infrared sensor 108 is turned off, at block 1312, to reduce current usage from the batteries and heat generation, and also the display back-light and temperature range indicators are turned on, at block 1314.

The measurement result is displayed for 4 seconds, at block 1316, and then the non-touch biologic detectors 100, 200 and 300 in FIG. 1-10, the non-touch thermometer 3300 in FIG. 33, the hand-held device 3000 in FIG. 30 and/or the computer 3100 in FIG. 31 are put in low power-off state, at block 1318.

In some implementations of methods and apparatus of FIG. 1-37 an operator can take the temperature of a subject at multiple locations on a patient and from the temperatures at multiple locations to determine the temperature at a number of other locations of the subject. The multiple source points of which the electromagnetic energy is sensed are mutually exclusive to the location of the correlated temperature. In one example, the carotid artery source point on the subject and a forehead source point are mutually exclusive to the core temperature of the subject, an axillary temperature of the subject, a rectal temperature of the subject and an oral temperature of the subject.

The correlation of action can include a calculation based on Formula 1:

$$T_{body} = |f_{stb}(T_{surface\ temp} + f_{ntc}(T_{ntc})) + F4_{body}|$$

Formula 1

where $T_{body}$ is the temperature of a body or subject

where $f_{stb}$ is a mathematical formula of a surface of a body

where $f_{ntc}$ is mathematical formula for ambient temperature reading

where $T_{surface\,temp}$ is a surface temperature determined from the sensing.

where $T_{ntc}$ is an ambient air temperature reading

where $F4_{body}$ is a calibration difference in axillary mode, which is stored or set in a memory of the apparatus either during manufacturing or in the field. The apparatus also sets, stores and retrieves $F4_{oral}$, $F4_{core}$, and $F4_{rectal}$ in the memory.

$f_{ntc}(T_{ntc})$ is a bias in consideration of the temperature sensing mode. For example $f_{axillary}(T_{axillary})$ = 0.2 °C, $f_{oral}(T_{oral})$ = 0.4 °C, $f_{rectal}(T_{rectal})$ = 0.5 °C and $f_{core}(T_{core})$ = 0.3 °C.

In some implementations of determining a correlated body temperature of carotid artery by biasing a sensed temperature of a carotid artery, the sensed temperature is biased by +0.5 °C to yield the correlated body temperature. In another example, the sensed temperature is biased by -0.5 °C to yield the correlated body temperature. An example of correlating body temperature of a carotid artery follows:

$f_{ntc}(T_{ntc})$ = 0.2 °C when $T_{ntc}$ =26.2 °C as retrieved from a data table for body sensing mode.
assumption: $T_{surface\,temp}$ = 37.8 °C

$$T_{surface\,temp} + f_{ntc}(T_{ntc}) = 37.8\ °C + 0.2\ °C = 38.0\ °C$$

$$f_{stb}(T_{surface\,temp} + f_{ntc}(T_{ntc})) = 38\ °C + 1.4\ °C = 39.4\ °C$$

assumption: $F4_{body}$ = 0.5 °C

$$T_{body} = |f_{stb}(T_{surface\,temp} + f_{ntc}(T_{ntc}))+F4_{body}| = |39.4\ °C +0.5\ C\,| = 39.9\ °C$$

The correlated temperature for the carotid artery is 40.0 °C.

In an example of correlating temperature of a plurality of external locations, such as a forehead and a carotid artery to an axillary temperature, first a forehead temperature is calculated using formula 1 as follows:

$f_{ntc}(T_{ntc})$ = 0.2 °C when $T_{ntc}$ =26.2 °C as retrieved from a data table for axillary sensing mode.
assumption: $T_{surface\,temp}$ = 37.8 °C

$$T_{surface\,temp} + f_{ntc}(T_{ntc}) = 37.8\ °C + 0.2\ °C = 38.0\ °C$$

$$f_{stb}(T_{surface\,temp} + f_{ntc}(T_{ntc})) = 38\ °C + 1.4\ °C = 39.4\ °C$$

assumption: $F4_{body}$ = 0 °C

$$T_{body} = |f_{stb}(T_{surface\,temp} + f_{ntc}(T_{ntc}))+F4b_{ody}| = |39.4\ °C +0\ C\,| = 39.4\ °C$$

And second, a carotid temperature is calculated using formula 1 as follows:

$f_{ntc}(T_{ntc})$ = 0.6 °C when $T_{ntc}$ =26.4 °C as retrieved from a data table.
assumption: $T_{surface\,temp}$ = 38.0 °C

$$T_{surface\,temp} + f_{ntc}(T_{ntc}) = 38.0\ °C + 0.6\ °C = 38.6\ °C$$

$$f_{stb}(T_{surface\,temp} + f_{ntc}(T_{ntc})) = 38.6\ °C + 1.4\ C = 40.0\ °C$$

assumption: $F4_{body}$ = 0 °C

$$T_{body} = |f_{stb}(T_{surface\,temp} + f_{ntc}(T_{ntc}))+F4_{body}| = |40.0\ °C +0\ C\,| = 40.0\ °C$$

Thereafter the correlated temperature for the forehead (39.4 °C) and the correlated temperature for the carotid artery (40.0 °C) are averaged, yielding the final result of the scan of the forehead and the carotid artery as 39.7 °C.

Vital Sign Motion Amplification Apparatus Implementations

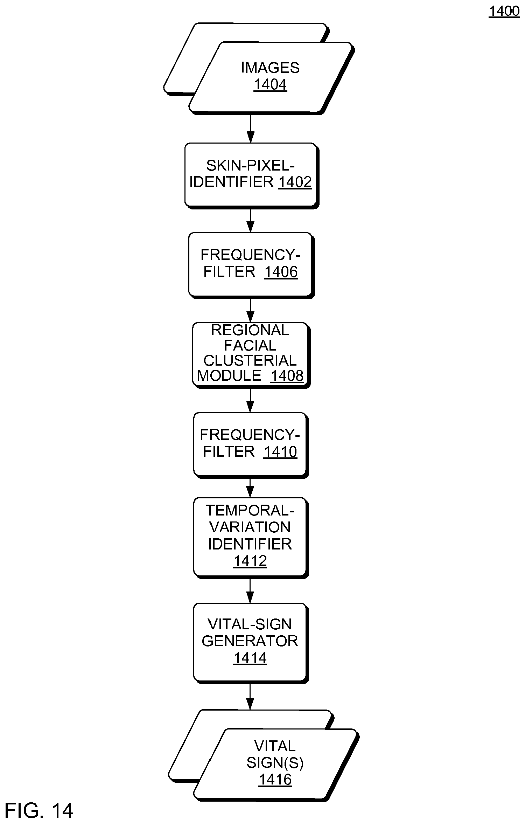

Apparatus in FIG. 14-22 use spatial and temporal signal processing to generate vital signs from a series of digital images.

FIG. 14 is a block diagram of an apparatus 1400 of motion amplification, according to an implementation. Apparatus 1400 analyzes the temporal and spatial variations in digital images of an animal subject in order to generate and communicate biological vital signs.

In some implementations, apparatus 1400 includes a skin-pixel-identifier 1402 that identifies pixel values that are representative of the skin in two or more images 1404. In some implementations the images 1404 are frames of a video. The skin-pixel-identifier 1402 performs block 2302 in FIG. 23. Some implementations of the skin-pixel-identifier 1402 perform an automatic seed point based clustering process on the two or more images 1404. In some implementations, apparatus 1400 includes a frequency filter 1406 that receives the output of the skin-pixel-identifier 1402 and applies a frequency filter to the output of the skin-pixel-identifier 1402. The frequency filter 1406 performs block 2304 in FIG. 23 to process the images 1404 in the frequency domain. In implementations where the apparatus in FIG. 14-22 or the methods in FIG. 23-27 are implemented on non-touch biologic detectors and thermometerss in FIG. 1-10, the images 1404 in FIG. 14-22 are the images 130 in FIG. 1-10. In some implementations the apparatus in FIG. 14-22 or the methods in FIG. 23-27 are implemented on the hand-held device 3000 in FIG. 30.

In some implementations, apparatus 1400 includes a regional facial clusterial module 1408 that applies spatial clustering to the output of the frequency filter 1406. The regional facial clusterial module 1408 performs block 2306 in FIG. 23. In some implementations the regional facial clusterial module 1408 includes fuzzy clustering, k-means clustering, expectation-maximization process, Ward's apparatus or seed point based clustering.

In some implementations, apparatus 1400 includes a frequency-filter 1410 that applies a frequency filter to the output of the regional facial clusterial module 1408. The frequency-filter 1410 performs block 2308 in FIG. 23. In some implementations, the frequency-filter 1410 is a one-dimensional spatial Fourier Transform, a high pass filter, a low pass filter, a bandpass filter or a weighted bandpass filter. Some implementations of frequency-filter 1410 includes de-noising (e.g. smoothing of the data with a Gaussian filter). The skin-pixel-identifier 1402, the frequency filter 1406, the regional facial clusterial module 1408 and the frequency-filter 1410 amplify temporal variations (as a temporal-variation-amplifier) in the two or more images 1404.

In some implementations, apparatus 1400 includes a temporal-variation identifier 1412 that identifies temporal variation of the output of the frequency-filter 1410. Thus, the temporal variation represents temporal variation of the images 1404. The temporal-variation identifier 1412 performs block 2310 in FIG. 23.

In some implementations, apparatus 1400 includes a vital-sign generator 1414 that generates one or more vital sign(s) 1416 from the temporal variation. The vital sign(s) 1416 are displayed for review by a healthcare worker or stored in a volatile or nonvolatile memory for later analysis, or transmitted to other devices for analysis.

Fuzzy clustering is a class of processes for cluster analysis in which the allocation of data points to clusters is not "hard" (all-or-nothing) but "fuzzy" in the same sense as fuzzy logic. Fuzzy logic being a form of many-valued logic which with reasoning that is approximate rather than fixed and exact. In fuzzy clustering, every point has a degree of belonging to clusters, as in fuzzy logic, rather than belonging completely to just one cluster. Thus, points on the edge of a cluster, may be in the cluster to a lesser degree than points in the center of cluster. An overview and comparison of different fuzzy clustering processes is available. Any point x has a set of coefficients giving the degree of being in the kth cluster $w_k(x)$. With fuzzy c-means, the centroid of a cluster is the mean of all points, weighted by a degree of belonging of each point to the cluster:

$$c_k = \frac{\sum_x w_k(x)^m x}{\sum_x w_k(x)^m}.$$

The degree of belonging, $w_k(x)$, is related inversely to the distance from x to the cluster center as calculated on the previous pass. The degree of belonging, $w_k(x)$ also depends on a parameter m that controls how much weight is given to the closest center.

k-means clustering is a process of vector quantization, originally from signal processing, that is popular for cluster analysis in data mining, k-means clustering partitions n observations into k clusters in which each observation belongs to the cluster with the nearest mean, serving as a prototype of the cluster. This results in a partitioning of the data space into Voronoi cells. A Voronoi Cell being a region within a Voronoi Diagram that is a set of points which is specified beforehand. A Voronoi Diagram is a technique of dividing space into a number of regions. k-means clustering uses cluster centers to model the data and tends to find clusters of comparable spatial extent, like K-means clustering, but each data point has a fuzzy degree of belonging to each separate cluster.

An expectation-maximization process is an iterative process for finding maximum likelihood or maximum a posteriori (MAP) estimates of parameters in statistical models, where the model depends on unobserved latent variables. The expectation-maximization iteration alternates between performing an expectation step, which creates a function for the expectation of the log-likelihood evaluated using the current estimate for the parameters, and a maximization step, which computes parameters maximizing the expected log-likelihood found on the expectation step. These parameter-estimates are then used to determine the distribution of the latent variables in the next expectation step.

The expectation maximization process seeks to find the maximization likelihood expectation of the marginal likelihood by iteratively applying the following two steps:

1. Expectation step (E step): Calculate the expected value of the log likelihood function, with respect to the conditional distribution of Z given X under the current estimate of the parameters $\theta^{(t)}$:

$$Q(\boldsymbol{\theta}|\boldsymbol{\theta}^{(t)}) = \mathrm{E}_{\mathbf{Z}|\mathbf{X},\boldsymbol{\theta}^{(t)}}\left[\log L(\boldsymbol{\theta}; \mathbf{X}, \mathbf{Z})\right]$$

2. Maximization step (M step): Find the parameter that maximizes this quantity:

$$\boldsymbol{\theta}^{(t+1)} = \arg\max_{\boldsymbol{\theta}} Q(\boldsymbol{\theta}|\boldsymbol{\theta}^{(t)})$$

Note that in typical models to which expectation maximization is applied:

1. The observed data points X may be discrete (taking values in a finite or countably infinite set) or continuous (taking values in an uncountably infinite set). There may in fact be a vector of observations associated with each data point.
2. The missing values (aka latent variables) Z are discrete, drawn from a fixed number of values, and there is one latent variable per observed data point.
3. The parameters are continuous, and are of two kinds: Parameters that are associated with all data points, and parameters associated with a particular value of a latent variable (i.e. associated with all data points whose corresponding latent variable has a particular value).

The Fourier Transform is an important image processing tool which is used to decompose an image into its sine and cosine components. The output of the transformation represents the image in the *Fourier* or frequency domain, while the input image is the spatial domain equivalent. In the Fourier domain image, each point represents a particular frequency contained in the spatial domain image.

The Discrete Fourier Transform is the sampled Fourier Transform and therefore does not contain all frequencies forming an image, but only a set of samples which is large enough to fully describe the spatial domain image. The number of frequencies corresponds to the number of pixels in the spatial domain image, i.e. the image in the spatial and Fourier domains are of the same size.

For a square image of size N×N, the two-dimensional DFT is given by:

$$F(k, l) = \sum_{i=0}^{N-1} \sum_{j=0}^{N-1} f(i, j) \, e^{-\iota 2\pi\left(\frac{ki}{N} + \frac{lj}{N}\right)}$$

where f(a,b) is the image in the spatial domain and the exponential term is the basis function corresponding to each point F(k,l) in the Fourier space. The equation can be interpreted as: the value of each point F(k,l) is obtained by multiplying the spatial image with the corresponding base function and summing the result.

The basis functions are sine and cosine waves with increasing frequencies, i.e. F(0,0) represents the DC-component of the image which corresponds to the average brightness and F(N-1,N-1) represents the highest frequency.

A high-pass filter (HPF) is an electronic filter that passes high-frequency signals but attenuates (reduces the amplitude of) signals with frequencies lower than the cutoff frequency. The actual amount of attenuation for each frequency varies from filter to filter. A high-pass filter is usually modeled as a linear time-invariant system. A high-pass filter can also be used in conjunction with a low-pass filter to make a bandpass filter. The simple first-order electronic high-pass filter is implemented by placing an input voltage across the series combination of a capacitor and a resistor and using the voltage across the resistor as an output. The product of the resistance and capacitance (RxC) is the time constant (T); the product is inversely proportional to the cutoff frequency $f_c$, that is:

$$f_c = \frac{1}{2\pi\tau} = \frac{1}{2\pi RC},$$

where $f_c$ is in hertz, $\tau$ is in seconds, $R$ is in ohms, and $C$ is in farads.

A low-pass filter is a filter that passes low-frequency signals and attenuates (reduces the amplitude of) signals with frequencies higher than the cutoff frequency. The actual amount of attenuation for each frequency varies depending on specific filter design. Low-pass filters are also known as high-cut filter, or treble cut filter in audio applications. A low-pass filter is the opposite of a high-pass filter. Low-pass filters provide a smoother form of a signal, removing the short-term fluctuations, and leaving the longer-term trend. One simple low-pass filter circuit consists of a resistor in series with a load, and a capacitor in parallel with the load. The capacitor exhibits reactance, and blocks low-frequency signals, forcing the low-frequency signals through the load instead. At higher frequencies the reactance drops, and the capacitor effectively functions as a short circuit. The combination of resistance and capacitance gives the time constant of the filter. The break frequency, also called the turnover frequency or cutoff frequency (in hertz), is determined by the time constant.

A band-pass filter is a device that passes frequencies within a certain range and attenuates frequencies outside that range. These filters can also be created by combining a low-pass filter with a high-pass filter. Bandpass is an adjective that describes a type of filter or filtering process; bandpass is distinguished from passband, which refers to the actual portion of affected spectrum. Hence, a dual bandpass filter has two passbands. A bandpass signal is a signal containing a band of frequencies not adjacent to zero frequency, such as a signal that comes out of a bandpass filter.

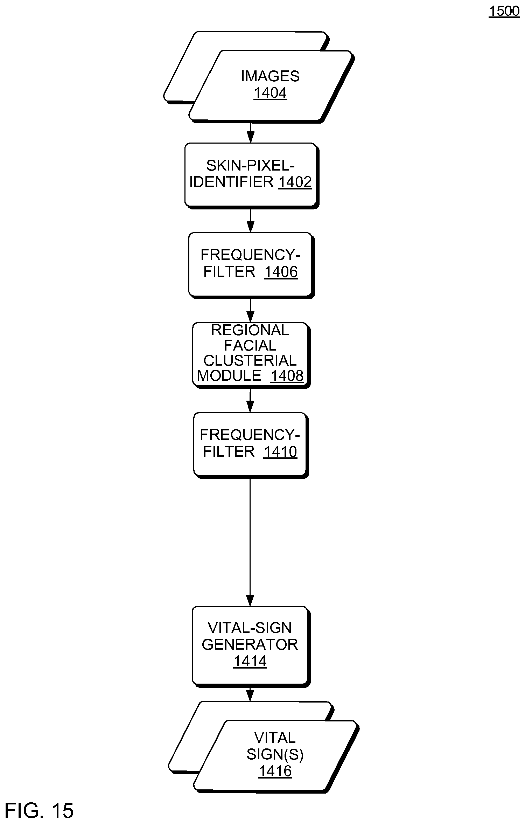

FIG. 15 is a block diagram of an apparatus 1500 of motion amplification, according to an implementation. Apparatus 1500 analyzes the temporal and spatial variations in digital images of an animal subject in order to generate and communicate biological vital signs.

In some implementations, apparatus 1500 includes a skin-pixel-identifier 1402 that identifies pixel values that are representative of the skin in two or more images 1404. The skin-pixel-identifier 1402 performs block 2302 in FIG. 23. Some implementations of the skin-pixel-identifier 1402 performs an automatic seed point based clustering process on the least two images 1404.

In some implementations, apparatus 1500 includes a frequency filter 1406 that receives the output of the skin-pixel-identifier 1402 and applies a frequency filter to the output of the skin-pixel-identifier 1402. The frequency filter 1406 performs block 2304 in FIG. 23 to process the images 1404 in the frequency domain.

In some implementations, apparatus 1500 includes a regional facial clusterial module 1408 that applies spatial clustering to the output of the frequency filter 1406. The regional facial clusterial module 1408 performs block 2306 in FIG. 23. In some implementations the regional facial clusterial module 1408 includes fuzzy clustering, k-means clustering, expectation-maximization process, Ward's apparatus or seed point based clustering.

In some implementations, apparatus 1500 includes a frequency-filter 1410 that applies a frequency filter to the output of the regional facial clusterial module 1408, to generate a temporal variation. The frequency-filter 1410 performs block 2308 in FIG. 23. In some implementations, the frequency-filter 1410 is a one-dimensional spatial Fourier Transform, a high pass filter, a low pass filter, a bandpass filter or a weighted bandpass filter. Some implementations of frequency-filter 1410 includes de-noising (e.g. smoothing of the data with a Gaussian filter).The skin-pixel-identifier 1402, the frequency filter 1406, the regional facial clusterial module 1408 and the frequency-filter 1410 amplify temporal variations in the two or more images 1404.

In some implementations, apparatus 1500 includes a vital-sign generator 1414 that generates one or more vital sign(s) 1416 from the temporal variation. The vital sign(s) 1416 are displayed for review by a healthcare worker or stored in a volatile or nonvolatile memory for later analysis, or transmitted to other devices for analysis.

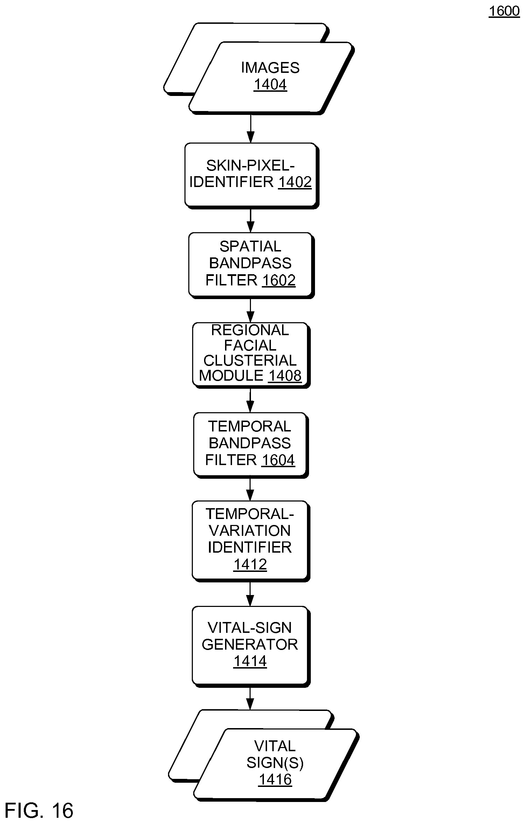

FIG. 16 is a block diagram of an apparatus 1600 of motion amplification, according to an implementation. Apparatus 1600 analyzes the temporal and spatial variations in digital images of an animal subject in order to generate and communicate biological vital signs.

In some implementations, apparatus 1600 includes a skin-pixel-identifier 1402 that identifies pixel values that are representative of the skin in two or more images 1404. The skin-pixel-identifier 1402 performs block 2302 in FIG. 23. Some implementations of the skin-pixel-identifier 1402 performs an automatic seed point based clustering process on the least two images 1404.

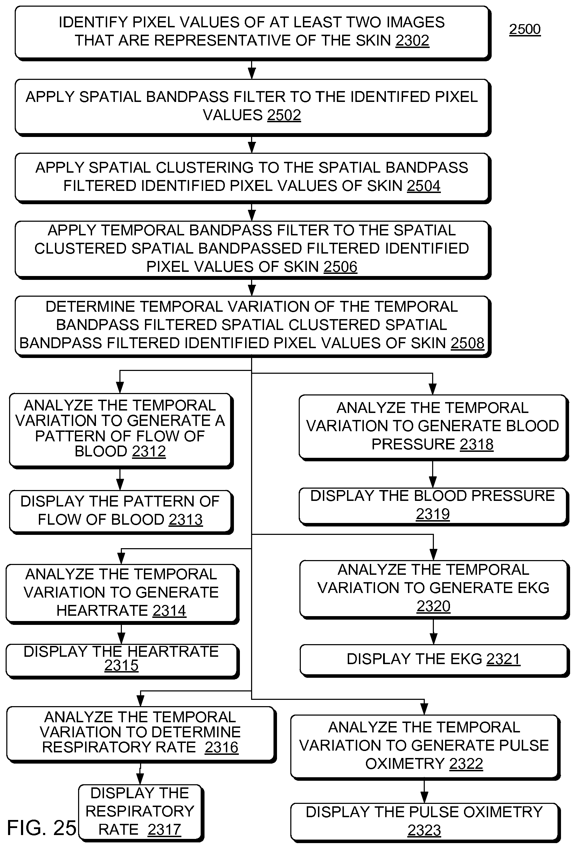

In some implementations, apparatus 1600 includes a spatial bandpass filter 1602 that receives the output of the skin-pixel-identifier 1402 and applies a spatial bandpass filter to the output of the skin-pixel-identifier 1402. The spatial bandpass filter 1602 performs block 2502 in FIG. 25 to process the images 1404 in the spatial domain.

In some implementations, apparatus 1600 includes a regional facial clusterial module 1408 that applies spatial clustering to the output of the frequency filter 1406. The regional facial clusterial module 1408 performs block 2504 in FIG. 25. In some implementations the regional facial clusterial module 1408 includes fuzzy clustering, k-means clustering, expectation-maximization process, Ward's apparatus or seed point based clustering.

In some implementations, apparatus 1600 includes a temporal bandpass filter 1604 that applies a frequency filter to the output of the regional facial clusterial module 1408. The temporal bandpass filter 1604 performs block 2506 in FIG. 25. In some implementations, the temporal bandpass filter 1604 is a one-dimensional spatial Fourier Transform, a high pass filter, a low pass filter, a bandpass filter or a weighted bandpass filter. Some implementations of temporal bandpass filter 1604 includes de-noising (e.g. smoothing of the data with a Gaussian filter).

The skin-pixel-identifier 1402, the spatial bandpass filter 1602, the regional facial clusterial module 1408 and the temporal bandpass filter 1604 amplify temporal variations in the two or more images 1404.

In some implementations, apparatus 1600 includes a temporal-variation identifier 1412 that identifies temporal variation of the output of the frequency-filter 1410. Thus, the temporal variation represents temporal variation of the images 1404. The temporal-variation identifier 1412 performs block 2508 in FIG. 25.

In some implementations, apparatus 1600 includes a vital-sign generator 1414 that generates one or more vital sign(s) 1416 from the temporal variation. The vital sign(s) 1416 are displayed for review by a healthcare worker or stored in a volatile or nonvolatile memory for later analysis, or transmitted to other devices for analysis.

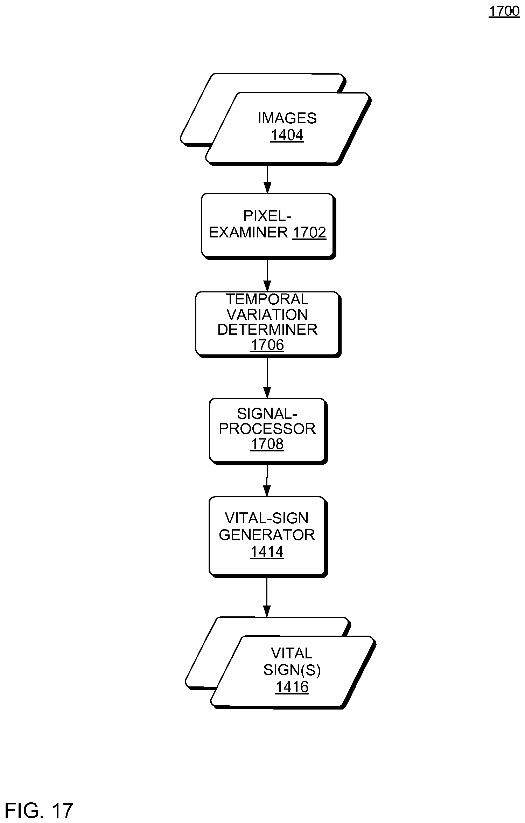

FIG. 17 is a block diagram of an apparatus 1700 of motion amplification, according to an implementation.

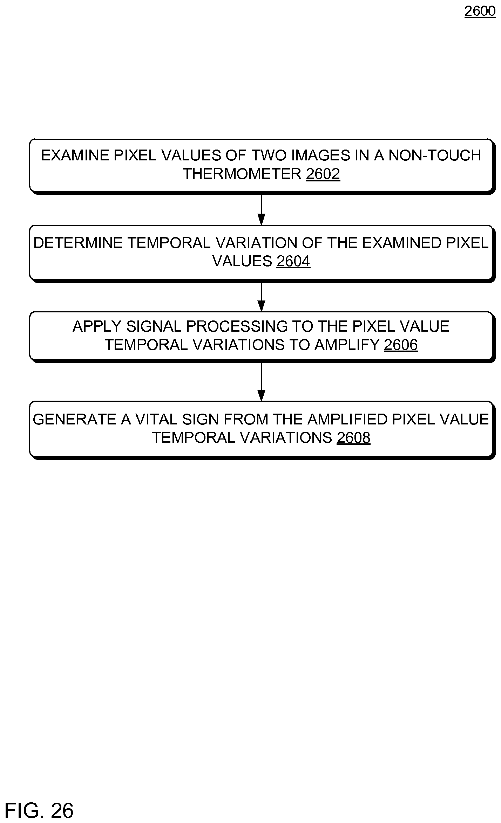

In some implementations, apparatus 1700 includes a pixel-examiner 1702 that examines pixel values of two or more images 1404. The pixel-examiner 1702 performs block 2602 in FIG. 26.

In some implementations, apparatus 1700 includes a temporal variation determiner 1706 that determines a temporal variation of examined pixel values. The temporal variation determiner 1706 performs block 2604 in FIG. 26.

In some implementations, apparatus 1700 includes a signal-processor 1708 that applies signal processing to the pixel value temporal variation, generating an amplified temporal variation. The signal-processor 1708 performs block 2606 in FIG. 26. The signal processing amplifies the temporal variation, even when the temporal variation is small. In some implementations, the signal processing performed by signal-processor 1708 is temporal bandpass filtering that analyzes frequencies over time. In some implementations, the signal processing performed by signal-processor 1708 is spatial processing that removes noise. Apparatus 1700 amplifies only small temporal variations in the signal-processing module.

In some implementations, apparatus 1600 includes a vital-sign generator 1414 that generates one or more vital sign(s) 1416 from the temporal variation. The vital sign(s) 1416 are displayed for review by a healthcare worker or stored in a volatile or nonvolatile memory for later analysis, or transmitted to other devices for analysis.

While apparatus 1700 can process large temporal variations, an advantage in apparatus 1700 is provided for small temporal variations. Therefore apparatus 1700 is most effective when the two or more images 1404 have small temporal variations between the two or more images 1404. In some implementations, a vital sign is generated from the amplified temporal variations of the two or more images 1404 from the signal-processor 1708.

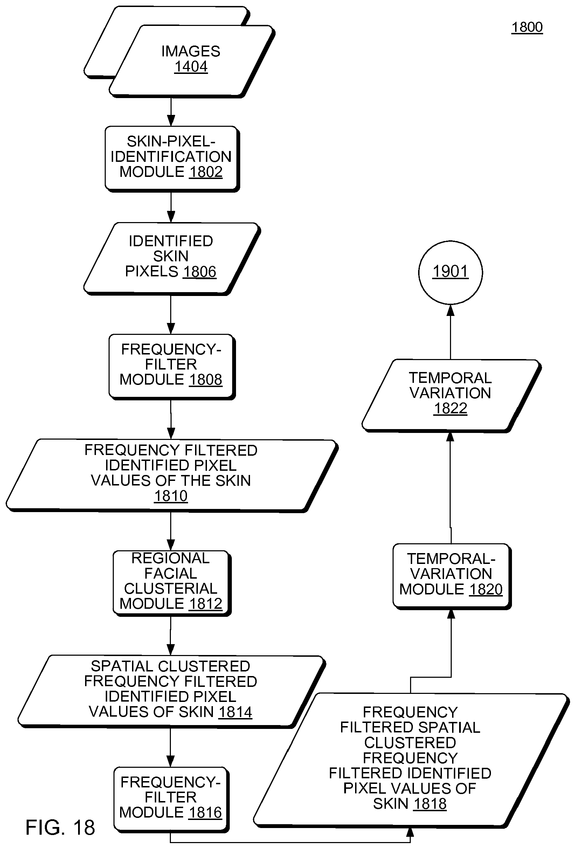

FIG. 18 is a block diagram of an apparatus 1800 of motion amplification, according to an implementation. Apparatus 1800 analyzes the temporal and spatial variations in digital images of an animal subject in order to generate and communicate biological vital signs.

In some implementations, apparatus 1800 includes a skin-pixel-identification module 1802 that identifies pixel values 1806 that are representative of the skin in two or more images 1804. The skin-pixel-identification module 1802 performs block 2302 in FIG. 23. Some implementations of the skin-pixel-identification module 1802 perform an automatic seed point based clustering process on the least two images 1804.

In some implementations, apparatus 1800 includes a frequency-filter module 1808 that receives the identified pixel values 1806 that are representative of the skin and applies a frequency filter to the identified pixel values 1806. The frequency-filter module 1808 performs block 2304 in FIG. 23 to process the images 1404 in the frequency domain. Each of the images 1404 is Fourier transformed, multiplied with a filter function and then re-transformed into the spatial domain. Frequency filtering is based on the Fourier Transform. The operator takes an image 1404 and a filter function in the Fourier domain. The image 1404 is then multiplied with the filter function in a pixel-by-pixel fashion using the formula:

$$G(k, l) = F(k, l)\, H(k, l)$$

where $F(k,l)$ is the input image 1404 of identified pixel values 1806 in the Fourier domain, $H(k,l)$ the filter function and $G(k,l)$ is the filtered image 1810. To obtain the resulting image in the spatial domain, $G(k,l)$ is re-transformed using the inverse Fourier Transform. In some implementations, the frequency-filter module 1808 is a two-dimensional spatial Fourier Transform, a high pass filter, a low pass filter, a bandpass filter or a weighted bandpass filter.

In some implementations, apparatus 1800 includes a spatial-cluster module 1812 that applies spatial clustering to the frequency filtered identified pixel values of skin 1810, generating spatial clustered frequency filtered identified pixel values of skin 1814. The spatial-cluster module 1812 performs block 2306 in FIG. 23. In some implementations the spatial-cluster module 1812 includes fuzzy clustering, k-means clustering, expectation-maximization process, Ward's apparatus or seed point based clustering.

In some implementations, apparatus 1800 includes a frequency-filter module 1816 that applies a frequency filter to the spatial clustered frequency filtered identified pixel values of skin 1814, which generates frequency filtered spatial clustered frequency filtered identified pixel values of skin 1818. The frequency-filter module 1816 performs block 2308 in FIG. 23. In some implementations, the frequency-filter module 1816 is a one-dimensional spatial Fourier Transform, a high pass filter, a low pass filter, a bandpass filter or a weighted bandpass filter. Some implementations of frequency-filter module 1816 includes de-noising (e.g. smoothing of the data with a Gaussian filter).

The skin-pixel-identification module 1802, the frequency-filter module 1808, the spatial-cluster module 1812 and the frequency-filter module 1816 amplify temporal variations in the two or more images 1404.

In some implementations, apparatus 1800 includes a temporal-variation module 1820 that determines temporal variation 1822 of the frequency filtered spatial clustered frequency filtered identified pixel values of skin 1818. Thus, temporal variation 1822 represents temporal variation of the images 1404. The temporal-variation module 1820 performs block 2310 in FIG. 23.

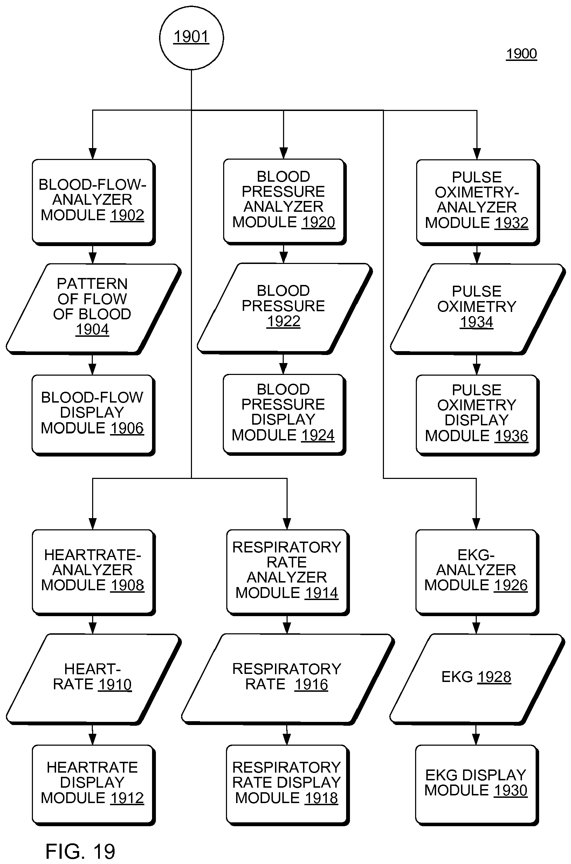

FIG. 19 is a block diagram of an apparatus 1900 to generate and present any one of a number of biological vital signs from amplified motion, according to an implementation.

In some implementations, apparatus 1900 includes a blood-flow-analyzer module 1902 that analyzes a temporal variation to generate a pattern of flow of blood 1904. One example of the temporal variation is temporal variation 1822 in FIG. 18. In some implementations, the pattern flow of blood 1904 is generated from motion changes in the pixels and the temporal variation of color changes in the skin of the images 1404. In some implementations, apparatus 1900 includes a blood-flow display module 1906 that displays the pattern of flow of blood 1904 for review by a healthcare worker.

In some implementations, apparatus 1900 includes a heartrate-analyzer module 1908 that analyzes the temporal variation to generate a heartrate 1910. In some implementations, the heartrate 1910 is generated from the frequency spectrum of the temporal signal in a frequency range for heart beats, such as (0-10 Hertz). In some implementations, apparatus 1900 includes a heartrate display module 1912 that displays the heartrate 1910 for review by a healthcare worker.

In some implementations, apparatus 1900 includes a respiratory rate-analyzer module 1914 that analyzes the temporal variation to determine a respiratory rate 1916. In some implementations, the respiratory rate 1916 is generated from the motion of the pixels in a frequency range for respiration (0-5 Hertz). In some implementations, apparatus 1900 includes respiratory rate display module 1918 that displays the respiratory rate 1916 for review by a healthcare worker.

In some implementations, apparatus 1900 includes a blood-pressure analyzer module 1920 that analyzes the temporal variation to a generate blood pressure 1922. In some implementations, the blood-pressure analyzer module 1920 generates the blood pressure 1922 by analyzing the motion of the pixels and the color changes based on a clustering process and potentially temporal data. In some implementations, apparatus 1900 includes a blood pressure display module 1924 that displays the blood pressure 1922 for review by a healthcare worker.

In some implementations, apparatus 1900 includes an EKG analyzer module 1926 that analyzes the temporal variation to generate an EKG 1928. In some implementations, apparatus 1900 includes an EKG display module 1930 that displays the EKG 1928 for review by a healthcare worker.

In some implementations, apparatus 1900 includes a pulse oximetry analyzer module 1932 that analyzes the temporal variation to generate pulse oximetry 1934. In some implementations, the pulse oximetry analyzer module 1932 generates the pulse oximetry 1934 by analyzing the temporal color changes based in conjunction with the k-means clustering process and potentially temporal data. In some implementations, apparatus 1900 includes a pulse oximetry display module 1936 that displays the pulse oximetry 1934 for review by a healthcare worker.

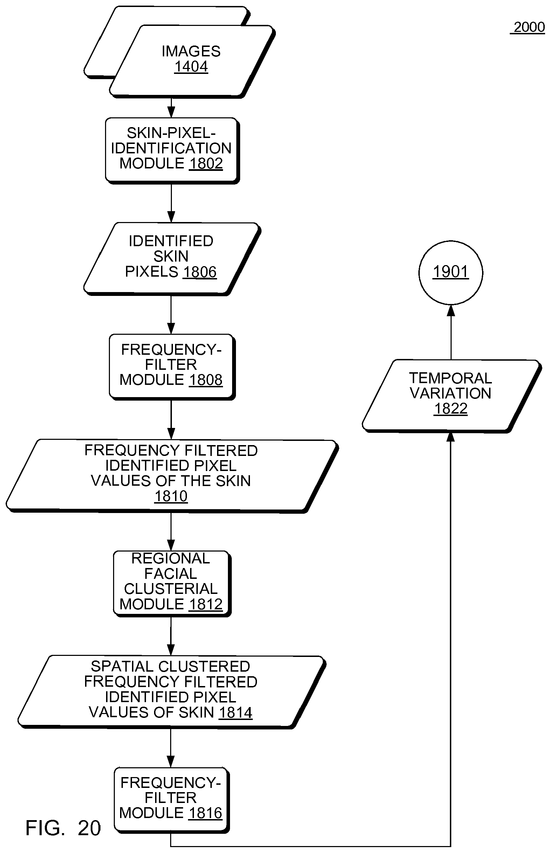

FIG. 20 is a block diagram of an apparatus 2000 of motion amplification, according to an implementation. Apparatus 2000 analyzes the temporal and spatial variations in digital images of an animal subject in order to generate and communicate biological vital signs.

In some implementations, apparatus 2000 includes a skin-pixel-identification module 1802 that identifies pixel values 1806 that are representative of the skin in two or more images 1404. The skin-pixel-identification module 1802 performs block 2302 in FIG. 23. Some implementations of the skin-pixel-identification module 1802 perform an automatic seed point based clustering process on the least two images 1404.

In some implementations, apparatus 2000 includes a frequency-filter module 1808 that receives the identified pixel values 1806 that are representative of the skin and applies a frequency filter to the identified pixel values 1806. The frequency-filter module 1808 performs block 2304 in FIG. 23 to process the images 1404 in the frequency domain.

Each of the images 1404 is Fourier transformed, multiplied with a filter function and then re-transformed into the spatial domain. Frequency filtering is based on the Fourier Transform. The operator takes an image 1404 and a filter function in the Fourier domain. The image 1404 is then multiplied with the filter function in a pixel-by-pixel fashion using the

[00243]        formula: $G(k, l) = F(k, l) H(k, l)$

where F(k,l) is the input image 1404 of identified pixel values 1806 in the Fourier domain, H(k,l) the filter function and G(k,l) is the filtered image 1810. To obtain the resulting image in the spatial domain, G(k,l) is re-transformed using the inverse Fourier Transform. In some implementations, the frequency-filter module 1808 is a two-dimensional spatial Fourier Transform, a high pass filter, a low pass filter, a bandpass filter or a weighted bandpass filter.

In some implementations, apparatus 2000 includes a spatial-cluster module 1812 that applies spatial clustering to the frequency filtered identified pixel values of skin 1810, generating spatial clustered frequency filtered identified pixel values of skin 1814. The spatial-cluster module 1812 performs block 2306 in FIG. 23. In some implementations the spatial clustering includes fuzzy clustering, k-means clustering, expectation-maximization process, Ward's apparatus or seed point based clustering.

In some implementations, apparatus 2000 includes a frequency-filter module 1816 that applies a frequency filter to the spatial clustered frequency filtered identified pixel values of skin 1814, which generates frequency filtered spatial clustered frequency filtered identified pixel values of skin 1818. The frequency-filter module 1816 performs block 2308 in FIG. 23 to generate a temporal variation 1822. In some implementations, the frequency-filter module 1816 is a one-dimensional spatial Fourier Transform, a high pass filter, a low pass filter, a bandpass filter or a weighted bandpass filter. Some implementations of the frequency-filter module 1816 includes de-noising (e.g. smoothing of the data with a Gaussian filter).The skin-pixel-identification module 1802, the frequency-filter module 1808, the spatial-cluster module 1812 and the frequency-filter module 1816 amplify temporal variations in the two or more images 1404.

The frequency-filter module 1816 is operably coupled to one of more modules in FIG. 19 to generate and present any one or a number of biological vital signs from amplified motion in the temporal variation 1822.

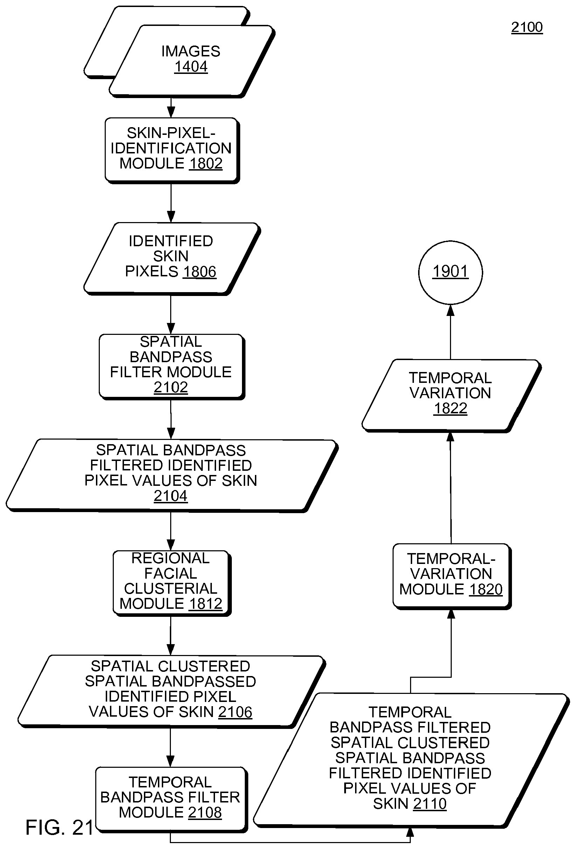

FIG. 21 is a block diagram of an apparatus 2100 of motion amplification, according to an implementation. Apparatus 2100 analyzes the temporal and spatial variations in digital images of an animal subject in order to generate and communicate biological vital signs.

In some implementations, apparatus 2100 includes a skin-pixel-identification module 1802 that identifies pixel values 1806 that are representative of the skin in two or more images 1404. The skin-pixel-identification module 1802 performs block 2302 in FIG. 25. Some implementations of the skin-pixel-identification module 1802 perform an automatic seed point based clustering process on the least two images 1404. In some implementations, apparatus 2100 includes a spatial bandpass filter module 2102 that applies a spatial bandpass filter to the identified pixel values 1806, generating spatial bandpassed filtered identified pixel values of skin 2104. In some implementations, the spatial bandpass filter module 2102 includes a two-dimensional spatial Fourier Transform, a high pass filter, a low pass filter, a bandpass filter or a weighted bandpass filter. The spatial bandpass filter module 2102 performs block 2502 in FIG. 25.

In some implementations, apparatus 2100 includes a spatial-cluster module 1812 that applies spatial clustering to the frequency filtered identified pixel values of skin 1810, generating spatial clustered spatial bandpassed identified pixel values of skin 2106. In some implementations the spatial clustering includes fuzzy clustering, k-means clustering, expectation-maximization process, Ward's apparatus or seed point based clustering. The spatial-cluster module 1812 performs block 2504 in FIG. 25.

In some implementations, apparatus 2100 includes a temporal bandpass filter module 2108 that applies a temporal bandpass filter to the spatial clustered spatial bandpass filtered identified pixel values of skin 2106, generating temporal bandpass filtered spatial clustered spatial bandpass filtered identified pixel values of skin 2110. In some implementations, the temporal bandpass filter is a one-dimensional spatial Fourier Transform, a high pass filter, a low pass filter, a bandpass filter or a weighted bandpass filter. The temporal bandpass filter module 2108 performs block 2506 in FIG. 25.

In some implementations, apparatus 2100 includes a temporal-variation module 1820 that determines temporal variation 2222 of the temporal bandpass filtered spatial clustered spatial bandpass filtered identified pixel values of skin 2110. Thus, temporal variation 2222 represents temporal variation of the images 1404. The temporal-variation module 2220 performs block 2508 of FIG. 25. The temporal-variation module 2220 is operably coupled to one or more modules in FIG. 19 to generate and present any one of a number of biological vital signs from amplified motion in the temporal variation 2222.

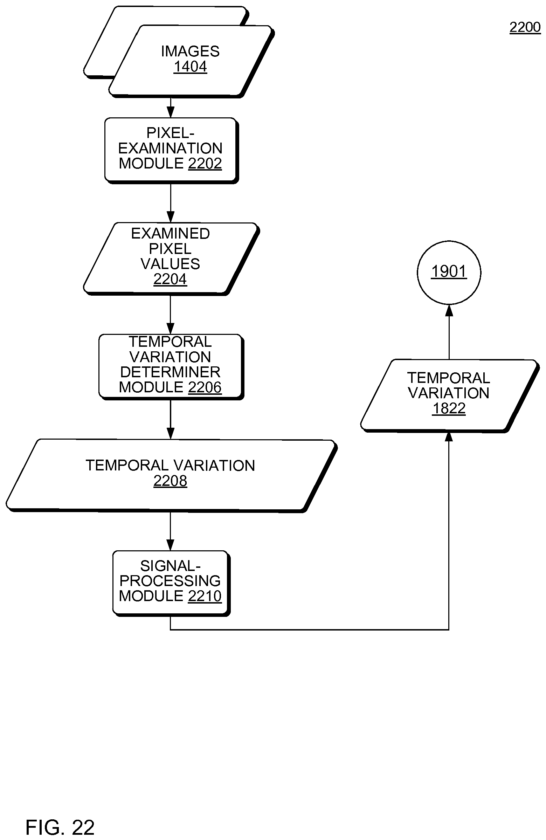

FIG. 22 is a block diagram of an apparatus 2200 of motion amplification, according to an implementation.

In some implementations, apparatus 2200 includes a pixel-examination-module 2202 that examines pixel values of two or more images 1404, generating examined pixel values 2204. The pixel-examination-module 2202 performs block 2602 in FIG. 26.

In some implementations, apparatus 2200 includes a temporal variation determiner module 2206 that determines a temporal variation 2208 of the examined pixel values 2204. The temporal variation determiner module 2206 performs block 2604 in FIG. 26.

In some implementations, apparatus 2200 includes a signal-processing module 2210 that applies signal processing to the pixel value temporal variations 2208, generating an amplified temporal variation 2222. The signal-processing module 2210 performs block 2606 in FIG. 26. The signal processing amplifies the temporal variation 2208, even when the temporal variation 2208 is small. In some implementations, the signal processing performed by signal-processing module 2210 is temporal bandpass filtering that analyzes frequencies over time. In some implementations, the signal processing performed by signal-processing module 2210 is spatial processing that removes noise. Apparatus 2200 amplifies only small temporal variations in the signal-processing module.

While apparatus 2200 can process large temporal variations, an advantage in apparatus 2200 is provided for small temporal variations. Therefore apparatus 2200 is most effective when the two or more images 1404 have small temporal variations between the two or more images 1404. In some implementations, a vital sign is generated from the amplified temporal variations of the two or more images 1404 from the signal-processing module 2210.

Vital Sign Amplification Method Implementations

FIG. 23-27 each use spatial and temporal signal processing to generate vital signs from a series of digital images.

FIG. 23 is a flowchart of a method 2300 of motion amplification, according to an implementation. Method 2300 analyzes the temporal and spatial variations in digital images of an animal subject in order to generate and communicate biological vital signs.

In some implementations, method 2300 includes identifying pixel values of two or more images that are representative of the skin, at block 2302. Some implementations of identifying pixel values that are representative of the skin includes performing an automatic seed point based clustering process on the least two images.

In some implementations, method 2300 includes applying a frequency filter to the identified pixel values that are representative of the skin, at block 2304. In some implementations, the frequency filter in block 2304 is a two-dimensional spatial Fourier Transform, a high pass filter, a low pass filter, a bandpass filter or a weighted bandpass filter.

In some implementations, method 2300 includes applying spatial clustering to the frequency filtered identified pixel values of skin, at block 2306. In some implementations the spatial clustering includes fuzzy clustering, k-means clustering, expectation-maximization process, Ward's method or seed point based clustering.

In some implementations, method 2300 includes applying a frequency filter to the spatial clustered frequency filtered identified pixel values of skin, at block 2308. In some implementations, the frequency filter in block 2308 is a one-dimensional spatial Fourier Transform, a high pass filter, a low pass filter, a bandpass filter or a weighted bandpass filter. Some implementations of applying a frequency filter at block 2308 include de-noising (e.g. smoothing of the data with a Gaussian filter).

Actions 2302, 2304, 2306 and 2308 amplify temporal variations in the two or more images.

In some implementations, method 2300 includes determining temporal variation of the frequency filtered spatial clustered frequency filtered identified pixel values of skin, at block 2310.

In some implementations, method 2300 includes analyzing the temporal variation to generate a pattern of flow of blood, at block 2312. In some implementations, the pattern flow of blood is generated from motion changes in the pixels and the temporal variation of color changes in the skin. In some implementations, method 2300 includes displaying the pattern of flow of blood for review by a healthcare worker, at block 2313.

In some implementations, method 2300 includes analyzing the temporal variation to generate heartrate, at block 2314. In some implementations, the heartrate is generated from the frequency spectrum of the temporal variation in a frequency range for heart beats, such as (0-10 Hertz). In some implementations, method 2300 includes displaying the heartrate for review by a healthcare worker, at block 2315.

In some implementations, method 2300 includes analyzing the temporal variation to determine respiratory rate, at block 2316. In some implementations, the respiratory rate is generated from the motion of the pixels in a frequency range for respiration (0-5 Hertz). In some implementations, method 2300 includes displaying the respiratory rate for review by a healthcare worker, at block 2317.

In some implementations, method 2300 includes analyzing the temporal variation to generate blood pressure, at block 2318. In some implementations, the blood pressure is generated by analyzing the motion of the pixels and the color changes based on the clustering process and potentially temporal data from the infrared sensor. In some implementations, method 2300 includes displaying the blood pressure for review by a healthcare worker, at block 2319.

In some implementations, method 2300 includes analyzing the temporal variation to generate EKG, at block 2320. In some implementations, method 2300 includes displaying the EKG for review by a healthcare worker, at block 2321.

In some implementations, method 2300 includes analyzing the temporal variation to generate pulse oximetry, at block 2322. In some implementations, the pulse oximetry is generated by analyzing the temporal color changes based in conjunction with the k-means clustering process and potentially temporal data from the infrared sensor. In some implementations, method 2300 includes displaying the pulse oximetry for review by a healthcare worker, at block 2323.

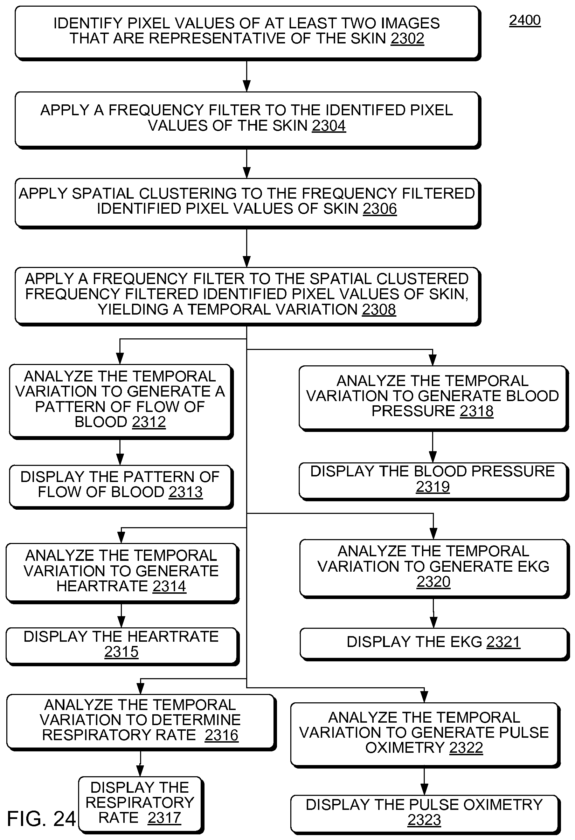

FIG. 24 is a flowchart of a method of motion amplification, according to an implementation that does not include a separate action of determining a temporal variation. Method 2400 analyzes the temporal and spatial variations in digital images of an animal subject in order to generate and communicate biological vital signs.

In some implementations, method 2400 includes identifying pixel values of two or more images that are representative of the skin, at block 2302. Some implementations of identifying pixel values that are representative of the skin includes performing an automatic seed point based clustering process on the least two images.

In some implementations, method 2400 includes applying a frequency filter to the identified pixel values that are representative of the skin, at block 2304. In some implementations, the frequency filter in block 2304 is a two-dimensional spatial Fourier Transform, a high pass filter, a low pass filter, a bandpass filter or a weighted bandpass filter.

In some implementations, method 2400 includes applying spatial clustering to the frequency filtered identified pixel values of skin, at block 2306. In some implementations the spatial clustering includes fuzzy clustering, k-means clustering, expectation-maximization process, Ward's method or seed point based clustering.

In some implementations, method 2400 includes applying a frequency filter to the spatial clustered frequency filtered identified pixel values of skin, at block 2308, yielding a temporal variation. In some implementations, the frequency filter in block 2308 is a one-dimensional spatial Fourier Transform, a high pass filter, a low pass filter, a bandpass filter or a weighted bandpass filter.

In some implementations, method 2400 includes analyzing the temporal variation to generate a pattern of flow of blood, at block 2312. In some implementations, the pattern flow of blood is generated from motion changes in the pixels and the temporal variation of color changes in the skin. In some implementations, method 2400 includes displaying the pattern of flow of blood for review by a healthcare worker, at block 2313.

In some implementations, method 2400 includes analyzing the temporal variation to generate heartrate, at block 2314. In some implementations, the heartrate is generated from the frequency spectrum of the temporal variation in a frequency range for heart beats, such as (0-10 Hertz). In some implementations, method 2400 includes displaying the heartrate for review by a healthcare worker, at block 2315.

In some implementations, method 2400 includes analyzing the temporal variation to determine respiratory rate, at block 2316. In some implementations, the respiratory rate is generated from the motion of the pixels in a frequency range for respiration (0-5 Hertz). In some implementations, method 2400 includes displaying the respiratory rate for review by a healthcare worker, at block 2317.

In some implementations, method 2400 includes analyzing the temporal variation to generate blood pressure, at block 2318. In some implementations, the blood pressure is generated by analyzing the motion of the pixels and the color changes based on the clustering process and potentially temporal data from the infrared sensor. In some implementations, method 2400 includes displaying the blood pressure for review by a healthcare worker, at block 2319.

In some implementations, method 2400 includes analyzing the temporal variation to generate EKG, at block 2320. In some implementations, method 2400 includes displaying the EKG for review by a healthcare worker, at block 2321.

In some implementations, method 2400 includes analyzing the temporal variation to generate pulse oximetry, at block 2322. In some implementations, the pulse oximetry is generated by analyzing the temporal color changes based in conjunction with the k-means clustering process and potentially temporal data from the infrared sensor. In some implementations, method 2400 includes displaying the pulse oximetry for review by a healthcare worker, at block 2323.

FIG. 25 is a flowchart of a method 2500 of motion amplification from which to generate and communicate biological vital signs, according to an implementation. Method 2500 analyzes the temporal and spatial variations in digital images of an animal subject in order to generate and communicate the biological vital signs.

In some implementations, method 2500 includes identifying pixel values of two or more images that are representative of the skin, at block 2302. Some implementations of identifying pixel values that are representative of the skin includes performing an automatic seed point based clustering process on the least two images.

In some implementations, method 2500 includes applying a spatial bandpass filter to the identified pixel values, at block 2502. In some implementations, the spatial filter in block 2502 is a two-dimensional spatial Fourier Transform, a high pass filter, a low pass filter, a bandpass filter or a weighted bandpass filter.

In some implementations, method 2500 includes applying spatial clustering to the spatial bandpass filtered identified pixel values of skin, at block 2504. In some implementations the spatial clustering includes fuzzy clustering, k-means clustering, expectation-maximization process, Ward's method or seed point based clustering.

In some implementations, method 2500 includes applying a temporal bandpass filter to the spatial clustered spatial bandpass filtered identified pixel values of skin, at block 2506. In some implementations, the temporal bandpass filter in block 2506 is a one-dimensional spatial Fourier Transform, a high pass filter, a low pass filter, a bandpass filter or a weighted bandpass filter.

In some implementations, method 2500 includes determining temporal variation of the temporal bandpass filtered spatial clustered spatial bandpass filtered identified pixel values of skin, at block 2508.

In some implementations, method 2500 includes analyzing the temporal variation to generate and visually display a pattern of flow of blood, at block 2312. In some implementations, the pattern flow of blood is generated from motion changes in the pixels and the temporal variation of color changes in the skin. In some implementations, method 2500 includes displaying the pattern of flow of blood for review by a healthcare worker, at block 2313.

In some implementations, method 2500 includes analyzing the temporal variation to generate heartrate, at block 2314. In some implementations, the heartrate is generated from the frequency spectrum of the temporal variation in a frequency range for heart beats, such as (0-10 Hertz). In some implementations, method 2500 includes displaying the heartrate for review by a healthcare worker, at block 2315.

In some implementations, method 2500 includes analyzing the temporal variation to determine respiratory rate, at block 2316. In some implementations, the respiratory rate is generated from the motion of the pixels in a frequency range for respiration (0-5 Hertz). In some implementations, method 2500 includes displaying the respiratory rate for review by a healthcare worker, at block 2317.

In some implementations, method 2500 includes analyzing the temporal variation to generate blood pressure, at block 2318. In some implementations, the blood pressure is generated by analyzing the motion of the pixels and the color changes based on the clustering process and potentially temporal data from the infrared sensor. In some implementations, method 2500 includes displaying the blood pressure for review by a healthcare worker, at block 2319.

In some implementations, method 2500 includes analyzing the temporal variation to generate EKG, at block 2320. In some implementations, method 2500 includes displaying the EKG for review by a healthcare worker, at block 2321.

In some implementations, method 2500 includes analyzing the temporal variation to generate pulse oximetry, at block 2322. In some implementations, the pulse oximetry is generated by analyzing the temporal color changes based in conjunction with the k-means clustering process and potentially temporal data from the infrared sensor. In some implementations, method 2500 includes displaying the pulse oximetry for review by a healthcare worker, at block 2323.

FIG. 26 is a flowchart of a method 2600 of motion amplification, according to an implementation. Method 2600 displays the temporal variations based on temporal variations in videos that are difficult or impossible to see with the naked eye. Method 2600 applies spatial decomposition to a video, and applies temporal filtering to the frames. The resulting signal is then amplified to reveal hidden information. Method 2600 can visualize flow of blood filling a face in the video and also amplify and reveal small motions, and other vital signs such as blood pressure, respiration, EKG and pulse. Method 2600 can execute in real time to show phenomena occurring at temporal frequencies selected by the operator. A combination of spatial and temporal processing of videos can amplify subtle variations that reveal important aspects of the world. Method 2600 considers a time series of color values at any spatial location (e.g., a pixel) and amplifies variation in a given temporal frequency band of interest. For example, method 2600 selects and then amplifies a band of temporal frequencies including plausible human heart rates. The amplification reveals the variation of redness as blood flows through the face. Lower spatial frequencies are temporally filtered (spatial pooling) to allow a subtle input signal to rise above the solid-state image transducer 128 and quantization noise. The temporal filtering approach not only amplifies color variation, but can also reveal low-amplitude motion.

Method 2600 can enhance the subtle motions around the chest of a breathing baby. Method 2600 mathematical analysis employs a linear approximation related to the brightness constancy assumption used in optical flow formulations. Method 2600 also derives the conditions under which the linear approximation holds. The derivation leads to a multiscale approach to magnify motion without feature tracking or motion estimation. Properties of a voxel of fluid are observed, such as pressure and velocity, which evolve over time. Method 2600 studies and amplifies the variation of pixel values over time, in a spatially-multiscale manner. The spatially-multiscale manner to motion magnification does not explicitly estimate motion, but rather exaggerates motion by amplifying temporal color changes at fixed positions. Method 2600 employs differential approximations that form the basis of optical flow processes. Method 2600 described herein employs localized spatial pooling and bandpass filtering to extract and reveal visually the signal corresponding to the pulse. The domain analysis allows amplification and visualization of the pulse signal at each location on the face. Asymmetry in facial blood flow can be a symptom of arterial problems.

Method 2600 described herein makes imperceptible motions visible using a multiscale approach. Method 2600 amplifies small motions, in one embodiment. Nearly invisible changes in a dynamic environment can be revealed through spatio-temporal processing of standard monocular video sequences. Moreover, for a range of amplification values that is suitable for various applications, explicit motion estimation is not required to amplify motion in natural videos. Method 2600 is well suited to small displacements and lower spatial frequencies. Single framework can amplify both spatial motion and purely temporal changes (e.g., a heart pulse) and can be adjusted to amplify particular temporal frequencies. A spatial decomposition module decomposes the input video into different spatial frequency bands, then applies the same temporal filter to the spatial frequency bands. The outputted filtered spatial bands are then amplified by an amplification factor, added back to the original signal by adders, and collapsed by a reconstruction module to generate the output video. The temporal filter and amplification factors can be tuned to support different applications. For example, the system can reveal unseen motions of a solid-state image transducer 128, caused by the flipping mirror during a photo burst.

Method 2600 combines spatial and temporal processing to emphasize subtle temporal changes in a video. Method 2600 decomposes the video sequence into different spatial frequency bands. These bands might be magnified differently because (a) the bands might exhibit different signal-to-noise ratios or (b) the bands might contain spatial frequencies for which the linear approximation used in motion magnification does not hold. In the latter case, method 2600 reduces the amplification for these bands to suppress artifacts. When the goal of spatial processing is to increase temporal signal-to-noise ratio by pooling multiple pixels, the method spatially low-pass filters the frames of the video and downsamples the video frames for computational efficiency. In the general case, however, method 2600 computes a full Laplacian pyramid.

Method 2600 then performs temporal processing on each spatial band. Method 2600 considers the time series corresponding to the value of a pixel in a frequency band and applies a bandpass filter to extract the frequency bands of interest. As one example, method 2600 may select frequencies within the range of 0.4-4 Hz, corresponding to 24-240 beats per minute, if the operator wants to magnify a pulse. If method 2600 extracts the pulse rate, then method 2600 can employ a narrow frequency band around that value. The temporal processing is uniform for all spatial levels and for all pixels within each level. Method 2600 then multiplies the extracted bandpassed signal by a magnification factor .alpha. The magnification factor .alpha. can be specified by the operator, and can be attenuated automatically. Method 2600 adds the magnified signal to the original signal and collapses the spatial pyramid to obtain the final output. Since natural videos are spatially and temporally smooth, and since the filtering is performed uniformly over the pixels, the method implicitly maintains spatiotemporal coherency of the results. The motion magnification amplifies small motion without tracking motion. Temporal processing produces motion magnification, shown using an analysis that relies on the first-order Taylor series expansions common in optical flow analyses.

Method 2600 begins with a pixel-examination module in the microprocessor 102 of the non-touch biologic detectors 100, 200 or 300 examining pixel values of two or more images 1404 from the solid-state image transducer 128, at block 2602.

Method 2600 thereafter determines the temporal variation of the examined pixel values, at block 2604 by a temporal-variation module in the microprocessor 102.

A signal-processing module in the microprocessor 102 applies signal processing to the pixel value temporal variations, at block 2606. Signal processing amplifies the determined temporal variations, even when the temporal variations are small. Method 2600 amplifies only small temporal variations in the signal-processing module. While method 2600 can be applied to large temporal variations, an advantage in method 2600 is provided for small temporal variations. Therefore method 2600 is most effective when the input images 1404 have small temporal variations between the images 1404. In some implementations, the signal processing at block 2606 is temporal bandpass filtering that analyzes frequencies over time. In some implementations, the signal processing at block 2606 is spatial processing that removes noise.

In some implementations, a vital sign is generated from the amplified temporal variations of the input images 1404 from the signal processor at block 2608. Examples of generating a vital signal from a temporal variation include as in actions 2312, 2314, 2316, 2318, 2320 and 2322 in FIG. 23, 24 and 25.

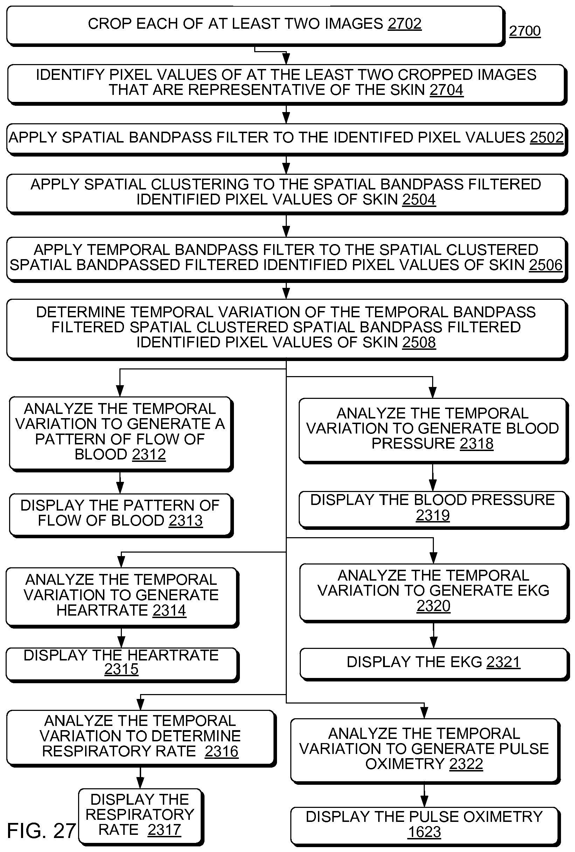

FIG. 27 is a flowchart of a method 2700 of motion amplification from which to generate and communicate biological vital signs, according to an implementation. Method 2700 analyzes the temporal and spatial variations in digital images of an animal subject in order to generate and communicate the biological vital signs.

In some implementations, method 2700 includes cropping at least two images to exclude areas that do not include a skin region, at block 2702. For example, the excluded area can be a perimeter area around the center of each image, so that an outside border area of the image is excluded. In some implementations of cropping out the border, about 72% of the width and about 72% of the height of each image is cropped out, leaving only 7.8% of the original uncropped image, which eliminates about 11/12 of each image and reduces the amount of processing time for the remainder of the actions in this process by about 12-fold. This one action alone at block 2702 in method 2700 can reduce the processing time of plurality of images 130 in comparison to method 2500 from 4 minutes to 30 seconds, which is of significant difference to the health workers who used devices that implement method 2700. In some implementations, the remaining area of the image after cropping in a square area and in other implementation the remaining area after cropping is a circular area. Depending upon the topography and shape of the area in the images that has the most pertinent portion of the imaged subject, different geometries and sizes are most beneficial. The action of cropping the images at block 2702 can be applied at the beginning of methods 2300, 2400, 2500 and 2600 in FIG. 23, 24, 25 and 26, respectively. In other implementations of apparatus 1400, 1500, 1600, 1700, 1800, 1900, 2000, 2100 and 2200, a cropper module that performs block 2702 is placed at the beginning of the modules to greatly decrease processing time of the apparatus.

In some implementations, method 2700 includes identifying pixel values of the at least two or more cropped images that are representative of the skin, at block 2704. Some implementations of identifying pixel values that are representative of the skin include performing an automatic seed point based clustering process on the least two images.

In some implementations, method 2700 includes applying a spatial bandpass filter to the identified pixel values, at block 2502. In some implementations, the spatial filter in block 2502 is a two-dimensional spatial Fourier Transform, a high pass filter, a low pass filter, a bandpass filter or a weighted bandpass filter.

In some implementations, method 2700 includes applying spatial clustering to the spatial bandpass filtered identified pixel values of skin, at block 2504. In some implementations the spatial clustering includes fuzzy clustering, k-means clustering, expectation-maximization process, Ward's method or seed point based clustering.

In some implementations, method 2700 includes applying a temporal bandpass filter to the spatial clustered spatial bandpass filtered identified pixel values of skin, at block 2506. In some implementations, the temporal bandpass filter in block 2506 is a one-dimensional spatial Fourier Transform, a high pass filter, a low pass filter, a bandpass filter or a weighted bandpass filter.

In some implementations, method 2700 includes determining temporal variation of the temporal bandpass filtered spatial clustered spatial bandpass filtered identified pixel values of skin, at block 2508.

In some implementations, method 2700 includes analyzing the temporal variation to generate and visually display a pattern of flow of blood, at block 2312. In some implementations, the pattern flow of blood is generated from motion changes in the pixels and the temporal variation of color changes in the skin. In some implementations, method 2700 includes displaying the pattern of flow of blood for review by a healthcare worker, at block 2313.

In some implementations, method 2700 includes analyzing the temporal variation to generate heartrate, at block 2314. In some implementations, the heartrate is generated from the frequency spectrum of the temporal variation in a frequency range for heart beats, such as (0-10 Hertz). In some implementations, method 2700 includes displaying the heartrate for review by a healthcare worker, at block 2315.

In some implementations, method 2700 includes analyzing the temporal variation to determine respiratory rate, at block 2316. In some implementations, the respiratory rate is generated from the motion of the pixels in a frequency range for respiration (0-5 Hertz). In some implementations, method 2700 includes displaying the respiratory rate for review by a healthcare worker, at block 2317.

In some implementations, method 2700 includes analyzing the temporal variation to generate blood pressure, at block 2318. In some implementations, the blood pressure is generated by analyzing the motion of the pixels and the color changes based on the clustering process and potentially temporal data from the infrared sensor. In some implementations, method 2700 includes displaying the blood pressure for review by a healthcare worker, at block 2319.

In some implementations, method 2700 includes analyzing the temporal variation to generate EKG, at block 2320. In some implementations, method 2700 includes displaying the EKG for review by a healthcare worker, at block 2321.

In some implementations, method 2700 includes analyzing the temporal variation to generate pulse oximetry, at block 2322. In some implementations, the pulse oximetry is generated by analyzing the temporal color changes based in conjunction with the k-means clustering process and potentially temporal data from the infrared sensor. In some implementations, method 2700 includes displaying the pulse oximetry for review by a healthcare worker, at block 2323.

Non-Touch Cubic Temperature Estimation Method Implementations

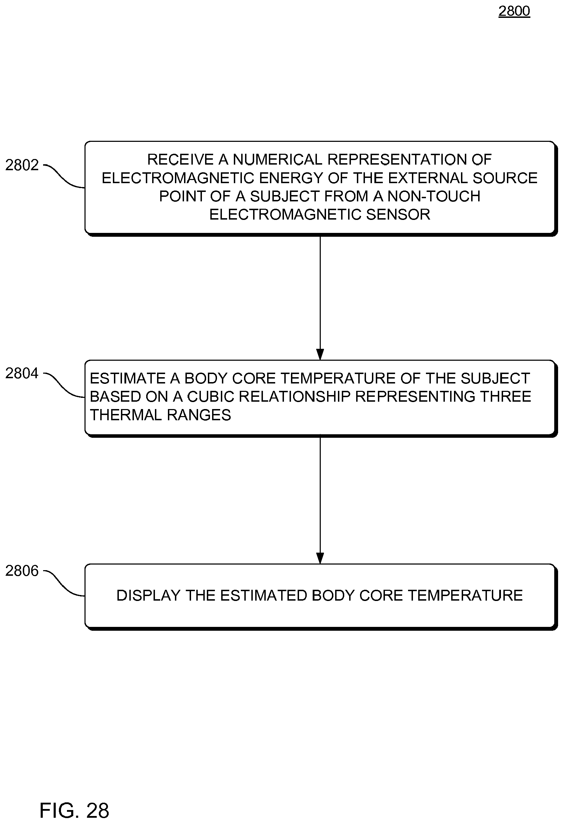

FIG. 28 is a flowchart of a method 2800 to estimate a body core temperature from an external source point in reference to a cubic relationship, according to an implementation.

Method 2800 includes receiving from a non-touch electromagnetic sensor a numerical representation of electromagnetic energy of the external source point of a subject, at block 2802.

Method 2800 also includes estimating the body core temperature of the subject from the numerical representation of the electromagnetic energy of the external source point, a representation of an ambient air temperature reading, a representation of a calibration difference, and a representation of a bias in consideration of the temperature sensing mode, at block 2804. The estimating at block 2804 is based on a cubic relationship representing three thermal ranges between the body core temperature and the numerical representation of the electromagnetic energy of the external source point. The cubic relationship includes a coefficient representative of different relationships between the external source point and the body core temperature in the three thermal ranges.

A cubic relationship for all ranges of ambient temperatures provides best results because a linear or a quadratic relationship provide inaccurate estimates of body temperature, yet a quartic relationship, a quintic relationship, sextic relationship, a septic relationship or an octic relationship provide estimates along a highly irregular curve that is far too wavy or twisting with relatively sharp deviations from one ambient temperature to another ambient temperature.

Method 2800 also includes displaying the body core temperature, at block 2806.

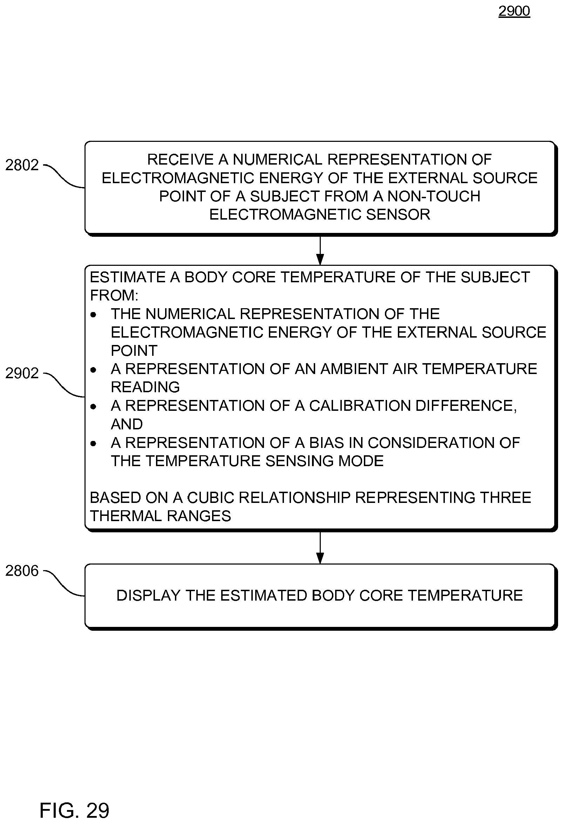

FIG. 29 is a flowchart of a method 2900 to estimate a body core temperature from an external source point and other measurements in reference to a cubic relationship, according to an implementation;

Method 2900 includes receiving from a non-touch electromagnetic sensor a numerical representation of electromagnetic energy of the external source point of a subject, at block 2802.

Method 2900 also includes estimating the body core temperature of the subject from the numerical representation of the electromagnetic energy of the external source point, a representation of an ambient air temperature reading, a representation of a calibration difference, and a representation of a bias in consideration of the temperature sensing mode, at block 2902. The estimating at block 2904 is based on a cubic relationship representing three thermal ranges between the body core temperature and the numerical representation of the electromagnetic energy of the external source point. The cubic relationship includes a coefficient representative of different relationships between the external source point and the body core temperature in the three thermal ranges, wherein the cubic relationship is:

$$T_B = AT_{Skin}^3 + BT_{Skin}^2 + CT_{Skin} + D - E(T_{Ambient} - 75), T_{Ambient} < T_1 \text{ or } T_{Ambient} > T_2 \text{ and } T_B = AT_{Skin}^3 + BT_{Skin}^2 + CT_{Skin} + D, T_1 < T_{Ambient} < T_2$$

where:

$T_B$ is the body core temperature
$T_{skin}$ is the numerical representation of the electromagnetic energy of the external source point
A is 0.0002299688
B is -0.0464237524
C is 3.05944877
D is 31.36205
E is 0.135
$T_{ambient}$ is the ambient air temperature
$T_1$ and $T_2$ are boundaries between the three thermal ranges
$T_1$ and $T_2$ are selected from a group of pairs of ambient temperatures consisting of 67°F and 82°F; 87°F and 95°F; and 86°F and 101°F

Method 2900 also includes displaying the body core temperature, at block 2806.

In some implementations, methods 2300-2900 are implemented as a sequence of instructions which, when executed by a microprocessor 102 in FIG. 1-10, microprocessor 3304 In FIG. 33, main processor 3002 in FIG. 30 or processing unit 3104 in FIG. 31, cause the processor to perform the respective method. In other implementations, methods 2300-2900 are implemented as a computer-accessible medium having computer executable instructions capable of directing a microprocessor, such as microprocessor 102 in FIG. 1-10, microprocessor 3304 in FIG. 33, main processor 3002 in FIG. 30 or processing unit 3104 in FIG. 31, to perform the respective method. In different implementations, the medium is a magnetic medium, an electronic medium, or an optical medium.

Hardware and Operating Environments

FIG. 30 is a block diagram of a hand-held device 3000, according to an implementation. The hand-held device 3000 may also have the capability to allow voice communication. Depending on the functionality provided by the hand-held device 3000, the hand-held device 3000 may be referred to as a data messaging device, a two-way pager, a cellular telephone with data messaging capabilities, a wireless Internet appliance, or a data communication device (with or without telephony capabilities).

The hand-held device 3000 includes a number of modules such as a main processor 3002 that controls the overall operation of the hand-held device 3000. Communication functions, including data and voice communications, are performed through a communication subsystem 3004. The communication subsystem 3004 receives messages from and sends messages to wireless networks 3005. In other implementations of the hand-held device 3000, the communication subsystem 3004 can be configured in accordance with the Global System for Mobile Communication (GSM), General Packet Radio Services (GPRS), Enhanced Data GSM Environment (EDGE), Universal Mobile Telecommunications Service (UMTS), data-centric wireless networks, voice-centric wireless networks, and dual-mode networks that can support both voice and data communications over the same physical base stations. Combined dual-mode networks include, but are not limited to, Code Division Multiple Access (CDMA) or CDMA2000 networks, GSM/GPRS networks (as mentioned above), and future third-generation (3G) networks like EDGE and UMTS. Some other examples of data-centric networks include Mobitex™ and DataTAC™ network communication systems. Examples of other voice-centric data networks include Personal Communication Systems (PCS) networks like GSM and Time Division Multiple Access (TDMA) systems.

The wireless link connecting the communication subsystem 3004 with the wireless network 3005 represents one or more different Radio Frequency (RF) channels. With newer network protocols, these channels are capable of supporting both circuit switched voice communications and packet switched data communications.

The main processor 3002 also interacts with additional subsystems such as a Random Access Memory (RAM) 3006, a flash memory 3008, a display 3010, an auxiliary input/output (I/O) subsystem 3012, a data port 3014, a keyboard 3016, a speaker 3018, a microphone 3020, short-range communications subsystem 3022 and other device subsystems 3024. In some implementations, the flash memory 3008 includes a hybrid femtocell/Wi-Fi protocol stack 3009. The stack 3009 supports authentication and authorization between the hand-held device 3000 into a shared Wi-Fi network and both a 3G and 4G mobile networks.

Some of the subsystems of the hand-held device 3000 perform communication-related functions, whereas other subsystems may provide "resident" or on-device functions. By way of example, the display 3010 and the keyboard 3016 may be used for both communication-related functions, such as entering a text message for transmission over the wireless network 3005, and device-resident functions such as a calculator or task list.

The hand-held device 3000 can transmit and receive communication signals over the wireless network 3005 after required network registration or activation procedures have been completed. Network access is associated with a subscriber or user of the hand-held device 3000. To identify a subscriber, the hand-held device 3000 requires a SIM/RUIM card 3026 (i.e. Subscriber Identity Module or a Removable User Identity Module) to be inserted into a SIM/RUIM interface 3028 in order to communicate with a network. The SIM card or RUIM 3026 is one type of a conventional "smart card" that can be used to identify a subscriber of the hand-held device 3000 and to personalize the hand-held device 3000, among other things. Without the SIM card 3026, the hand-held device 3000 is not fully operational for communication with the wireless network 3005. By inserting the SIM card/RUIM 3026 into the SIM/RUIM interface 3028, a subscriber can access all subscribed services. Services may include: web browsing and messaging such as e-mail, voice mail, Short Message Service (SMS), and Multimedia Messaging Services (MMS). More advanced services may include: point of sale, field service and sales force automation. The SIM card/RUIM 3026 includes a processor and memory for storing information. Once the SIM card/RUIM 3026 is inserted into the SIM/RUIM interface 3028, the SIM is coupled to the main processor 3002. In order to identify the subscriber, the SIM card/RUIM 3026 can include some user parameters such as an International Mobile Subscriber Identity (IMSI). An advantage of using the SIM card/RUIM 3026 is that a subscriber is not necessarily bound by any single physical mobile device. The SIM card/RUIM 3026 may store additional subscriber information for the hand-held device 3000 as well, including datebook (or calendar) information and recent call information. Alternatively, user identification information can also be programmed into the flash memory 3008.

The hand-held device 3000 is a battery-powered device and includes a battery interface 3032 for receiving one or more rechargeable batteries 3030. In one or more implementations, the battery 3030 can be a smart battery with an embedded microprocessor. The battery interface 3032 is coupled to a regulator 3033, which assists the battery 3030 in providing power V+ to the hand-held device 3000. Although current technology makes use of a battery, future technologies such as micro fuel cells may provide the power to the hand-held device 3000.

The hand-held device 3000 also includes an operating system 3034 and modules 3036 to 3049 which are described in more detail below. The operating system 3034 and the modules 3036 to 3049 that are executed by the main processor 3002 are typically stored in a persistent nonvolatile medium such as the flash memory 3008, which may alternatively be a read-only memory (ROM) or similar storage element (not shown). Those skilled in the art will appreciate that portions of the operating system 3034 and the modules 3036 to 3049, such as specific device applications, or parts thereof, may be temporarily loaded into a volatile store such as the RAM 3006. Other modules can also be included.

The subset of modules 3036 that control basic device operations, including data and voice communication applications, will normally be installed on the hand-held device 3000 during its manufacture. Other modules include a message application 3038 that can be any suitable module that allows a user of the hand-held device 3000 to transmit and receive electronic messages. Various alternatives exist for the message application 3038 as is well known to those skilled in the art. Messages that have been sent or received by the user are typically stored in the flash memory 3008 of the hand-held device 3000 or some other suitable storage element in the hand-held device 3000. In one or more implementations, some of the sent and received messages may be stored remotely from the hand-held device 3000 such as in a data store of an associated host system with which the hand-held device 3000 communicates.

The modules can further include a device state module 3040, a Personal Information Manager (PIM) 3042, and other suitable modules (not shown). The device state module 3040 provides persistence, i.e. the device state module 3040 ensures that important device data is stored in persistent memory, such as the flash memory 3008, so that the data is not lost when the hand-held device 3000 is turned off or loses power.

The PIM 3042 includes functionality for organizing and managing data items of interest to the user, such as, but not limited to, e-mail, contacts, calendar events, voice mails, appointments, and task items. A PIM application has the ability to transmit and receive data items via the wireless network 3005. PIM data items may be seamlessly integrated, synchronized, and updated via the wireless network 3005 with the hand-held device 3000 subscriber's corresponding data items stored and/or associated with a host computer system. This functionality creates a mirrored host computer on the hand-held device 3000 with respect to such items. This can be particularly advantageous when the host computer system is the hand-held device 3000 subscriber's office computer system.

The hand-held device 3000 also includes a connect module 3044, and an IT policy module 3046. The connect module 3044 implements the communication protocols that are required for the hand-held device 3000 to communicate with the wireless infrastructure and any host system, such as an enterprise system, with which the hand-held device 3000 is authorized to interface. Examples of a wireless infrastructure and an enterprise system are given in FIGS. 30 and 31, which are described in more detail below.

The connect module 3044 includes a set of APIs that can be integrated with the hand-held device 3000 to allow the hand-held device 3000 to use any number of services associated with the enterprise system. The connect module 3044 allows the hand-held device 3000 to establish an end-to-end secure, authenticated communication pipe with the host system. A subset of applications for which access is provided by the connect module 3044 can be used to pass IT policy commands from the host system to the hand-held device 3000. This can be done in a wireless or wired manner. These instructions can then be passed to the IT policy module 3046 to modify the configuration of the hand-held device 3000. Alternatively, in some cases, the IT policy update can also be done over a wired connection.

The IT policy module 3046 receives IT policy data that encodes the IT policy. The IT policy module 3046 then ensures that the IT policy data is authenticated by the hand-held device 3000. The IT policy data can then be stored in the flash memory 3006 in its native form. After the IT policy data is stored, a global notification can be sent by the IT policy module 3046 to all of the applications residing on the hand-held device 3000. Applications for which the IT policy may be applicable then respond by reading the IT policy data to look for IT policy rules that are applicable.

The IT policy module 3046 can include a parser 3047, which can be used by the applications to read the IT policy rules. In some cases, another module or application can provide the parser. Grouped IT policy rules, described in more detail below, are retrieved as byte streams, which are then sent (recursively) into the parser to determine the values of each IT policy rule defined within the grouped IT policy rule. In one or more implementations, the IT policy module 3046 can determine which applications are affected by the IT policy data and transmit a notification to only those applications. In either of these cases, for applications that are not being executed by the main processor 3002 at the time of the notification, the applications can call the parser or the IT policy module 3046 when the applications are executed to determine if there are any relevant IT policy rules in the newly received IT policy data.

All applications that support rules in the IT Policy are coded to know the type of data to expect. For example, the value that is set for the "WEP User Name" IT policy rule is known to be a string; therefore the value in the IT policy data that corresponds to this rule is interpreted as a string. As another example, the setting for the "Set Maximum Password Attempts" IT policy rule is known to be an integer, and therefore the value in the IT policy data that corresponds to this rule is interpreted as such.

After the IT policy rules have been applied to the applicable applications or configuration files, the IT policy module 3046 sends an acknowledgement back to the host system to indicate that the IT policy data was received and successfully applied.

The programs 3037 can also include a temporal-variation-amplifier 3048 and a vital sign generator 3049. In some implementations, the temporal-variation-amplifier 3048 includes a skin-pixel-identifier 1402, a frequency-filter 1406, a regional facial clusterial module 1408 and a frequency filter 1410 as in FIG. 14 and 15. In some implementations, the temporal-variation-amplifier 3048 includes a skin-pixel-identifier 1402, a spatial bandpass-filter 1602, regional facial clusterial module 1408 and a temporal bandpass filter 1604 as in FIG. 16. In some implementations, the temporal-variation-amplifier 3048 includes a pixel-examiner 1702, a temporal variation determiner 1706 and signal processor 1708 as in FIG. 17. In some implementations, the temporal-variation-amplifier 3048 includes a skin-pixel-identification module 1802, a frequency-filter module 1808, spatial-cluster module 1812 and a frequency filter module 1816 as in FIG. 18 and 19. In some implementations, the temporal-variation-amplifier module 1802, a spatial bandpass filter module 2102, a spatial-cluster module 1812 and a temporal bandpass filter module 2106 as in FIG. 21. In some implementations, the temporal-variation-amplifier 3048 includes a pixel-examination-module 2202, a temporal variation determiner module 2206 and a signal processing module 2210 as in FIG. 22. The solid-state image transducer 128 captures images 130 and the vital sign generator 3049 generates the vital sign(s) 1416 that is displayed by display 3010 or transmitted by communication subsystem 3004 or short-range communications subsystem 3022, enunciated by speaker 3018 or stored by flash memory 3008.

Other types of modules can also be installed on the hand-held device 3000. These modules can be third party modules, which are added after the manufacture of the hand-held device 3000. Examples of third party applications include games, calculators, utilities, etc.

The additional applications can be loaded onto the hand-held device 3000 through at least one of the wireless network 3005, the auxiliary I/O subsystem 3012, the data port 3014, the short-range communications subsystem 3022, or any other suitable device subsystem 3024. This flexibility in application installation increases the functionality of the hand-held device 3000 and may provide enhanced on-device functions, communication-related functions, or both. For example, secure communication applications may enable electronic commerce functions and other such financial transactions to be performed using the hand-held device 3000.

The data port 3014 enables a subscriber to set preferences through an external device or module and extends the capabilities of the hand-held device 3000 by providing for information or module downloads to the hand-held device 3000 other than through a wireless communication network. The alternate download path may, for example, be used to load an encryption key onto the hand-held device 3000 through a direct and thus reliable and trusted connection to provide secure device communication.

The data port 3014 can be any suitable port that enables data communication between the hand-held device 3000 and another computing device. The data port 3014 can be a serial or a parallel port. In some instances, the data port 3014 can be a USB port that includes data lines for data transfer and a supply line that can provide a charging current to charge the battery 3030 of the hand-held device 3000.

The short-range communications subsystem 3022 provides for communication between the hand-held device 3000 and different systems or devices, without the use of the wireless network 3005. For example, the subsystem 3022 may include an infrared device and associated circuits and modules for short-range communication. Examples of short-range communication standards include standards developed by the Infrared Data Association (IrDA), Bluetooth, and the 802.11 family of standards developed by IEEE.

Bluetooth is a wireless technology standard for exchanging data over short distances (using short-wavelength radio transmissions in the ISM band from 2400-2480 MHz) from fixed and mobile devices, creating personal area networks (PANs) with high levels of security. Created by telecom vendor Ericsson in 2694, Bluetooth was originally conceived as a wireless alternative to RS-232 data cables. Blutooth can connect several devices, overcoming problems of synchronization. Bluetooth operates in the range of 2400-2483.5 MHz (including guard bands), which is in the globally unlicensed Industrial, Scientific and Medical (ISM) 2.4 GHz short-range radio frequency band. Bluetooth uses a radio technology called frequency-hopping spread spectrum. The transmitted data is divided into packets and each packet is transmitted on one of the 79 designated Bluetooth channels. Each channel has a bandwidth of 1 MHz. The first channel starts at 2402 MHz and continues up to 2480 MHz in 1 MHz steps. The first channel usually performs 1600 hops per second, with Adaptive Frequency-Hopping (AFH) enabled. Originally Gaussian frequency-shift keying (GFSK) modulation was the only modulation scheme available; subsequently, since the introduction of Bluetooth 2.0+EDR, $\pi$/4-DQPSK and 8DPSK modulation may also be used between compatible devices. Devices functioning with GFSK are said to be operating in basic rate (BR) mode where an instantaneous data rate of 1 Mbit/s is possible. The term Enhanced Data Rate (EDR) is used to describe $\pi$/4-DPSK and 8DPSK schemes, each giving 2 and 3 Mbit/s respectively. The combination of these (BR and EDR) modes in Bluetooth radio technology is classified as a "BR/EDR radio". Bluetooth is a packet-based protocol with a master-slave structure. One master may communicate with up to 7 slaves in a piconet; all devices share the master's clock. Packet exchange is based on the basic clock, defined by the master, which ticks at 312.5 $\mu$s intervals. Two clock ticks make up a slot of 625 $\mu$s; two slots make up a slot pair of 1250 $\mu$s. In the simple case of single-slot packets the master transmits in even slots and receives in odd slots; the slave, conversely, receives in even slots and transmits in odd slots. Packets may be 1, 3 or 5 slots long but in all cases the master transmit will begin in even slots and the slave transmit in odd slots. A master Bluetooth device can communicate with a maximum of seven devices in a piconet (an ad-hoc computer network using Bluetooth technology), though not all devices reach this maximum. The devices can switch roles, by agreement, and the slave can become the master (for example, a headset initiating a connection to a phone will necessarily begin as master, as initiator of the connection; but may subsequently prefer to be slave). The Bluetooth Core Specification provides for the connection of two or more piconets to form a scatternet, in which certain devices simultaneously play the master role in one piconet and the slave role in another. At any given time, data can be transferred between the master and one other device (except for the little-used broadcast mode. The master chooses which slave device to address; typically, the master switches rapidly from one device to another in a round-robin fashion. Since the master chooses which slave to address, whereas a slave is (in theory) supposed to listen in each receive slot, being a master is a lighter burden than being a slave. Being a master of seven slaves is possible; being a slave of more than one master is difficult. Many of the services offered over Bluetooth can expose private data or allow the connecting party to control the Bluetooth device. For security reasons it is necessary to be able to recognize specific devices and thus enable control over which devices are allowed to connect to a given Bluetooth device. At the same time, it is useful for Bluetooth devices to be able to establish a connection without user intervention (for example, as soon as the Bluetooth devices of each other are in range). To resolve this conflict, Bluetooth uses a process called bonding, and a bond is created through a process called pairing. The pairing process is triggered either by a specific request from a user to create a bond (for example, the user explicitly requests to "Add a Bluetooth device"), or the pairing process is triggered automatically when connecting to a service where (for the first time) the identity of a device is required for security purposes. These two cases are referred to as dedicated bonding and general bonding respectively. Pairing often involves some level of user interaction; this user interaction is the basis for confirming the identity of the devices. Once pairing successfully completes, a bond will have been formed between the two devices, enabling those two devices to connect to each other in the future without requiring the pairing process in order to confirm the identity of the devices. When desired, the bonding relationship can later be removed by the user. Secure Simple Pairing (SSP): This is required by Bluetooth v2.1, although a Bluetooth v2.1 device may only use legacy pairing to interoperate with a v2.0 or earlier device. Secure Simple Pairing uses a form of public key cryptography, and some types can help protect against man in the middle, or MITM attacks. SSP has the following characteristics: Just works: As implied by the name, this method just works. No user interaction is required; however, a device may prompt the user to confirm the pairing process. This method is typically used by headsets with very limited IO capabilities, and is more secure than the fixed PIN mechanism which is typically used for legacy pairing by this set of limited devices. This method provides no man in the middle (MITM) protection. Numeric comparison: If both devices have a display and at least one can accept a binary Yes/No user input, both devices may use Numeric Comparison. This method displays a 6-digit numeric code on each device. The user should compare the numbers to ensure that the numbers are identical. If the comparison succeeds, the user(s) should confirm pairing on the device(s) that can accept an input. This method provides MITM protection, assuming the user confirms on both devices and actually performs the comparison properly. Passkey Entry: This method may be used between a device with a display and a device with numeric keypad entry (such as a keyboard), or two devices with numeric keypad entry. In the first case, the display is used to show a 6-digit numeric code to the user, who then enters the code on the keypad. In the second case, the user of each device enters the same 6-digit number. Both of these cases provide MITM protection. Out of band (OOB): This method uses an external means of communication, such as Near Field Communication (NFC) to exchange some information used in the pairing process. Pairing is completed using the Bluetooth radio, but requires information from the OOB mechanism. This provides only the level of MITM protection that is present in the OOB mechanism. SSP is considered simple for the following reasons: In most cases, SSP does not require a user to generate a passkey. For use-cases not requiring MITM protection, user interaction can be eliminated. For numeric comparison, MITM protection can be achieved with a simple equality comparison by the user. Using OOB with NFC enables pairing when devices simply get close, rather than requiring a lengthy discovery process.

In use, a received signal such as a text message, an e-mail message, or web page download will be processed by the communication subsystem 3004 and input to the main processor 3002. The main processor 3002 will then process the received signal for output to the display 3010 or alternatively to the auxiliary I/O subsystem 3012. A subscriber may also compose data items, such as e-mail messages, for example, using the keyboard 3016 in conjunction with the display 3010 and possibly the auxiliary I/O subsystem 3012. The auxiliary subsystem 3012 may include devices such as: a touch screen, mouse, track ball, infrared fingerprint detector, or a roller wheel with dynamic button pressing capability. The keyboard 3016 is preferably an alphanumeric keyboard and/or telephone-type keypad. However, other types of keyboards may also be used. A composed item may be transmitted over the wireless network 3005 through the communication subsystem 3004.

For voice communications, the overall operation of the hand-held device 3000 is substantially similar, except that the received signals are output to the speaker 3018, and signals for transmission are generated by the microphone 3020. Alternative voice or audio I/O subsystems, such as a voice message recording subsystem, can also be implemented on the hand-held device 3000. Although voice or audio signal output is accomplished primarily through the speaker 3018, the display 3010 can also be used to provide additional information such as the identity of a calling party, duration of a voice call, or other voice call related information.

FIG. 31 is a block diagram of a hardware and operating environment 3100 in which different implementations can be practiced. The description of FIG. 31 provides an overview of computer hardware and a suitable computing environment in conjunction with which some implementations can be implemented. Implementations are described in terms of a computer executing computer-executable instructions. However, some implementations can be implemented entirely in computer hardware in which the computer-executable instructions are implemented in read-only memory. Some implementations can also be implemented in client/server computing environments where remote devices that perform tasks are linked through a communications network. Program modules can be located in both local and remote memory storage devices in a distributed computing environment.

FIG. 31 illustrates an example of a computer environment 3100 useful in the context of the environment of FIG. 1-16, in accordance with an implementation. The computer environment 3100 includes a computation resource 3102 capable of implementing the processes described herein. It will be appreciated that other devices can alternatively used that include more modules, or fewer modules, than those illustrated in FIG. 31.

The illustrated operating environment 3100 is only one example of a suitable operating environment, and the example described with reference to FIG. 31 is not intended to suggest any limitation as to the scope of use or functionality of the implementations of this disclosure. Other well-known computing systems, environments, and/or configurations can be suitable for implementation and/or application of the subject matter disclosed herein.

The computation resource 3102 includes one or more processors or processing units 3104, a system memory 3106, and a bus 3108 that couples various system modules including the system memory 3106 to processing unit 3104 and other elements in the environment 3100. The bus 3108 represents one or more of any of several types of bus structures, including a memory bus or memory controller, a peripheral bus, an accelerated graphics port and a processor or local bus using any of a variety of bus architectures, and can be compatible with SCSI (small computer system interconnect), or other conventional bus architectures and protocols.

The system memory 3106 includes nonvolatile read-only memory (ROM) 3110 and random access memory (RAM) 3112, which can or can not include volatile memory elements. A basic input/output system (BIOS) 3114, containing the elementary routines that help to transfer information between elements within computation resource 3102 and with external items, typically invoked into operating memory during start-up, is stored in ROM 3110.

The computation resource 3102 further can include a non-volatile read/write memory 3116, represented in FIG. 31 as a hard disk drive, coupled to bus 3108 via a data media interface 3117 (e.g., a SCSI, ATA, or other type of interface); a magnetic disk drive (not shown) for reading from, and/or writing to, a removable magnetic disk 3120 and an optical disk drive (not shown) for reading from, and/or writing to, a removable optical disk 3126 such as a CD, DVD, or other optical media.

The non-volatile read/write memory 3116 and associated computer-readable media provide nonvolatile storage of computer-readable instructions, data structures, program modules and other data for the computation resource 3102. Although the exemplary environment 3100 is described herein as employing a non-volatile read/write memory 3116, a removable magnetic disk 3120 and a removable optical disk 3126, it will be appreciated by those skilled in the art that other types of computer-readable media which can store data that is accessible by a computer, such as magnetic cassettes, FLASH memory cards, random access memories (RAMs), read only memories (ROM), and the like, can also be used in the exemplary operating environment.

A number of program modules can be stored via the non-volatile read/write memory 3116, magnetic disk 3120, optical disk 3126, ROM 3110, or RAM 3112, including an operating system 3130, one or more application programs 3132, program modules 3134 and program data 3136. Examples of computer operating systems conventionally employed include the NUCLEUS® operating system, the LINUX® operating system, and others, for example, providing capability for supporting application programs 3132 using, for example, code modules written in the C++® computer programming language. The application programs 3132 and/or the program modules 3134 can also include a temporal-variation-amplifier (as shown in 3048 in FIG. 30) and a vital sign generator (as shown in 3049 in FIG. 31). In some implementations, the temporal-variation-amplifier 3048 in the application programs 3132 and/or the program modules 3134 includes a skin-pixel-identifier 1402, a frequency-filter 1406, regional facial clusterial module 1408 and a frequency filter 1410 as in FIG. 14 and 15. In some implementations, the temporal-variation-amplifier 3048 in application programs 3132 and/or the program modules 3134 includes a skin-pixel-identifier 1402, a spatial bandpass-filter 1602, regional facial clusterial module 1408 and a temporal bandpass filter 1604 as in FIG. 16. In some implementations, the temporal-variation-amplifier 3048 in the application programs 3132 and/or the program modules 3134 includes a pixel-examiner 1702, a temporal variation determiner 1706 and signal processor 1708 as in FIG. 17. In some implementations, the temporal-variation-amplifier 3048 in the application programs 3132 and/or the program modules 3134 includes a skin-pixel-identification module 1802, a frequency-filter module 1808, spatial-cluster module 1812 and a frequency filter module 1816 as in FIG. 18 and 19. In some implementations, the temporal-variation-amplifier 3048 in the application programs 3132 and/or the program modules 3134 includes a skin-pixel-identification module 1802, a spatial bandpass filter module 2102, a spatial-cluster module 1812 and a temporal bandpass filter module 2106 as in FIG. 21. In some implementations, the temporal-variation-amplifier 3048 in the application programs 3132 and/or the program modules 3134 includes a pixel-examination-module 2202, a temporal variation determiner module 2206 and a signal processing module 2210 as in FIG. 22. The solid-state image transducer 128 captures images 130 that are processed by the temporal-variation-amplifier 3048 and the vital sign generator 3049 to generate the vital sign(s) 1416 that is displayed by display 3150 or transmitted by computation resource 3102, enunciated by a speaker or stored in program data 3136.

A user can enter commands and information into computation resource 3102 through input devices such as input media 3138 (e.g., keyboard/keypad, tactile input or pointing device, mouse, foot-operated switching apparatus, joystick, touchscreen or touchpad, microphone, antenna etc.). Such input devices 3138 are coupled to the processing unit 3104 through a conventional input/output interface 3142 that is, in turn, coupled to the system bus. Display 3150 or other type of display device is also coupled to the system bus 3108 via an interface, such as a video adapter 3152.

The computation resource 3102 can include capability for operating in a networked environment using logical connections to one or more remote computers, such as a remote computer 3160. The remote computer 3160 can be a personal computer, a server, a router, a network PC, a peer device or other common network node, and typically includes many or all of the elements described above relative to the computation resource 3102. In a networked environment, program modules depicted relative to the computation resource 3102, or portions thereof, can be stored in a remote memory storage device such as can be associated with the remote computer 3160. By way of example, remote application programs 3162 reside on a memory device of the remote computer 3160. The logical connections represented in FIG. 31 can include interface capabilities, e.g., such as interface capabilities in FIG. 12, a storage area network (SAN, not illustrated in FIG. 31), local area network (LAN) 3172 and/or a wide area network (WAN) 3174, but can also include other networks.

Such networking environments are commonplace in modern computer systems, and in association with intranets and the Internet. In certain implementations, the computation resource 3102 executes an Internet Web browser program (which can optionally be integrated into the operating system 3130), such as the "Internet Explorer®" Web browser manufactured and distributed by the Microsoft Corporation of Redmond, Washington.

When used in a LAN-coupled environment, the computation resource 3102 communicates with or through the local area network 3172 via a network interface or adapter 3176 and typically includes interfaces, such as a modem 3178, or other apparatus, for establishing communications with or through the WAN 3174, such as the Internet. The modem 3178, which can be internal or external, is coupled to the system bus 3108 via a serial port interface.

In a networked environment, program modules depicted relative to the computation resource 3102, or portions thereof, can be stored in remote memory apparatus. It will be appreciated that the network connections shown are exemplary, and other means of establishing a communications link between various computer systems and elements can be used.

A user of a computer can operate in a networked environment using logical connections to one or more remote computers, such as a remote computer 3160, which can be a personal computer, a server, a router, a network PC, a peer device or other common network node. Typically, a remote computer 3160 includes many or all of the elements described above relative to the computer 3100 of FIG. 31.

The computation resource 3102 typically includes at least some form of computer-readable media. Computer-readable media can be any available media that can be accessed by the computation resource 3102. By way of example, and not limitation, computer-readable media can comprise computer storage media and communication media.

Computer storage media include volatile and nonvolatile, removable and non-removable media, implemented in any method or technology for storage of information, such as computer-readable instructions, data structures, program modules or other data. The term "computer storage media" includes, but is not limited to, RAM, ROM, EEPROM, FLASH memory or other memory technology, CD, DVD, or other optical storage, magnetic cassettes, magnetic tape, magnetic disk storage or other magnetic storage devices, or any other media which can be used to store computer-intelligible information and which can be accessed by the computation resource 3102.

Communication media typically embodies computer-readable instructions, data structures, program modules or other data, represented via, and determinable from, a modulated data signal, such as a carrier wave or other transport mechanism, and includes any information delivery media. The term "modulated data signal" means a signal that has one or more of its characteristics set or changed in such a manner as to encode information in the signal in a fashion amenable to computer interpretation.

By way of example, and not limitation, communication media include wired media, such as wired network or direct-wired connections, and wireless media, such as acoustic, RF, infrared and other wireless media. The scope of the term computer-readable media includes combinations of any of the above.

FIG. 32 is a representation of display 3200 that is presented on the display device of apparatus in FIG. 1-10, according to an implementation.

Some implementations of display 3200 include a representation of three detection modes 3202, a first detection mode being detection and display of surface temperature, a second detection mode being detection and display of body temperature and a third detection mode being detection and display of room temperature.

Some implementations of display 3200 include a representation of Celsius 3204 that is activated when the apparatus is in Celsius mode.

Some implementations of display 3200 include a representation of a sensed temperature 3206.

Some implementations of display 3200 include a representation of Fahrenheit 3208 that is activated when the apparatus is in Fahrenheit mode.

Some implementations of display 3200 include a representation of a mode 3210 of site temperature sensing, a first site mode being detection of an axillary surface temperature, a second site mode being detection of an oral temperature, a third site mode being detection of a rectal temperature and a fourth site mode being detection of a core temperature.

Some implementations of display 3200 include a representation of a temperature traffic light 3212, in which a green traffic light indicates that the temperature 120 is good; an amber traffic light indicates that the temperature 120 is low; and a red traffic light indicates that the temperature 120 is high.

Some implementations of display 3200 include a representation of a probe mode 3214 that is activated when the sensed temperature 3206 is from a contact sensor.

Some implementations of display 3200 include a representation of the current time/date 3216 of the apparatus.

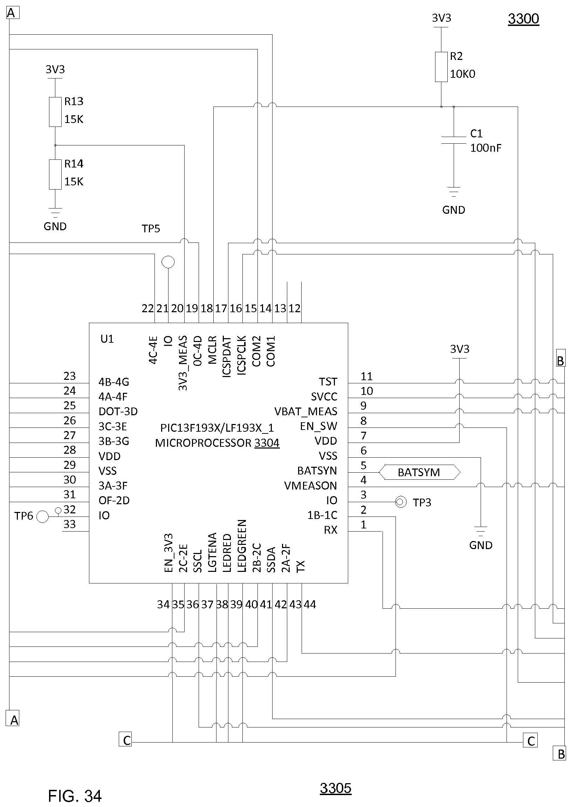

FIG. 33-37 are schematics of the electronic components of a non-touch thermometer 3300 having a digital IR sensor. FIG. 33 is a portion of the schematic of the non-touch thermometer 3300 having a digital IR sensor, according to an implementation. As discussed above in regards to FIG. 2 and FIG. 3, thermal isolation of the digital IR sensor is an important feature. In a second circuit board 3301, a digital IR sensor 108 is thermally isolated from the heat of the microprocessor 3304 (shown in FIG. 34) through a first digital interface 3302. The digital IR sensor 108 is not mounted on the same circuit board 3305 as the microprocessor 3304 (shown in FIG. 34) which reduces heat transfer from a first circuit board 3305 to the digital IR sensor 108. The non-touch thermometer 3300 also includes a second circuit board 3301, the second circuit board 3301 including a second digital interface 3312, the second digital interface 3312 being operably coupled to the first digital interface 3302 and a digital infrared sensor 108 being operably coupled to the second digital interface 3312, the digital infrared sensor 108 having ports that provide only digital readout. The microprocessor 3304 (shown in FIG. 34) is operable to receive from the ports that provide only digital readout a digital signal that is representative of an infrared signal generated by the digital infrared sensor 108 and the microprocessor 3304 (shown in FIG. 34) is operable to determine a temperature from the digital signal that is representative of the infrared signal. The first circuit board 3305 includes all of the components in FIG. 33, FIG. 34 and FIG. 35 other than the second circuit board 3301, the digital IR sensor 108 and the second digital interface 3312.

FIG. 34 is a portion of the schematic of the non-touch thermometer 3300 having the digital IR sensor, according to an implementation. A non-touch thermometer 3300 includes a first circuit board 3305, the first circuit board 3305 including the microprocessor 3304.

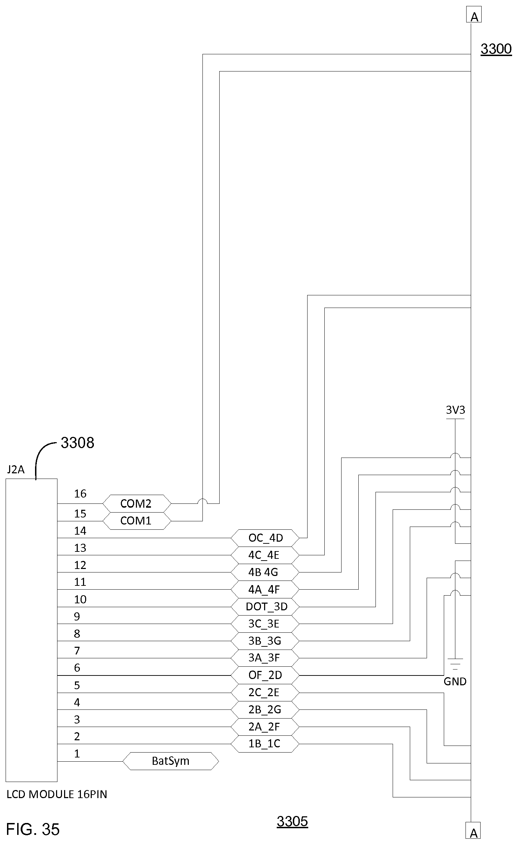

FIG. 35 is a portion of the schematic of the non-touch thermometer 3300 having the digital IR sensor, according to an implementation. The first circuit board 3305 includes a display device that is operably coupled to the microprocessor 3304 through a display interface 3308.

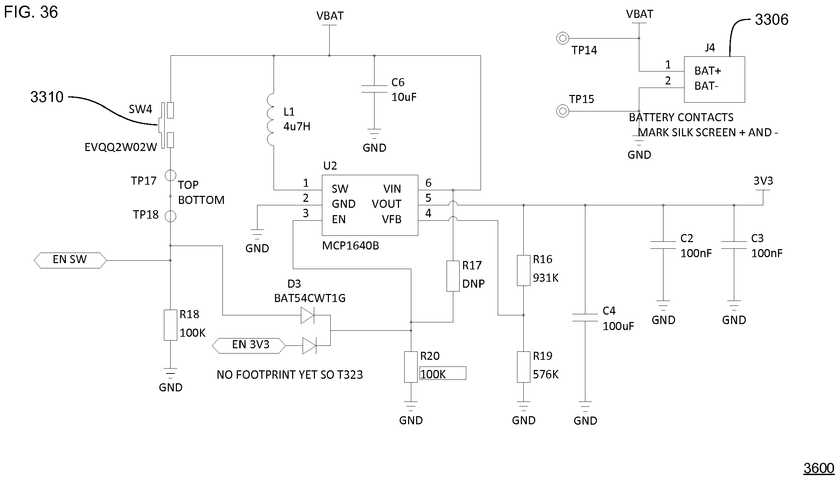

FIG. 36 is a circuit 3600 that is a portion of the schematic of the non-touch thermometer 3300 having the digital IR sensor, according to an implementation. Circuit 3600 includes a battery 3306 that is operably coupled to the microprocessor 3304, a single button 3310 that is operably coupled to the microprocessor 3304.

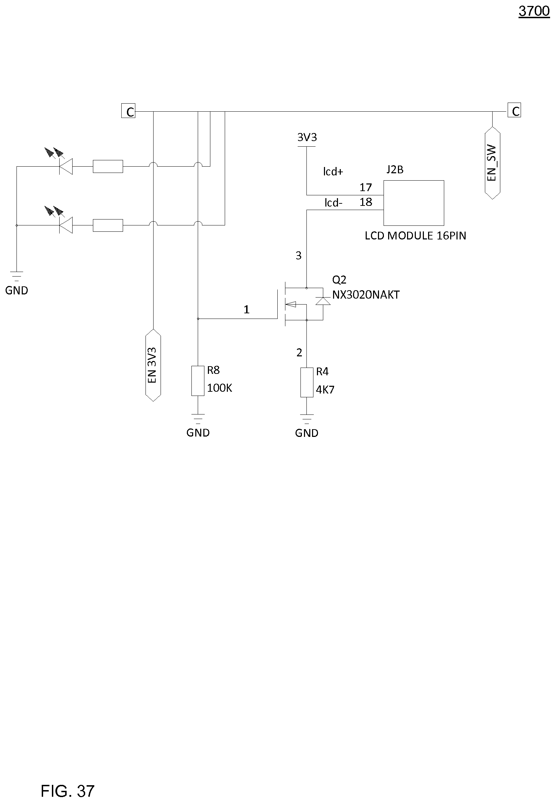

FIG. 37 is a circuit 3700 that is a portion of the schematic of the non-touch thermometer 3300 having the digital IR sensor, according to an implementation.

The non-touch thermometer further includes a housing, and where the battery 104 is fixedly attached to the housing. The non-touch thermometer where an exterior portion of the housing further includes a magnet.

In some implementations, the microprocessor 102, microprocessor 404, microprocessor 3304 in FIG. 33, main processor 3002 in FIG. 30 or processing unit 3104 in FIG. 31 that do not use the digital infrared sensor 108 can be a digital signal processor (DSP) that is specialized for signal processing such as the Texas Instruments® C6000 series DSPs, the Freescale® MSC81xx family.

In some implementations, the microprocessor 404, microprocessor 3304 in FIG. 33, main processor 3002 in FIG. 30 or processing unit 3104 in FIG. 31 can be a graphics processing unit GPU that use a specialized electronic circuit designed to rapidly manipulate and alter memory to accelerate the creation of images in a frame buffer, such as the Nvidia® GeForce 8 series.

In some implementations, the microprocessor 102, microprocessor 404, microprocessor 3304 in FIG. 33, main processor 3002 in FIG. 30 or processing unit 3104 in FIG. 31 can be field-programmable gate array (FPGA) that is an integrated circuit designed to be configured by a customer or a designer after manufacturing according to a hardware description language (HDL) using programmable logic components called "logic blocks", and a hierarchy of reconfigurable interconnects that allow the blocks to be "wired together" that are changeable logic gates that can be inter-wired in different configurations that perform analog functions and/or digital functions. Logic blocks can be configured to perform complex combinational functions, or merely simple logic gates such as AND and XOR. In most FPGAs, the logic blocks also include memory elements, which may be simple flip-flops or more complete blocks of memory.

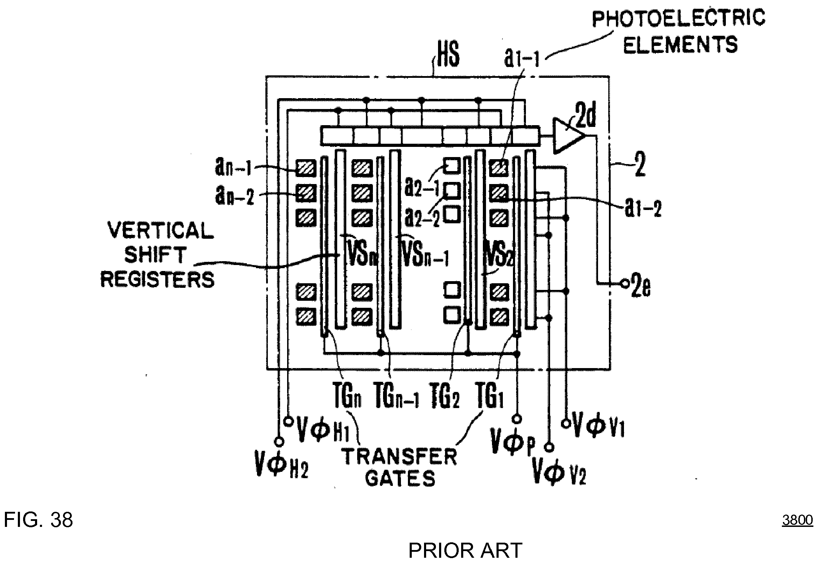

FIG. 38 is a block diagram of a solid-state image transducer 3800, according to an implementation. The solid-state image transducer 3800 includes a great number of photoelectric elements, $a_{1,1}$, $a_{2,1}$, ... , $a_{mn}$, in the minute segment form, transfer gates TG1, TG2, ... , TGn responsive to a control pulse $V_{\varphi P}$ for transferring the charges stored on the individual photoelectric elements as an image signal to vertical shift registers VS1, VS2, ... , VSn, and a horizontal shift register HS for transferring the image signal from the vertical shift registers VSs, through a buffer amplifier 2d to an outlet 2e. After the one-frame image signal is stored, the image signal is transferred to vertical shift register by the pulse $V_{\varphi P}$ and the contents of the vertical shift registers VSs are transferred upward line by line in response to a series of control pulses $V_{\varphi V1}$, $V_{\varphi V2}$. During the time interval between the successive two vertical transfer control pulses, the horizontal shift register HS responsive to a series of control pulses $V_{\varphi H1}$, $V_{\varphi H2}$ transfers the contents of the horizontal shift registers HSs in each line row by row to the right as viewed in figure 38. As a result, the one-frame image signal is formed by reading out the outputs of the individual photoelectric elements in such order.

A non-touch biologic detector or thermometer that senses temperature through a digital infrared sensor, an analog sensor or other non-touch electromagnetic sensor is described. A technical effect of the apparatus and methods disclosed herein is display and communication of a body core temperature that is estimated from signals from the non-touch electromagnetic sensor to a much higher accuracy than previous systems. Another technical effect of the apparatus and methods disclosed herein is generating a temporal variation of images from which a vital sign can be determined and displayed or stored. Although specific implementations are illustrated and described herein, it will be appreciated by those of ordinary skill in the art that any arrangement which is generated to achieve the same purpose may be substituted for the specific implementations shown. This application is intended to cover any adaptations or variations.

In particular, one of skill in the art will readily appreciate that the names of the methods and apparatus are not intended to limit implementations. Furthermore, additional methods and apparatus can be added to the modules, functions can be rearranged among the modules, and new modules to correspond to future enhancements and physical devices used in implementations can be introduced without departing from the scope of implementations. One of skill in the art will readily recognize that implementations are applicable to future non-touch temperature sensing devices, different temperature measuring sites on humans or animals and new display devices.

The terminology used in this application meant to include all temperature sensors, processors and operator environments and alternate technologies which provide the same functionality as described herein.

**Claims**

1. An apparatus to estimate a body core temperature from an external source point, the apparatus comprising:

   a housing (100);
   a non-touch electromagnetic sensor (108) operably mounted to the housing (100), the non-touch electromagnetic sensor (108) being operable to receive electromagnetic energy from the external source point of a subject and operable to generate a numerical representation of the electromagnetic energy of the external source point;
   a microprocessor (102) mounted in the housing, electrically coupled to the non-touch electromagnetic sensor (108) and operable to estimate the body core temperature of the subject from the numerical representation of the electromagnetic energy of the external source point,
   wherein estimating the body core temperature of the subject further comprises:
   calculating the body core temperature based on a cubic relationship representing three thermal ranges between the numerical representation of the electromagnetic energy of the external source point and the body core temperature, wherein the cubic relationship includes a coefficient representative of different relationships between the external source point and the body core temperature in the three thermal ranges;
   a button (106) operably coupled to the microprocessor (102); and
   a display device (114) operably coupled to the microprocessor (102) that is operable to display the body core temperature,
   **characterised in that** the cubic relationship is

   $$T_B = AT_{Skin}^3 + BT_{Skin}^2 + CT_{Skin} + D - E\left(T_{Ambient} - 75\right), T_{Ambient} < T_1 \text{ or } T_{Ambient} >$$
   $$T_2 \text{ and } T_B = AT_{Skin}^3 + BT_{Skin}^2 + CT_{Skin} + D, \ T_1 < T_{Ambient} < T_2$$

   where $T_B$ is the body core temperature, $T_{skin}$ is the numerical representation of the electromagnetic energy of the external source point, A is 0.0002299688, B is -0.0464237524, C is 3.05944877, D is 31.36205 and E is 0.135, where $T_{ambient}$ is an ambient temperature, where $T_1$ and $T_2$ are boundaries between the three thermal ranges and $T_1$ and $T_2$ are selected from a group of pairs of ambient temperatures consisting of 19.4°C (67°F) and 27.8°C (82°F); 30.6°C (87°F) and 35.0°C (95°F); and 30.0°C (86°F) and 38.3°C (101°F).

2. The apparatus of claim 1, wherein the non-touch electromagnetic sensor (108) further comprises:
   an analog infrared sensor.

3. The apparatus of claim 1, wherein the non-touch electromagnetic sensor (108) further comprises:
   a digital infrared sensor.

4. The apparatus of claim 3, wherein the digital infrared sensor (108) further comprises:
   being operably coupled to the microprocessor with no analog-to-digital converter operably coupled between the digital infrared sensor and the microprocessor, the digital infrared sensor having only digital readout ports, the digital infrared sensor having no analog sensor readout ports.

5. The apparatus of claim 1, wherein microprocessor (102) further comprises:

   a temporal-variation-amplifier of a plurality of images that is operable to generate a temporal variation;
   a vital-sign generator that is operably coupled to the temporal-variation-amplifier that is operable to generate vital sign from the temporal variation; and
   the display device being operably coupled to the vital-sign generator and that is operable to display the vital sign.

6. The apparatus of claim 5, wherein the temporal-variation-amplifier further comprises:

a skin-pixel-identifier that identifies pixel values that are representative of skin in the plurality of images;
a first frequency filter that is operably coupled to the skin-pixel-identifier and that is applied to output of the skin-pixel-identifier;
a regional facial clusterial module that is operably coupled to the first frequency filter and that applies spatial clustering to output of the first frequency filter; and
a second frequency filter that is operably regional facial clusterial module and that is applied to output of the regional facial clusterial module, generating the temporal variation.

7. The apparatus of claim 6, wherein the skin-pixel-identifier further comprises:
an automatic seed point based clustering apparatus.

8. The apparatus of claim 6, wherein the regional facial clusterial module further comprises:
a fuzzy clusterer.

9. The apparatus of claim 6, wherein the regional facial clusterial module further comprises:
an expectation-maximizer.

10. The apparatus of claim 6, wherein the first frequency filter further comprises:
a one-dimensional spatial Fourier Transformation apparatus.

11. The apparatus of claim 6, wherein the first frequency filter further comprises:
a high pass filter.

**Patentansprüche**

1. Vorrichtung zur Schätzung einer Körperkerntemperatur aus einem externen Quellenpunkt, wobei die Vorrichtung umfasst:

ein Gehäuse (100);
einen berührungslosen elektromagnetischen Sensor (108), der am Gehäuse (100) wirkmäßig montiert ist, wobei der berührungslose elektromagnetische Sensor (108) betätigbar ist, um elektromagnetische Energie aus dem externen Quellenpunkt einer Person zu empfangen, und betätigbar ist, um eine numerische Darstellung der elektromagnetischen Energie des externen Quellenpunkts zu erzeugen;
einen Mikroprozessor (102), der im Gehäuse montiert ist, der mit dem berührungslosen elektromagnetischen Sensor (108) elektrisch gekoppelt ist und betätigbar ist, um die Körperkerntemperatur der Person aus der numerischen Darstellung der elektromagnetischen Energie des externen Quellenpunkts zu schätzen,
worin die Schätzung der Körperkerntemperatur der Person ferner umfasst:

Berechnen der Körperkerntemperatur aufgrund eines kubischen Zusammenhangs, der drei thermische Bereiche zwischen der numerischen Darstellung der elektromagnetischen Energie des externen Quellenpunkts und der Körperkerntemperatur darstellt, worin der kubische Zusammenhang einen Koeffizienten umfasst, der die unterschiedlichen Zusammenhänge zwischen dem externen Quellenpunkt und der Körperkerntemperatur in den drei thermischen Bereichen darstellt;
einen Druckknopf (106), der mit dem Mikroprozessor (102) wirkmäßig gekoppelt ist; und
ein Anzeigegerät (114), das mit dem Mikroprozessor (102) wirkmäßig gekoppelt ist, das betätigbar ist, um die Körperkerntemperatur anzuzeigen,

**dadurch gekennzeichnet, dass** der kubische Zusammenhang

$$T_B = AT_{Skin}^3 + BT_{Skin}^2 + CT_{Skin} + D - E(T_{Ambient} - 75), T_{Ambient} < T_1 \ or \ T_{Ambient} >$$

$$T_2 \ and \ T_B = AT_{Skin}^3 + BT_{Skin}^2 + CT_{Skin} + D, \ T_1 < T_{Ambient} < T_2$$

ist, wo $T_B$ die Körperkerntemperatur ist, $T_{skin}$ die numerische Darstellung der elektromagnetischen Energie des externen Quellenpunkts ist, A 0,0002299688 ist, B -0,0464237524 ist, C 3,05944877 ist, D 31,36205 ist und E 0,135 ist, wo $T_{ambient}$ eine Umgebungstemperatur ist, wo $T_1$ und $T_2$ Grenzen zwischen den drei thermischen Bereichen sind und $T_1$ und $T_2$ aus einer Gruppe von Paaren von Umgebungstemperaturen gewählt sind, die aus 19,4°C (67°F) und 27,8°C (82°F); 30,6°C (87°F) und 35,0°C (95°F); und 30,0°C (86°F) und 38,3°C (101°F) bestehen.

2. Vorrichtung nach Anspruch 1, worin der berührungslose elektromagnetische Sensor (108) ferner umfasst:
einen analogen Infrarotsensor.

3. Vorrichtung nach Anspruch 1, worin der berührungslose elektromagnetische Sensor (108) ferner umfasst:
einen digitalen Infrarotsensor.

4. Vorrichtung nach Anspruch 3, worin der digitale Infrarotsensor (108) ferner umfasst:
er mit dem Mikroprozessor mit keinem Analog-DigitalWandler, der zwischen dem digitalen Infrarotsensor und dem Mikroprozessor wirkmäßig gekoppelt ist, wirkmäßig gekoppelt ist, wobei der digitale Infrarotsensor nur digitale Ausleseports aufweist, wobei der digitale Infrarotsensor keine analogen Sensor-Ausleseports aufweist.

5. Vorrichtung nach Anspruch 1, worin der Mikroprozessor (102) ferner umfasst:

einen zeitlichen Änderungsverstärker von einer Vielzahl von Bildern, der betätigbar ist, um eine zeitliche Änderung zu erzeugen;
einen Vitalzeichenerzeuger, der mit dem zeitlichen Änderungsverstärker wirkmäßig gekoppelt ist und der betätigbar ist, um ein Vitalzeichen aus der zeitlichen Änderung zu erzeugen; und
wobei das Anzeigegerät mit dem Vitalzeichenerzeuger wirkmäßig gekoppelt ist und betätigbar ist, um das Vitalzeichen anzuzeigen.

6. Vorrichtung nach Anspruch 5, worin der zeitliche Änderungsverstärker ferner umfasst:

einen Hautpixelidentifikator, der Pixelwerte identifiziert, die die Haut in der Vielzahl von Bildern darstellen;
einen ersten Frequenzfilter, der mit dem Hautpixelidentifikator wirkmäßig gekoppelt ist und auf dem Ausgang des Hautpixelidentifikators appliziert ist;
ein regionales Gesichtsclustermodul, das mit dem ersten Frequenzfilter wirkmäßig gekoppelt ist und räumliche Clusterbildung auf dem Ausgang des ersten Frequenzfilters appliziert; und
einen zweiten Frequenzfilter, der mit dem regionalen Gesichtsclustermodul wirkmäßig gekoppelt ist und auf dem Ausgang des regionalen Gesichtsclustermoduls appliziert ist, wodurch die zeitliche Änderung erzeugt wird.

7. Vorrichtung nach Anspruch 6, worin der Hautpixelidentifikator ferner umfasst:
eine automatische saatpunkt-basierte Clusterbildungsvorrichtung.

8. Vorrichtung nach Anspruch 6, worin das regionale Gesichtsclustermodul ferner umfasst:
einen Fuzzy-Clusterer.

9. Vorrichtung nach Anspruch 6, worin das regionale Gesichtsclustermodul ferner umfasst:
einen Erwartungsmaximierer.

10. Vorrichtung nach Anspruch 6, worin der erste Frequenzfilter ferner umfasst:
eine eindimensionale räumliche Fourier-Transformationsvorrichtung.

11. Vorrichtung nach Anspruch 6, worin der erste Frequenzfilter ferner umfasst:
einen Hochpassfilter.

**Revendications**

1. Appareil pour estimer une température interne du corps à partir d'un point source externe, l'appareil comprenant :

un boîtier (100) ;
un capteur électromagnétique sans contact (108) monté de façon fonctionnelle sur le boîtier (100), le capteur électromagnétique sans contact (108) étant agencé pour recevoir de l'énergie électromagnétique du point d'origine externe d'un sujet, et étant agencé pour générer une représentation numérique de l'énergie électromagnétique du point source externe ;

un microprocesseur (102) monté dans le boîtier, couplé électriquement au capteur électromagnétique sans contact (108) et agencé pour estimer la température interne du corps du sujet à partir de la représentation numérique de l'énergie électromagnétique du point source externe,

dans lequel l'estimation de la température interne du corps du sujet comprend également l'étape consistant à : calculer la température interne du corps représentant trois plages thermiques entre la représentation numérique de l'énergie électromagnétique du point source externe et la température interne du corps, dans lequel la relation cubique comprend un coefficient représentant les relations différentes entre le point source externe et la température interne du corps dans les trois plages thermiques ;

un poussoir (106) couplé de façon fonctionnelle au microprocesseur (102) ; et un dispositif d'affichage (114) couplé de façon fonctionnelle au microprocesseur (102) qui est agencé pour afficher la température interne du corps,

**caractérisé en ce que** la relation cubique est

$$T_B = AT_{Skin}^3 + BT_{Skin}^2 + CT_{Skin} + D - E(T_{ambient} - 75), T_{ambient} < T_1 \text{ or } T_{ambient} >$$
$$T_2 \text{ and } T_B = AT_{Skin}^3 + BT_{Skin}^2 + CT_{Skin} + D, \qquad T_1 < T_{ambient} < T_2$$

où $T_B$ est la température interne du corps, $T_{skin}$ est la représentation numérique de l'énergie électromagnétique du point source externe, A est 0,0002299688, B est -0,0464237524, C est 3,05944877, D est 31,36205 et E est 0,135, où $T_{ambient}$ est une température ambiante, où $T_1$ et $T_2$ sont des limites entre les trois plages thermiques et $T_1$ et $T_2$ sont sélectionnés dans un groupe de paires de températures ambiantes, qui sont 19,4°C (67°F) et 27,8°C (82°F); 30,6°C (87°F) et 35,0°C (95°F); et 30,0°C (86°F) et 38,3°C (101°F).

2. Appareil selon la revendication 1, dans lequel le capteur électromagnétique son contact (108) comprend également : un capteur infrarouge analogique.

3. Appareil selon la revendication 1, dans lequel le capteur électromagnétique son contact (108) comprend également : un capteur infrarouge numérique.

4. Appareil selon la revendication 3, dans lequel le capteur infrarouge numérique (108) comprend également : un couplage fonctionnel avec le microprocesseur sans aucun convertisseur analogique-numérique couplé de façon fonctionnelle entre le capteur infrarouge numérique et le microprocesseur, le capteur infrarouge numérique n'ayant que des ports de lecture numériques, le capteur infrarouge numérique n'ayant pas de ports de lecture de capteur analogiques.

5. Appareil selon la revendication 1, dans lequel le microprocesseur (102) comprend également :

un amplificateur à variation temporelle d'une pluralité d'images, agencé pour générer une variation temporelle ; un générateur de signes vitaux, couplé de façon fonctionnelle à l'amplificateur à variation temporelle, qui est agencé pour générer un signe vital à partir de la variation temporelle ; le dispositif d'affichage étant couplé de façon fonctionnelle au générateur de signes vitaux.

6. Appareil selon la revendication 5, dans lequel l'amplificateur à variation temporelle comprend également : un identificateur de pixels de peau, identifiant les valeurs des pixels représentant la peau dans la pluralité d'images ; un premier filtre de fréquence couplé de façon fonctionnelle à l'identificateur de pixels de peau, et appliqué à la sortie de l'identificateur de pixels de peau ; un module de clustering facial régional couplé de façon fonctionnelle au premier filtre de fréquence et appliquant un clustering spatial à la sortie du premier filtre de fréquence ; et un deuxième filtre de fréquence qui est un module de clustering facial régional fonctionnel et qui est appliqué à la sortie du module de clustering facial régional, générant la variation temporelle.

7. Appareil selon la revendication 6, dans lequel l'identificateur de pixels de peau comprend également : un appareil de clustering automatique à base de points germes.

8. Appareil selon la revendication 6, dans lequel le module de clustering facial régional comprend également : un dispositif de clustering flou.

9. Appareil selon la revendication 6, dans lequel le module de clustering facial régional comprend également : un maximisateur d'espérance.

10. Appareil selon la revendication 6, dans lequel le premier filtre de fréquence comprend également : un appareil d'application de transformée de Fourier spatiale monodimensionnelle.

11. Appareil selon la revendication 6, dans lequel le premier filtre de fréquence comprend également : un filtre passe-haut.

FIG. 1

DIGITAL INFRARED
SENSOR 108

INFRARED
SIGNAL
116

AMBIENT
AIR
SENSOR
122

200

ANALOG PORT(S)
204

DIGITAL PORT(S)
110

A/D 202

BUTTON
106

DIGITAL
READOUT
SIGNAL(S) 112

MICROPROCESSOR 102

CALIBRATION
DIFFERENCE 408

TEMPERATURE
ESTIMATOR 118

BIAS 410

BATTERY
104

VITAL SIGN
GENERATOR
134

TEMPORAL-
VARIATION-
AMPLIFIER 132

ESTIMATED
BODY
TEMPERATURE
412

VITAL
SIGN(S)
136

AT LEAST
TWO IMAGES
130

DISPLAY DEVICE 114

ESTIMATED
BODY
TEMPERATURE
412

VITAL
SIGN(S)
136

SOLID-STATE
IMAGE
TRANSDUCER 128

FIG. 2

DIGITAL INFRARED
SENSOR 108

INFRARED
SIGNAL
116

DIGITAL PORT(S)
110

AMBIENT
AIR
SENSOR
122

300

BUTTON
106

DIGITAL
READOUT
SIGNAL(S) 112

MICROPROCESSOR 102

TEMPERATURE
ESTIMATOR 118

CALIBRATION
DIFFERENCE 408

BIAS 410

BATTERY
104

VITAL SIGN
GENERATOR
134

TEMPORAL-
VARIATION-
AMPLIFIER 132

ESTIMATED
BODY
TEMPERATURE
412

VITAL
SIGN(S)
136

AT LEAST
TWO IMAGES
130

LED COLOR DISPLAY DEVICE 302

ESTIMATED
BODY
TEMPERATURE
412

VITAL
SIGN(S)
136

SOLID-STATE
IMAGE
TRANSDUCER 128

FIG. 3

FIG. 4

NON-TOUCH ELECTROMAGNETIC SENSOR 402

INFRARED SIGNAL 116

AMBIENT AIR SENSOR 122

BUTTON 106

400

MICROPROCESSOR 404

CALIBRATION DIFFERENCE 408

BIAS 410

CUBIC TEMPERATURE ESTIMATOR 406

BATTERY 104

ESTIMATED BODY TEMPERATURE 412

DISPLAY DEVICE 114

ESTIMATED BODY TEMPERATURE 412

ANALOG INFRARED SENSOR 502

INFRARED SIGNAL 116

AMBIENT AIR SENSOR 122

500

BUTTON 106

MICROPROCESSOR 404

BATTERY 104

CUBIC TEMPERATURE ESTIMATOR 406

CALIBRATION DIFFERENCE 408

BIAS 410

ESTIMATED BODY TEMPERATURE 412

DISPLAY DEVICE 114

ESTIMATED BODY TEMPERATURE 412

FIG. 5

DIGITAL INFRARED
SENSOR 108

INFRARED SIGNAL 116

AMBIENT
AIR
SENSOR
122

600

DIGITAL PORT(S)
110

BUTTON
106

DIGITAL
READOUT
SIGNAL(S) 112

MICROPROCESSOR 404

BATTERY
104

CUBIC
TEMPERATURE
ESTIMATOR 406

CALIBRATION
DIFFERENCE 408

BIAS 410

ESTIMATED BODY
TEMPERATURE 412

DISPLAY DEVICE 114

ESTIMATED BODY
TEMPERATURE 412

FIG. 6

700

NON-TOUCH ELECTROMAGNETIC SENSOR 402

INFRARED SIGNAL 116

AMBIENT AIR SENSOR 122

BUTTON 106

MICROPROCESSOR 702

CUBIC TEMPERATURE ESTIMATOR 406

CALIBRATION DIFFERENCE 408

BIAS 410

BATTERY 104

VITAL SIGN GENERATOR 134

TEMPORAL-VARIATION-AMPLIFIER 132

ESTIMATED BODY TEMPERATURE 412

VITAL SIGN(S) 136

AT LEAST TWO IMAGES 130

DISPLAY DEVICE 114

ESTIMATED BODY TEMPERATURE 412

VITAL SIGN(S) 136

SOLID-STATE IMAGE TRANSDUCER 128

FIG. 7

ANALOG INFRARED
SENSOR 502

INFRARED
SIGNAL
116

AMBIENT
AIR
SENSOR
122

800

BUTTON
106

MICROPROCESSOR 702

CALIBRATION
DIFFERENCE 408

CUBIC
TEMPERATURE
ESTIMATOR 406

BIAS 410

BATTERY
104

VITAL SIGN
GENERATOR
134

TEMPORAL-
VARIATION-
AMPLIFIER 132

ESTIMATED
BODY
TEMPERATURE
412

VITAL
SIGN(S)
136

AT LEAST
TWO IMAGES
130

DISPLAY DEVICE 114

ESTIMATED
BODY
TEMPERATURE
412

VITAL
SIGN(S)
136

SOLID-STATE
IMAGE
TRANSDUCER 128

FIG. 8

FIG. 9

INFRARED
SIGNAL
116

AMBIENT
AIR
SENSOR
122

1000

DIGITAL INFRARED
SENSOR 108

ANALOG PORT(S)
204

DIGITAL PORT(S)
110

A/D 202

BUTTON
106

DIGITAL
READOUT
SIGNAL(S) 112

MICROPROCESSOR 702

CUBIC
TEMPERATURE
ESTIMATOR 406

CALIBRATION
DIFFERENCE 408

BIAS 410

BATTERY
104

VITAL SIGN
GENERATOR
134

TEMPORAL-
VARIATION-
AMPLIFIER 132

ESTIMATED
BODY
TEMPERATURE
412

VITAL
SIGN(S)
136

AT LEAST
TWO IMAGES
130

DISPLAY DEVICE 114

ESTIMATED
BODY
TEMPERATURE
412

VITAL
SIGN(S)
136

SOLID-STATE
IMAGE
TRANSDUCER 128

FIG. 10

1100

1102 — RECEIVE FROM DIGITAL READOUT PORTS OF A DIGITAL INFRARED SENSOR A DIGITAL SIGNAL THAT IS REPRESENTATIVE OF AN INFRARED SIGNAL DETECTED BY THE DIGITAL INFRARED SENSOR

1104 — DETERMINE A TEMPERATURE FROM THE DIGITAL SIGNAL THAT IS REPRESENTATIVE OF THE INFRARED SIGNAL

FIG. 11

1200

1201 — RECEIVE A TEMPERATURE

1202 — TEMP IS IN RANGE OF 32.0°C AND 37.3°C ?

NO

YES

SET COLOR TO AMBER 1204

1208 — TEMP IS IN RANGE OF 37.4°C AND 38.0°C ?

NO

YES

SET COLOR TO GREEN 1210

1212 — TEMP IS > 38.0°C ?

NO

YES

SET COLOR TO RED 1214

1206 — ACTIVATE DISPLAY DEVICE IN ACCORDANCE WITH THE COLOR

FIG. 12

YES

IN USE ? 1302

1300

NO

1302 — POWER OFF ALL MODULES OTHER THAN POWER SUPPLY

NO

IN USE ? 1306

YES

1308 — POWER ON ONLY THE MICROPROCESSOR, DIGITAL IR SENSOR AND LCD FOR 1 SECOND

1310 — READ THE TEMPERATURE MEASUREMENT AND GENERATE THE BODY CORE TEMPERATURE RESULT

1312 — POWER OFF THE DIGITAL IR SENSOR

1314 — POWER ON THE DISPLAY BACK-LIGHT AND TEMPERATURE RANGE INDICATOR

1316 — DISPLAY THE BODY CORE TEMPERATURE RESULT

FIG. 13

1400

IMAGES
1404

SKIN-PIXEL-
IDENTIFIER 1402

FREQUENCY-
FILTER 1406

REGIONAL
FACIAL
CLUSTERIAL
MODULE 1408

FREQUENCY-
FILTER 1410

TEMPORAL-
VARIATION
IDENTIFIER
1412

VITAL-SIGN
GENERATOR
1414

VITAL
SIGN(S)
1416

FIG. 14

1500

IMAGES
1404

SKIN-PIXEL-
IDENTIFIER 1402

FREQUENCY-
FILTER 1406

REGIONAL
FACIAL
CLUSTERIAL
MODULE 1408

FREQUENCY-
FILTER 1410

VITAL-SIGN
GENERATOR
1414

VITAL
SIGN(S)
1416

FIG. 15

1600

IMAGES
1404

↓

SKIN-PIXEL-
IDENTIFIER 1402

↓

SPATIAL
BANDPASS
FILTER 1602

↓

REGIONAL
FACIAL
CLUSTERIAL
MODULE 1408

↓

TEMPORAL
BANDPASS
FILTER 1604

↓

TEMPORAL-
VARIATION
IDENTIFIER
1412

↓

VITAL-SIGN
GENERATOR
1414

↓

VITAL
SIGN(S)
1416

FIG. 16

1700

IMAGES
1404

PIXEL-
EXAMINER 1702

TEMPORAL
VARIATION
DETERMINER
1706

SIGNAL-
PROCESSOR
1708

VITAL-SIGN
GENERATOR
1414

VITAL
SIGN(S)
1416

FIG. 17

1800

IMAGES
1404

SKIN-PIXEL-
IDENTIFICATION
MODULE 1802

IDENTIFIED
SKIN
PIXELS 1806

FREQUENCY-
FILTER
MODULE 1808

FREQUENCY FILTERED
IDENTIFIED PIXEL
VALUES OF THE SKIN
1810

REGIONAL
FACIAL
CLUSTERIAL
MODULE 1812

SPATIAL CLUSTERED
FREQUENCY FILTERED
IDENTIFIED PIXEL
VALUES OF SKIN 1814

1901

TEMPORAL
VARIATION
1822

TEMPORAL-
VARIATION
MODULE 1820

FREQUENCY
FILTERED SPATIAL
CLUSTERED
FREQUENCY
FILTERED IDENTIFIED
PIXEL VALUES OF
SKIN 1818

FREQUENCY-
FILTER
MODULE 1816

FIG. 18

FIG. 19

2000

IMAGES
1404

SKIN-PIXEL-
IDENTIFICATION
MODULE 1802

IDENTIFIED
SKIN
PIXELS 1806

FREQUENCY-
FILTER
MODULE 1808

1901

FREQUENCY FILTERED
IDENTIFIED PIXEL
VALUES OF THE SKIN
1810

TEMPORAL
VARIATION
1822

REGIONAL
FACIAL
CLUSTERIAL
MODULE 1812

SPATIAL CLUSTERED
FREQUENCY FILTERED
IDENTIFIED PIXEL
VALUES OF SKIN 1814

FREQUENCY-
FILTER
MODULE 1816

FIG. 20

2100

IMAGES
1404

SKIN-PIXEL-
IDENTIFICATION
MODULE 1802

IDENTIFIED
SKIN
PIXELS 1806

SPATIAL
BANDPASS
FILTER MODULE
2102

SPATIAL BANDPASS
FILTERED IDENTIFIED
PIXEL VALUES OF SKIN
2104

REGIONAL
FACIAL
CLUSTERIAL
MODULE 1812

SPATIAL CLUSTERED
SPATIAL BANDPASSED
IDENTIFIED PIXEL
VALUES OF SKIN 2106

TEMPORAL
BANDPASS FILTER
MODULE 2108

1901

TEMPORAL
VARIATION
1822

TEMPORAL-
VARIATION
MODULE 1820

TEMPORAL
BANDPASS FILTERED
SPATIAL CLUSTERED
SPATIAL BANDPASS
FILTERED IDENTIFIED
PIXEL VALUES OF
SKIN 2110

FIG. 21

2200

IMAGES
1404

PIXEL-
EXAMINATION
MODULE 2202

EXAMINED
PIXEL
VALUES
2204

1901

TEMPORAL
VARIATION
DETERMINER
MODULE 2206

TEMPORAL
VARIATION
1822

TEMPORAL VARIATION
2208

SIGNAL-
PROCESSING
MODULE 2210

FIG. 22

IDENTIFY PIXEL VALUES OF AT LEAST TWO IMAGES
THAT ARE REPRESENTATIVE OF THE SKIN 2302

2300

APPLY A FREQUENCY FILTER TO THE IDENTIFED PIXEL
VALUES OF THE SKIN 2304

APPLY SPATIAL CLUSTERING TO THE FREQUENCY FILTERED
IDENTIFIED PIXEL VALUES OF SKIN 2306

APPLY A FREQUENCY FILTER TO THE SPATIAL CLUSTERED
FREQUENCY FILTERED IDENTIFIED PIXEL VALUES OF SKIN
2308

DETERMINE TEMPORAL VARIATION OF THE FREQUENCY
FILTERED SPATIAL CLUSTERED FREQUENCY FILTERED
IDENTIFIED PIXEL VALUES OF SKIN 2310

ANALYZE THE TEMPORAL
VARIATION TO GENERATE A
PATTERN OF FLOW OF
BLOOD 2312

ANALYZE THE TEMPORAL
VARIATION TO GENERATE BLOOD
PRESSURE 2318

DISPLAY THE PATTERN OF
FLOW OF BLOOD 2313

DISPLAY THE BLOOD PRESSURE
2319

ANALYZE THE TEMPORAL
VARIATION TO GENERATE
HEARTRATE 2314

ANALYZE THE TEMPORAL
VARIATION TO GENERATE EKG
2320

DISPLAY THE HEARTRATE
2315

DISPLAY THE EKG 2321

ANALYZE THE TEMPORAL
VARIATION TO DETERMINE
RESPIRATORY RATE 2316

ANALYZE THE TEMPORAL
VARIATION TO GENERATE PULSE
OXIMETRY 2322

DISPLAY THE
RESPIRATORY
RATE 2317

DISPLAY THE PULSE OXIMETRY
2323

FIG. 23

IDENTIFY PIXEL VALUES OF AT LEAST TWO IMAGES THAT ARE REPRESENTATIVE OF THE SKIN 2302

2400

APPLY A FREQUENCY FILTER TO THE IDENTIFED PIXEL VALUES OF THE SKIN 2304

APPLY SPATIAL CLUSTERING TO THE FREQUENCY FILTERED IDENTIFIED PIXEL VALUES OF SKIN 2306

APPLY A FREQUENCY FILTER TO THE SPATIAL CLUSTERED FREQUENCY FILTERED IDENTIFIED PIXEL VALUES OF SKIN, YIELDING A TEMPORAL VARIATION 2308

ANALYZE THE TEMPORAL VARIATION TO GENERATE A PATTERN OF FLOW OF BLOOD 2312

DISPLAY THE PATTERN OF FLOW OF BLOOD 2313

ANALYZE THE TEMPORAL VARIATION TO GENERATE HEARTRATE 2314

DISPLAY THE HEARTRATE 2315

ANALYZE THE TEMPORAL VARIATION TO DETERMINE RESPIRATORY RATE 2316

DISPLAY THE RESPIRATORY RATE 2317

ANALYZE THE TEMPORAL VARIATION TO GENERATE BLOOD PRESSURE 2318

DISPLAY THE BLOOD PRESSURE 2319

ANALYZE THE TEMPORAL VARIATION TO GENERATE EKG 2320

DISPLAY THE EKG 2321

ANALYZE THE TEMPORAL VARIATION TO GENERATE PULSE OXIMETRY 2322

DISPLAY THE PULSE OXIMETRY 2323

FIG. 24

IDENTIFY PIXEL VALUES OF AT LEAST TWO IMAGES THAT ARE REPRESENTATIVE OF THE SKIN 2302

2500

APPLY SPATIAL BANDPASS FILTER TO THE IDENTIFED PIXEL VALUES 2502

APPLY SPATIAL CLUSTERING TO THE SPATIAL BANDPASS FILTERED IDENTIFIED PIXEL VALUES OF SKIN 2504

APPLY TEMPORAL BANDPASS FILTER TO THE SPATIAL CLUSTERED SPATIAL BANDPASSED FILTERED IDENTIFIED PIXEL VALUES OF SKIN 2506

DETERMINE TEMPORAL VARIATION OF THE TEMPORAL BANDPASS FILTERED SPATIAL CLUSTERED SPATIAL BANDPASS FILTERED IDENTIFIED PIXEL VALUES OF SKIN 2508

ANALYZE THE TEMPORAL VARIATION TO GENERATE A PATTERN OF FLOW OF BLOOD 2312

DISPLAY THE PATTERN OF FLOW OF BLOOD 2313

ANALYZE THE TEMPORAL VARIATION TO GENERATE HEARTRATE 2314

DISPLAY THE HEARTRATE 2315

ANALYZE THE TEMPORAL VARIATION TO DETERMINE RESPIRATORY RATE 2316

DISPLAY THE RESPIRATORY RATE 2317

ANALYZE THE TEMPORAL VARIATION TO GENERATE BLOOD PRESSURE 2318

DISPLAY THE BLOOD PRESSURE 2319

ANALYZE THE TEMPORAL VARIATION TO GENERATE EKG 2320

DISPLAY THE EKG 2321

ANALYZE THE TEMPORAL VARIATION TO GENERATE PULSE OXIMETRY 2322

DISPLAY THE PULSE OXIMETRY 2323

FIG. 25

2600

EXAMINE PIXEL VALUES OF TWO IMAGES IN A NON-TOUCH
THERMOMETER 2602

DETERMINE TEMPORAL VARIATION OF THE EXAMINED PIXEL
VALUES 2604

APPLY SIGNAL PROCESSING TO THE PIXEL VALUE
TEMPORAL VARIATIONS TO AMPLIFY 2606

GENERATE A VITAL SIGN FROM THE AMPLIFIED PIXEL VALUE
TEMPORAL VARIATIONS 2608

FIG. 26

CROP EACH OF AT LEAST TWO IMAGES 2702

2700

IDENTIFY PIXEL VALUES OF AT THE LEAST TWO CROPPED IMAGES THAT ARE REPRESENTATIVE OF THE SKIN 2704

APPLY SPATIAL BANDPASS FILTER TO THE IDENTIFED PIXEL VALUES 2502

APPLY SPATIAL CLUSTERING TO THE SPATIAL BANDPASS FILTERED IDENTIFIED PIXEL VALUES OF SKIN 2504

APPLY TEMPORAL BANDPASS FILTER TO THE SPATIAL CLUSTERED SPATIAL BANDPASSED FILTERED IDENTIFIED PIXEL VALUES OF SKIN 2506

DETERMINE TEMPORAL VARIATION OF THE TEMPORAL BANDPASS FILTERED SPATIAL CLUSTERED SPATIAL BANDPASS FILTERED IDENTIFIED PIXEL VALUES OF SKIN 2508

ANALYZE THE TEMPORAL VARIATION TO GENERATE A PATTERN OF FLOW OF BLOOD 2312

DISPLAY THE PATTERN OF FLOW OF BLOOD 2313

ANALYZE THE TEMPORAL VARIATION TO GENERATE HEARTRATE 2314

DISPLAY THE HEARTRATE 2315

ANALYZE THE TEMPORAL VARIATION TO DETERMINE RESPIRATORY RATE 2316

DISPLAY THE RESPIRATORY RATE 2317

ANALYZE THE TEMPORAL VARIATION TO GENERATE BLOOD PRESSURE 2318

DISPLAY THE BLOOD PRESSURE 2319

ANALYZE THE TEMPORAL VARIATION TO GENERATE EKG 2320

DISPLAY THE EKG 2321

ANALYZE THE TEMPORAL VARIATION TO GENERATE PULSE OXIMETRY 2322

DISPLAY THE PULSE OXIMETRY 1623

FIG. 27

2800

2802 — RECEIVE A NUMERICAL REPRESENTATION OF ELECTROMAGNETIC ENERGY OF THE EXTERNAL SOURCE POINT OF A SUBJECT FROM A NON-TOUCH ELECTROMAGNETIC SENSOR

2804 — ESTIMATE A BODY CORE TEMPERATURE OF THE SUBJECT BASED ON A CUBIC RELATIONSHIP REPRESENTING THREE THERMAL RANGES

2806 — DISPLAY THE ESTIMATED BODY CORE TEMPERATURE

FIG. 28

2900

2802 — RECEIVE A NUMERICAL REPRESENTATION OF ELECTROMAGNETIC ENERGY OF THE EXTERNAL SOURCE POINT OF A SUBJECT FROM A NON-TOUCH ELECTROMAGNETIC SENSOR

2902 — ESTIMATE A BODY CORE TEMPERATURE OF THE SUBJECT FROM:
- THE NUMERICAL REPRESENTATION OF THE ELECTROMAGNETIC ENERGY OF THE EXTERNAL SOURCE POINT
- A REPRESENTATION OF AN AMBIENT AIR TEMPERATURE READING
- A REPRESENTATION OF A CALIBRATION DIFFERENCE, AND
- A REPRESENTATION OF A BIAS IN CONSIDERATION OF THE TEMPERATURE SENSING MODE

BASED ON A CUBIC RELATIONSHIP REPRESENTING THREE THERMAL RANGES

2806 — DISPLAY THE ESTIMATED BODY CORE TEMPERATURE

FIG. 29

3000

3000

REGULATOR — 3033

3026 — SIM/RUIM

3028 — SIM/RUIM INTERFACE

3032 — BATTERY INTERFACE

V+

3030 — BATTERY

OPERATING SYSTEM 3034

PROGRAMS 3036

MESSAGE APPLICATION 3038

DEVICE STATE MODULE 3040

PIM 3042

CONNECT MODULE 3044

IT POLICY MODULE 3046

PARSER 3047

TEMPORAL-VARIATION-AMPLIFIER 3048

VITAL-SIGN GENERATOR 3049

DISPLAY 3010

FLASH MEMORY 3008

STACK 3009

IMAGES 130

RAM 3006

COMMUNICATION SUBSYSTEM 3004

NETWORK

3005

MAIN PROCESSOR 3002

AUXILIARY I/O — 3012

DATA PORT — 3014

KEYBOARD — 3016

SPEAKER — 3018

MICROPHONE — 3020

OTHER DEVICE SUBSYSTEMS — 3024

SHORT-RANGE COMMUNICATIONS — 3022

AT LEAST TWO IMAGES 130

SOLID-STATE IMAGE TRANSDUCER — 128

FIG. 30

3100

3120

3130

3160 REMOTE COMPUTING DEVICE

3174

3178

3102

3150 DISPLAY

COMPUTATION RESOURCE

MODEM

INTERNET

LAN 3172

3162 REMOTE APPLICATION PROGRAMS

3102

3108

3106

3152

3176

3104

SYSTEM MEMORY

VIDEO ADAPTER

NETWORK ADAPTER

OPERATING SYSTEM        3130

APPLICATION PROGRAMS 3132

3117

DATA MEDIA INTERFACES

SYSTEM BUS

PROGRAM MODULES        3134

PROGRAM DATA            3136

3112            RAM

OPERATING SYSTEM        3130

APPLICATION PROGRAMS    3132

3116

PROCESSING UNIT

BIOS        3114

PROGRAM MODULES        3134

PROGRAM DATA            3136

3110            ROM

3142

I/O INTERFACES

INPUT MEDIA

3138

AT LEAST TWO IMAGES 130

FIG. 31

SOLID-STATE IMAGE TRANSDUCER

128

FIG. 32

FIG. 33

FIG. 34

3305

EP 2 997 886 B1

A

3300

3V3

3308

J2A

| 16 | COM2 |
| 15 | COM1 |
| 14 | OC_4D |
| 13 | 4C_4E |
| 12 | 4B 4G |
| 11 | 4A_4F |
| 10 | DOT_3D |
| 9 | 3C_3E |
| 8 | 3B_3G |
| 7 | 3A_3F |
| 6 | OF_2D |
| 5 | 2C_2E |
| 4 | 2B_2G |
| 3 | 2A_2F |
| 2 | 1B_1C |
| 1 | BatSym |

GND

LCD MODULE 16PIN

FIG. 35

3305

A

71

FIG. 36

3600

3306

3310

J4
BAT+
BAT-
BATTERY CONTACTS
MARK SILK SCREEN + AND -
GND

VBAT
TP14
TP15

3V3
C3 100nF
C2 100nF
GND
GND
C4 100uF
GND

R16 931K
R19 576K
GND

R17 DNP
R20 100K
GND

U2
SW
GND
EN
VIN
VOUT
VFB
MCP1640B
1
2
3
6
5
4

C6 10uF
GND
VBAT
L1 4u7H
GND

D3 BAT54CWT1G
EN 3V3
NO FOOTPRINT YET SO T323

SW4
EVQQ2W02W
TOP
BOTTOM
TP17
TP18
R18 100K
GND
EN SW

EP 2 997 886 B1

3700

FIG. 37

FIG. 38   PRIOR ART

**REFERENCES CITED IN THE DESCRIPTION**

**Patent documents cited in the description**

- JP 11113858 B